# EUROPEAN PATENT SPECIFICATION

(11) **EP 3 887 371 B1**
(45) Date of publication and mention of the grant of the patent: **22.02.2023**
(21) Application number: 19809441.9
(22) Date of filing: 25.11.2019
(51) Int. Cl.: C07D 471/04, C07D 487/04, A61K 31/437, A61P 31/04

(54) **ARYL COMPOUNDS FOR THE TREATMENT AND PROPHYLAXIS OF BACTERIAL INFECTION**
ARYLVERBINDUNGEN ZUR BEHANDLUNG UND PROPHYLAXE EINER BAKTERIELLEN INFEKTION
COMPOSÉS ARYLE POUR LE TRAITEMENT ET LA PROPHYLAXIE D'UNE INFECTION BACTÉRIENNE

(30) Priority: 27.11.2018 WO PCT/CN2018/117721
(43) Date of publication of application: 06.10.2021
(73) Proprietor: F. Hoffmann-La Roche AG, 4070 Basel (CH)
(72) Inventor: DEY, Fabian, 4070 Basel (CH); HU, Yimin, Shanghai, 201203 (CN); LIU, Yongqiang, Shanghai, 201203 (CN); SHEN, Hong, Shanghai, 201203 (CN); SHI, Houguang, Shanghai, 201203 (CN); TAN, Xuefei, Shanghai, 201203 (CN); YAN, Shixiang, Shanghai, 201203 (CN); ZHANG, Weixing, Shanghai, 201203 (CN); ZHANG, Zhiwei, Shanghai, 201203 (CN); ZHOU, Chengang, Shanghai, 201203 (CN); ZHOU, Mingwei, Shanghai, 201203 (CN); ZHU, Wei, Shanghai, 201203 (CN)
(74) Representative: Jochnowitz, Evan
(86) International application number: PCT/EP2019/082336
(87) International publication number: WO 2020/109191

(56) References cited:
- WO-A1-2012/125746
- WO-A1-2018/178041

## Description

The present invention relates to organic compounds useful for therapy and/or prophylaxis in a mammal, and in particular to inhibitors of DNA gyrase and/or topoisomerase IV useful for treatment and/or prophylaxis of bacterial infection.

### FIELD OF THE INVENTION

Bacterial infections pose a continuing medical problem because anti-bacterial drugs eventually engender resistance in the bacteria on which they are used. Bacterial resistance against virtually all current antibiotic drugs are increasing. Many forms of antibiotic resistance can even cross international boundaries and spread with remarkable speed. Thus novel classes of antibacterial compounds are urgently needed.

One target for development of anti-bacterial drugs has been DNA gyrase and topoisomerase IV (bacterial type IIA topoisomerases), which are essential to cell life, that solve DNA topological problems resulting from the replication, transcription, and recombination of DNA. DNA Gyrase controls DNA supercoiling and relieves topological stress that occurs when the DNA strands are untwisted such as during replication. Topoisomerase IV primarily resolves linked chromosome dimers at the conclusion of DNA replication. Both enzymes can introduce double stranded DNA breaks; pass a second DNA strand through the break and rejoining the broken strands. The activity of both enzymes is driven by the binding and hydrolysis of ATP. Bacterial DNA gyrase consists of two A (GyrA) and two B (GyrB) subunits. Binding and cleavage of the DNA is associated with GyrA, whereas ATP is bound and hydrolyzed by GyrB. Bacterial Topoisomerase IV is also a hetero-tetramer that consists of two C (ParC) and two E (ParE) subunits. The latter subunits bind ATP like GyrB in order to supply energy necessary for catalytic turnover of the enzymes.

Inhibition of DNA gyrase and topoisomerase IV has potential for the development of broad-spectrum antibiotics. The enzymes are highly conserved across a broad range of gram-positive and gram-negative pathogens. There are two classes of antibiotics that demonstrated such mechanism of action. The first, well-represented by the quinolones, inhibits GyrA and ParC subunits by stabilizing the cleaved DNA-enzyme complex, thus inhibiting overall gyrase function, leading to cell death. Novobiocin, the only marketed drug in the second class, exerts its effect by blocking the ATPase activity of the enzymes. Novobiocin was identified in 1950s. But its use declined rapidly and it was eventually withdrawn from the market, mainly due to its low permeability in many bacteria strains, rise of spontaneous resistance development, and the development of more effective drugs, such as penicillinase-stable penicillins and the first cephalosporins in 1960s and 1970s.

Recently, strong inhibition of DNA gyrase and/or topoisomerase IV has been recognized to be important for low resistance development in bacterial strains treated by inhibitors of the enzymes. Inhibitors of bacterial DNA gyrase and/or topoisomerase IV with different mechanism of action compare to the widely used quinolones will exhibit minimal cross resistance, and will be potentially useful in combating quinolone resistance that has increased significantly in the past few years.

### SUMMARY OF THE INVENTION

The present invention relates to novel compounds of formula (I), 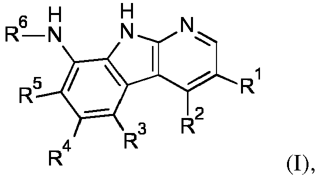 wherein
R¹ is naphthyridinyl;
   pyrazolo[1,5-a]pyrimidinyl which is unsubstituted or substituted by cyano;
   pyridinyl which is substituted by 2-oxo-3H-1,3,4-oxadiazolyl; amino; aminoC₁₋₆alkyl; aminocarbonyl; aminocarbonylC₁₋₆alkyl; C₁₋₆alkoxyaminocarbonyl; C₁₋₆alkoxyC₁₋₆alkylaminocarbonyl; C₁₋₆alkoxyC₁₋₆alkylC₃₋₇cycloalkylaminocarbonyl; C₁₋₆alkyl; C₁₋₆alkyl-1,3,4-oxadiazolylC₁₋₆alkyl; C₁₋₆alkylaminocarbonyl; cyano; halogen; hydroxyC₁₋₆alkyl; imidazolylC₁₋₆alkyl; morpholinyl; or morpholinylC₁₋₆alkyl; or
   pyrimidinyl which is unsubstituted or substituted by amino; aminoC₁₋₆alkoxy; aminoC₁₋₆alkyl; aminocarbonyl; azetidinyloxy; C₁₋₆alkoxy; C₁₋₆alkoxyaminocarbonylC₃₋₇cycloalkyl; C₁₋₆alkoxyC₁₋₆alkylamino; C₁₋₆alkyl; C₁₋₆alkylamino; C₁₋₆alkylaminocarbonylC₃₋₇cycloalkyl; carboxy(C₃₋₇cycloalkyl)C₁₋₆alkylamino; carboxyC₁₋₆alkoxy; carboxyC₁₋₆alkylamino; carboxyC₃₋₇cycloalkyl; hydroxyC₁₋₆alkyl; morpholinyl; piperazinyl; piperidinylamino; piperidinyloxy; pyrrolidinylamino; pyrrolidinyloxy; or tetrahydrofuranylamino;
R² is 4,5,6,7-tetrahydropyrazolo[3,4-c]pyridinyl substituted by cyano;
   pyrazolyl which is substituted once or twice by substituents independently selected from haloC₁₋₆alkyl; aminoC₁₋₆alkyl; aminocarbonyl; C₁₋₆alkyl; cyano; and hydroxyC₁₋₆alkyl;
   pyrrolo[3,4-c]pyrazolyl which is unsubstituted or substituted by cyano or haloC₁₋₆alkyl; or
   thiazolyl;
R³ is H or halogen;
R⁴ is halogen;
R⁵ is H or halogen;
R⁶ is C₁₋₆alkyl;
or pharmaceutically acceptable salt thereof.

Objects of the present invention are novel compounds of formula (I), their manufacture, medicaments based on a compound in accordance with the invention and their production as well as the compounds of formula (I) for use in the treatment or prophylaxis of bacterial infection. The compounds of formula (I) as DNA for use as gyrase and/or topoisomerase IV inhibitors is also one of the object of the present invention. The compounds of formula (I) showed superior anti-bacterial activity, good solubility, good CC₅₀ profiles, improved microsomal stability and/or improved PK profile.

### DETAILED DESCRIPTION OF THE INVENTION

Unless otherwise defined, all technical and scientific terms used herein have the same meaning as commonly understood by one of ordinary skill in the art to which this invention belongs. Furthermore, the following definitions are set forth to illustrate and define the meaning and scope of the various terms used to describe the invention.

Any references in the description to methods of treatment refer to the compounds, pharmaceutical compositions and medicaments of the present invention for use in a method for treatment of the human (or animal) body by therapy (or for diagnosis).

### DEFINITIONS

The term "C₁₋₆alkyl" denotes a saturated, linear or branched chain alkyl group containing 1 to 6, particularly 1 to 4 carbon atoms, for example methyl, ethyl, propyl, isopropyl, n-butyl, isobutyl, tert-butyl and the like. Particular "C₁₋₆alkyl" groups are methyl, ethyl and propyl.

The term "C₁₋₆alkoxy" denotes a group of the formula C₁₋₆alkyl-O-. Examples of C₁₋₆alkoxy group include, but not limited to, methoxy, ethoxy, n-propoxy, isopropoxy, n-butoxy, isobutoxy and tert-butoxy. Particular "C₁₋₆alkoxy" groups are methoxy, ethoxy and isopropoxy.

The term "C₃₋₇cycloalkyl" denotes to a saturated carbon ring containing from 3 to 7 carbon atoms, particularly from 3 to 6 carbon atoms, for example, cyclopropyl, cyclobutyl, cyclopentyl, cyclohexyl, cycloheptyl and the like. Particular "C₃₋₇cycloalkyl" group is cyclopropyl.

The term "halogen" and "halo" are used interchangeably herein and denote fluoro, chloro, bromo, or iodo.

The term "haloC₁₋₆alkyl" denotes an alkyl group wherein at least one of the hydrogen atoms of the alkyl group has been replaced by same or different halogen atoms, particularly fluoro atom. Examples of haloC₁₋₆alkyl include monofluoro-, difluoro-or trifluoro-methyl, -ethyl or -propyl, for example 3,3,3-trifluoropropyl, 2-fluoroethyl, 2,2,2-trifluoroethyl, fluoromethyl, difluoromethyl, trifluoromethyl and trifluoroethyl.

The term "azetidinyloxy" denotes azetidinyl-O-.

The term "piperidinyloxy" denotes piperidinyl-O-.

The term "pyrrolidinyloxy" denotes pyrrolidinyl-O-.

The term "pharmaceutically acceptable salts" denotes salts which are not biologically or otherwise undesirable. Pharmaceutically acceptable salts include both acid and base addition salts.

The term "pharmaceutically acceptable acid addition salt" denotes those pharmaceutically acceptable salts formed with inorganic acids such as hydrochloric acid, hydrobromic acid, sulfuric acid, nitric acid, carbonic acid, phosphoric acid, and organic acids selected from aliphatic, cycloaliphatic, aromatic, araliphatic, heterocyclic, carboxylic, and sulfonic classes of organic acids such as formic acid, acetic acid, propionic acid, glycolic acid, gluconic acid, lactic acid, pyruvic acid, oxalic acid, malic acid, maleic acid, maloneic acid, succinic acid, fumaric acid, tartaric acid, citric acid, aspartic acid, ascorbic acid, glutamic acid, anthranilic acid, benzoic acid, cinnamic acid, mandelic acid, embonic acid, phenylacetic acid, methanesulfonic acid, ethanesulfonic acid, p-toluenesulfonic acid, and salicyclic acid.

The term "pharmaceutically acceptable base addition salt" denotes those pharmaceutically acceptable salts formed with an organic or inorganic base. Examples of acceptable inorganic bases include sodium, potassium, ammonium, calcium, magnesium, iron, zinc, copper, manganese, and aluminum salts. Salts derived from pharmaceutically acceptable organic nontoxic bases includes salts of primary, secondary, and tertiary amines, substituted amines including naturally occurring substituted amines, cyclic amines and basic ion exchange resins, such as isopropylamine, trimethylamine, diethylamine, triethylamine, tripropylamine, ethanolamine, 2-diethylaminoethanol, trimethamine, dicyclohexylamine, lysine, arginine, histidine, caffeine, procaine, hydrabamine, choline, betaine, ethylenediamine, glucosamine, methylglucamine, theobromine, purines, piperizine, piperidine, N-ethylpiperidine, and polyamine resins.

The term "therapeutically effective amount" denotes an amount of a compound or molecule of the present invention that, when administered to a subject, (i) treats or prevents the particular disease, condition or disorder, (ii) attenuates, ameliorates or eliminates one or more symptoms of the particular disease, condition, or disorder, or (iii) prevents or delays the onset of one or more symptoms of the particular disease, condition or disorder described herein. The therapeutically effective amount will vary depending on the compound, the disease state being treated, the severity of the disease treated, the age and relative health of the subject, the route and form of administration, the judgement of the attending medical or veterinary practitioner, and other factors.

The term "pharmaceutical composition" denotes a mixture or solution comprising a therapeutically effective amount of an active pharmaceutical ingredient together with pharmaceutically acceptable excipients to be administered to a mammal, e.g., a human in need thereof.

### INHIBITORS OF DNA GYRASE AND/OR TOPOISOMERASE IV

The present invention relates to a compound of formula (I), 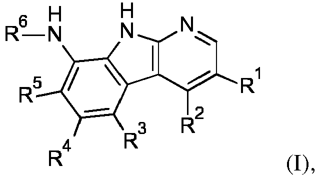 wherein
R¹ is naphthyridinyl;
   pyrazolo[1,5-a]pyrimidinyl which is unsubstituted or substituted by cyano;
   pyridinyl which is substituted by 2-oxo-3H-1,3,4-oxadiazolyl; amino; aminoC₁₋₆alkyl; aminocarbonyl; aminocarbonylC₁₋₆alkyl; C₁₋₆alkoxyaminocarbonyl; C₁₋₆alkoxyC₁₋₆alkylaminocarbonyl; C₁₋₆alkoxyC₁₋₆alkylC₃₋₇cycloalkylaminocarbonyl; C₁₋₆alkyl; C₁₋₆alkyl-1,3,4-oxadiazolylC₁₋₆alkyl; C₁₋₆alkylaminocarbonyl; cyano; halogen; hydroxyC₁₋₆alkyl; imidazolylC₁₋₆alkyl; morpholinyl; or morpholinylC₁₋₆alkyl; or
   pyrimidinyl which is unsubstituted or substituted by amino; aminoC₁₋₆alkoxy; aminoC₁₋₆alkyl; aminocarbonyl; azetidinyloxy; C₁₋₆alkoxy; C₁₋₆alkoxyaminocarbonylC₃₋₇cycloalkyl; C₁₋₆alkoxyC₁₋₆alkylamino; C₁₋₆alkyl; C₁₋₆alkylamino; C₁₋₆alkylaminocarbonylC₃₋₇cycloalkyl; carboxy(C₃₋₇cycloalkyl)C₁₋₆alkylamino; carboxyC₁₋₆alkoxy; carboxyC₁₋₆alkylamino; carboxyC₃₋₇cycloalkyl; hydroxyC₁₋₆alkyl; morpholinyl; piperazinyl; piperidinylamino; piperidinyloxy; pyrrolidinylamino; pyrrolidinyloxy; or tetrahydrofuranylamino;
R² is 4,5,6,7-tetrahydropyrazolo[3,4-c]pyridinyl substituted by cyano;
   pyrazolyl which is substituted once or twice by substituents independently selected from haloC₁₋₆alkyl; aminoC₁₋₆alkyl; aminocarbonyl; C₁₋₆alkyl; cyano; and hydroxyC₁₋₆alkyl;
   pyrrolo[3,4-c]pyrazolyl which is unsubstituted or substituted by cyano or haloC₁₋₆alkyl; or
   thiazolyl;
R³ is H or halogen;
R⁴ is halogen;
R⁵ is H or halogen;
R⁶ is C₁₋₆alkyl;
or pharmaceutically acceptable salt thereof.

A further embodiment of present invention is (ii) a compound of formula (I) according to (i), wherein
R¹ is naphthyridinyl;
   pyrazolo[1,5-a]pyrimidinyl which is unsubstituted or substituted by cyano;
   pyridinyl which is substituted by 2-oxo-3H-1,3,4-oxadiazolyl; amino; aminocarbonyl; aminocarbonylmethyl; aminomethyl; cyano; fluoro; hydroxyethyl; hydroxymethyl; imidazolylmethyl; methoxyaminocarbonyl; methoxyethylaminocarbonyl; methoxymethylcyclopropylaminocarbonyl; methyl; methyl-1,3,4-oxadiazolylmethyl; methylaminocarbonyl; morpholinyl; or morpholinylmethyl; or
   pyrimidinyl which is unsubstituted or substituted by amino; amino(dimethyl)ethoxy; aminocarbonyl; aminomethyl; azetidinyloxy; carboxy(cyclopropyl)methylamino; carboxy(dimethyl)ethoxy; carboxy(dimethyl)ethylamino; carboxycyclopropyl; ethoxy; ethyl; hydroxy(methyl)ethyl; hydroxyethyl; hydroxymethyl; methoxy; methoxyaminocarbonylcyclopropyl; methoxyethylamino; methyl; methylamino; methylaminocarbonylcyclopropyl; morpholinyl; piperazinyl; piperidinylamino; piperidinyloxy; pyrrolidinylamino; pyrrolidinyloxy; or tetrahydrofuranylamino;
R² is 4,5,6,7-tetrahydropyrazolo[3,4-c]pyridinyl substituted by cyano;
   pyrazolyl which is substituted once or twice by substituents independently selected from aminocarbonyl; aminomethyl; cyano; difluoromethyl; ethyl; hydroxymethyl; and trifluoromethyl;
   pyrrolo[3,4-c]pyrazolyl which is unsubstituted or substituted by cyano or trifluoromethyl; or
   thiazolyl;
R³ is H or fluoro;
R⁴ is fluoro or chloro;
R⁵ is H or fluoro;
R⁶ is ethyl or methyl;
or pharmaceutically acceptable salt thereof.

A further embodiment of present invention is (iii) a compound of formula (I) according to (i) or (ii), or pharmaceutically acceptable salt thereof, wherein
R¹ is naphthyridinyl;
   pyrazolo[1,5-a]pyrimidinyl;
   pyridinyl which is substituted by 2-oxo-3H-1,3,4-oxadiazolyl; amino; aminoC₁₋₆alkyl; aminocarbonyl; C₁₋₆alkoxyaminocarbonyl; C₁₋₆alkoxyC₁₋₆alkylaminocarbonyl; C₁₋₆alkylaminocarbonyl; cyano; or hydroxyC₁₋₆alkyl; or
   pyrimidinyl which is unsubstituted or substituted by amino; aminoC₁₋₆alkoxy; aminoC₁₋₆alkyl; aminocarbonyl; C₁₋₆alkoxy; C₁₋₆alkoxyC₁₋₆alkylamino; C₁₋₆alkyl; C₁₋₆alkylamino; hydroxyC₁₋₆alkyl; piperazinyl; piperidinylamino; piperidinyloxy; pyrrolidinylamino; pyrrolidinyloxy; or tetrahydrofuranylamino.

A further embodiment of present invention is (iv) a compound of formula (I) according to any one of (i) to (iii), or pharmaceutically acceptable salt thereof, wherein R² is pyrazolyl which is substituted once or twice by substituents independently selected from haloC₁₋₆alkyl, aminoC₁₋₆alkyl, aminocarbonyl, C₁₋₆alkyl, cyano, and hydroxyC₁₋₆alkyl.

A further embodiment of present invention is (v) a compound of formula (I) according to any one of (i) to (iv), wherein
R¹ is naphthyridinyl;
   pyrazolo[1,5-a]pyrimidinyl;
   pyridinyl which is substituted by 2-oxo-3H-1,3,4-oxadiazolyl; amino; aminoC₁₋₆alkyl; aminocarbonyl; C₁₋₆alkoxyaminocarbonyl; C₁₋₆alkoxyC₁₋₆alkylaminocarbonyl; C₁₋₆alkylaminocarbonyl; cyano; or hydroxyC₁₋₆alkyl; or
   pyrimidinyl which is unsubstituted or substituted by amino; aminoC₁₋₆alkoxy; aminoC₁₋₆alkyl; aminocarbonyl; C₁₋₆alkoxy; C₁₋₆alkoxyC₁₋₆alkylamino; C₁₋₆alkyl; C₁₋₆alkylamino; hydroxyC₁₋₆alkyl; piperazinyl; piperidinylamino; piperidinyloxy; pyrrolidinylamino; pyrrolidinyloxy; or tetrahydrofuranylamino;
R² is pyrazolyl which is substituted once or twice by substituents independently selected from haloC₁₋₆alkyl, aminoC₁₋₆alkyl, aminocarbonyl, C₁₋₆alkyl, cyano, and hydroxyC₁₋₆alkyl;
R³ is H or halogen;
R⁴ is halogen;
R⁵ is H or halogen;
R⁶ is C₁₋₆alkyl;
or pharmaceutically acceptable salt thereof.

A further embodiment of present invention is (vi) a compound of formula (I) according to any one of (i) to (v), wherein
R¹ is naphthyridinyl; pyrazolo[1,5-a]pyrimidinyl; cyanopyridinyl; aminocarbonylpyridinyl; hydroxymethylpyridinyl; aminopyridinyl; methylaminocarbonylpyridinyl; aminomethylpyridinyl; methoxyaminocarbonylpyridinyl; 2-oxo-3H-1,3,4-oxadiazolylpyridinyl; hydroxyethylpyridinyl; methoxyethylaminocarbonylpyridinyl; amino(dimethyl)ethoxypyrimidinyl; aminocarbonylpyrimidinyl; aminomethylpyrimidinyl; aminopyrimidinyl; ethoxypyrimidinyl; hydroxy(methyl)ethylpyrimidinyl; hydroxyethylpyrimidinyl; hydroxymethylpyrimidinyl; methoxyethylaminopyrimidinyl; methoxypyrimidinyl; methylaminopyrimidinyl; methylpyrimidinyl; piperazinylpyrimidinyl; piperidinylaminopyrimidinyl; piperidinyloxypyrimidinyl; pyrrolidinylaminopyrimidinyl; pyrrolidinyloxypyrimidinyl; or tetrahydrofuranylaminopyrimidinyl;
R² is aminocarbonyl(trifluoromethyl)pyrazolyl; aminomethyl(trifluoromethyl)pyrazolyl; cyanopyrazolyl; difluoromethylpyrazolyl; ethylpyrazolyl; hydroxymethyl(trifluoromethyl)pyrazolyl; or trifluoromethylpyrazolyl;
R³ is H or fluoro;
R⁴ is fluoro or chloro;
R⁵ is H or fluoro;
R⁶ is methyl or ethyl;
or pharmaceutically acceptable salt thereof.

Another embodiment of present invention is that (vii) compounds of formula (I) are selected from:
5-[8-(Ethylamino)-6-fluoro-4-[3-(trifluoromethyl)pyrazol-1-yl]-9H-pyrido[2,3-b]indol-3-yl]pyridine-3-carbonitrile;
6-Fluoro-3-(2-methoxypyrimidin-5-yl)-N-methyl-4-[3-(trifluoromethyl)pyrazol-1-yl]-9H-pyrido[2,3-b]indol-8-amine;
5-[8-(Ethylamino)-6-fluoro-4-[4-(trifluoromethyl)pyrazol-1-yl]-9H-pyrido[2,3-b]indol-3-yl]pyridine-3-carbonitrile;
N-Ethyl-6-fluoro-3-(2-methoxypyrimidin-5-yl)-4-[3-(trifluoromethyl)pyrazol-1-yl]-9H-pyrido[2,3-b]indol-8-amine;
5-[4-(3-Cyanopyrazol-1-yl)-8-(ethylamino)-6-fluoro-9H-pyrido[2,3-b]indol-3-yl]pyridine-3-carbonitrile;
3-(2-Aminopyrimidin-5-yl)-N-ethyl-6-fluoro-4-[3-(trifluoromethyl)pyrazol-1-yl]-9H-pyrido[2,3-b]indol-8-amine;
N-ethyl-6-fluoro-3-(6-methyl-3-pyridyl)-4-[3-(trifluoromethyl)pyrazol-1-yl]-9H-pyrido[2,3-b]indol-8-amine;
5-[6-Fluoro-8-(methylamino)-4-[4-(trifluoromethyl)pyrazol-1-yl]-9H-pyrido[2,3-b]indol-3-yl]pyridine-3-carbonitrile;
5-[4-(4-Cyanopyrazol-1-yl)-6-fluoro-8-(methylamino)-9H-pyrido[2,3-b]indol-3-yl]pyridine-3-carbonitrile;
N-Ethyl-6-fluoro-3-[2-(4-piperidylamino)pyrimidin-5-yl]-4-[3-(trifluoromethyl)pyrazol-1-yl]-9H-pyrido[2,3-b]indol-8-amine;
N-Ethyl-6-fluoro-3-(1,5-naphthyridin-3-yl)-4-[3-(trifluoromethyl)pyrazol-1-yl]-9H-pyrido[2,3-b]indol-8-amine;
N-Ethyl-6-fluoro-3-pyrimidin-5-yl-4-[3-(trifluoromethyl)pyrazol-1-yl]-9H-pyrido[2,3-b]indol-8-amine;
N-Ethyl-6-fluoro-3-[2-(2-methoxyethylamino)pyrimidin-5-yl]-4-[3-(trifluoromethyl)pyrazol-1-yl]-9H-pyrido[2,3-b]indol-8-amine;
5-[4-[4-(Aminomethyl)-3-(trifluoromethyl)pyrazol-1-yl]-6-fluoro-8-(methylamino)-9H-pyrido [2,3-b]indol-3-yl]pyridine-3-carbonitrile;
5-[8-(Ethylamino)-6-fluoro-4-[3-(trifluoromethyl)pyrazol-1-yl]-9H-pyrido[2,3-b]indol-3-yl]pyridine-3-carboxamide;
N-Ethyl-6-fluoro-3-(2-morpholinopyrimidin-5-yl)-4-[3-(trifluoromethyl)pyrazol-1-yl]-9H-pyrido[2,3-b]indol-8-amine;
N-Ethyl-6-fluoro-3-[2-(methylamino)pyrimidin-5-yl]-4-[3-(trifluoromethyl)pyrazol-1-yl]-9H-pyrido[2,3-b]indol-8-amine;
N-Ethyl-6-fluoro-3-(5-fluoro-3-pyridyl)-4-[3-(trifluoromethyl)pyrazol-1-yl]-9H-pyrido[2,3-b]indol-8-amine;
N-Ethyl-6-fluoro-3-pyrazolo[1,5-a]pyrimidin-6-yl-4-[3-(trifluoromethyl)pyrazol-1-yl]-9H-pyrido[2,3-b]indol-8-amine;
N-Ethyl-6-fluoro-3-(2-pyrrolidin-3-yloxypyrimidin-5-yl)-4-[3-(trifluoromethyl)pyrazol-1-yl]-9H-pyrido[2,3-b]indol-8-amine;
4-(5,6-Dihydro-4H-pyrrolo[3,4-c]pyrazol-1-yl)-N-ethyl-6-fluoro-3-(2-methoxypyrimidin-5-yl)-9H-pyrido[2,3-b]indol-8-amine;
3-(2-Ethoxypyrimidin-5-yl)-N-ethyl-6-fluoro-4-[3-(trifluoromethyl)pyrazol-1-yl]-9H-pyrido[2,3-b]indol-8-amine;
3-[2-(Azetidin-3-yloxy)pyrimidin-5-yl]-N-ethyl-6-fluoro-4-[3-(trifluoromethyl)pyrazol-1-yl]-9H-pyrido[2,3-b]indol-8-amine;
N-Ethyl-6-fluoro-3-[2-(pyrrolidin-3-ylamino)pyrimidin-5-yl]-4-[3-(trifluoromethyl)pyrazol-1-yl]-9H-pyrido[2,3-b]indol-8-amine;
N-Ethyl-6-fluoro-3-[2-(tetrahydrofuran-3-ylamino)pyrimidin-5-yl]-4-[3-(trifluoromethyl)pyrazol-1-yl]-9H-pyrido[2,3-b]indol-8-amine;
5-[4-[4-(Aminomethyl)-3-(trifluoromethyl)pyrazol-1-yl]-8-(ethylamino)-6-fluoro-9H-pyrido [2,3-b]indol-3-yl]pyridine-3-carbonitrile;
4-[4-(Aminomethyl)-3-(trifluoromethyl)pyrazol-1-yl]-N-ethyl-6-fluoro-3-(2-methoxypyrimidin-5-yl)-9H-pyrido[2,3-b]indol-8-amine;
5-[8-(Ethylamino)-5,6-difluoro-4-[3-(trifluoromethyl)pyrazol-1-yl]-9H-pyrido[2,3-b]indol-3-yl]pyridine-3-carbonitrile;
N-Ethyl-5,6-difluoro-3-(2-methoxypyrimidin-5-yl)-4-[3-(trifluoromethyl)pyrazol-1-yl]-9H-pyrido[2,3-b]indol-8-amine;
3-[2-(2-Amino-2-methyl-propoxy)pyrimidin-5-yl]-N-ethyl-6-fluoro-4-[3-(trifluoromethyl)pyrazol-1-yl]-9H-pyrido[2,3-b]indol-8-amine;
2-[5-[8-(Ethylamino)-6-fluoro-4-[3-(trifluoromethyl)pyrazol-1-yl]-9H-pyrido[2,3-b]indol-3-yl]pyrimidin-2-yl]propan-2-ol;
5-[8-(Ethylamino)-4-(3-ethylpyrazol-1-yl)-6-fluoro-9H-pyrido[2,3-b]indol-3-yl]pyridine-3-carbonitrile;
6-Chloro-N-ethyl-3-(2-methoxypyrimidin-5-yl)-4-[3-(trifluoromethyl)pyrazol-1-yl]-9H-pyrido[2,3-b]indol-8-amine;
5-[8-(Ethylamino)-6-fluoro-4-[3-(trifluoromethyl)-5,6-dihydro-4H-pyrrolo[3,4-c]pyrazol-1-yl]-9H-pyrido[2,3-b]indol-3-yl]pyridine-3-carbonitrile;
N-Ethyl-6-fluoro-3-(2-methoxypyrimidin-5-yl)-4-[3-(trifluoromethyl)-5,6-dihydro-4H-pyrrolo[3,4-c]pyrazol-1-yl]-9H-pyrido[2,3-b]indol-8-amine;
[5-[8-(Ethylamino)-6-fluoro-4-[3-(trifluoromethyl)pyrazol-1-yl]-9H-pyrido[2,3-b]indol-3-yl]-3-pyridyl]methanol;
3-(5-Amino-3-pyridyl)-N-ethyl-6-fluoro-4-[3-(trifluoromethyl)pyrazol-1-yl]-9H-pyrido[2,3-b]indol-8-amine;
1- [6-Fluoro-3-(2-methoxypyrimidin-5-yl)-8-(methylamino)-9H-pyrido[2,3-b]indol-4-yl]-5,6-dihydro-4H-pyrrolo[3,4-c]pyrazole-3-carbonitrile;
1-[8-(Ethylamino)-6-fluoro-3-(2-methoxypyrimidin-5-yl)-9H-pyrido[2,3-b]indol-4-yl]-4,5,6,7-tetrahydropyrazolo[3,4-c]pyridine-3-carbonitrile;
3-(6-Amino-3-pyridyl)-N-ethyl-6-fluoro-4-[3-(trifluoromethyl)pyrazol-1-yl]-9H-pyrido[2,3-b]indol-8-amine;
5-[4-[3-(Difluoromethyl)pyrazol-1-yl]-8-(ethylamino)-6-fluoro-9H-pyrido[2,3-b]indol-3-yl]pyridine-3-carbonitrile;
N-Ethyl-6-fluoro-3-[6-(morpholinomethyl)-3-pyridyl]-4-[3-(trifluoromethyl)pyrazol-1-yl]-9H-pyrido[2,3-b]indol-8-amine;
5-[8-(Ethylamino)-6-fluoro-4-[3-(trifluoromethyl)pyrazol-1-yl]-9H-pyrido[2,3-b]indol-3-yl]-N-methyl-pyridine-3-carboxamide;
N-Ethyl-6-fluoro-3-(5-morpholino-3-pyridyl)-4-[3-(trifluoromethyl)pyrazol-1-yl]-9H-pyrido[2,3-b]indol-8-amine;
3-[5-(Aminomethyl)-3-pyridyl]-N-ethyl-6-fluoro-4-[3-(trifluoromethyl)pyrazol-1-yl]-9H-pyrido[2,3-b]indol-8-amine;
3-[2-(Aminomethyl)pyrimidin-5-yl]-N-ethyl-6-fluoro-4-[3-(trifluoromethyl)pyrazol-1-yl]-9H-pyrido[2,3-b]indol-8-amine;
3-(2-Ethylpyrimidin-5-yl)-6-fluoro-N-methyl-4-[3-(trifluoromethyl)pyrazol-1-yl]-9H-pyrido[2,3-b]indol-8-amine;
[5-[8-(Ethylamino)-6-fluoro-4-[3-(trifluoromethyl)pyrazol-1-yl]-9H-pyrido[2,3-b]indol-3-yl]pyrimidin-2-yl]methanol;
5-[4-[4-(Aminomethyl)-3-(trifluoromethyl)pyrazol-1-yl]-6,7-difluoro-8-(methylamino)-9H-pyrido [2,3 -b] indol- 3 -yl]pyridine- 3 -carbonitrile;
N-ethyl-6-fluoro-3-(2-methylpyrimidin-5-yl)-4-[3-trifluoromethyl)pyrazol-1-yl]-9H-pyrido[2,3-b]indol-8-amine;
5-[8-(ethylamino)-6-fluoro-4-thiazol-5-yl-9H-pyrido[2,3-b]indol-3-yl]pyridine-3-carbonitrile;
N-ethyl-6-fluoro-3-(2-piperazin-1-ylpyrimidin-5-yl)-4-[3-(trifluoromethyl)pyrazol-1-yl]-9H-pyrido[2,3-b]indol-8-amine;
N-ethyl-6-fluoro-3-[2-(4-piperidyloxy)pyrimidin-5-yl]-4-[3-(trifluoromethyl)pyrazol-1-yl]-9H-pyrido[2,3-b]indol-8-amine;
1-[5-[8-(ethylamino)-6-fluoro-4-[3-(trifluoromethyl)pyrazol-1-yl]-9H-pyrido[2,3-b]indol-3-yl]pyrimidin-2-yl]ethanol;
N-ethyl-6-fluoro-3-[2-[(3R)-pyrrolidin-3-yl]oxypyrimidin-5-yl]-4-[3-(trifluoromethyl)pyrazol-1-yl]-9H-pyrido[2,3-b]indol-8-amine;
N-ethyl-6-fluoro-3-[2-[(3*S*)-pyrrolidin-3-yl]oxypyrimidin-5-yl]-4-[3-(trifluoromethyl)pyrazol-1-yl]-9H-pyrido[2,3-b]indol-8-amine;
(1S)-1-[5-[8-(ethylamino)-6-fluoro-4-[3-(trifluoromethyl)pyrazol-1-yl]-9H-pyrido[2,3-b]indol-3-yl]pyrimidin-2-yl]ethanol;
N-ethyl-6-fluoro-3-[5-[(5-methyl-1,3,4-oxadiazol-2-yl)methyl]-3-pyridyl]-4-[3-(trifluoromethyl)pyrazol-1-yl]-9H-pyrido[2,3-b]indol-8-amine;
6-[8-(ethylamino)-6-fluoro-4-[3-(trifluoromethyl)pyrazol-1-yl]-9H-pyrido[2,3-b]indol-3-yl]pyrazolo[1,5-a]pyrimidine-3-carbonitrile;
4-[4-(Aminomethyl)-3-(trifluoromethyl)pyrazol-1-yl]-N-ethyl-6-fluoro-3-pyrazolo[1,5-a]pyrimidin-6-yl-9H-pyrido[2,3-b]indol-8-amine;
1-[5-[8-(ethylamino)-6-fluoro-4-[3-(trifluoromethyl)pyrazol-1-yl]-9H-pyrido[2,3-b]indol-3-yl]pyrimidin-2-yl]cyclopropanecarboxylic acid;
1-(5-(8-(ethylamino)-6-fluoro-4-(3-(trifluoromethyl)-1H-pyrazol-1-yl)-9H-pyrido[2,3-b]indol-3-yl)pyrimidin-2-yl)-N-methylcyclopropanecarboxamide;
1-(5-(8-(ethylamino)-6-fluoro-4-(3-(trifluoromethyl)-1H-pyrazol-1-yl)-9H-pyrido[2,3-b]indol-3-yl)pyrimidin-2-yl)-N-methoxycyclopropanecarboxamide;
5-[8-(ethylamino)-6-fluoro-4-[3-(trifluoromethyl)pyrazol-1-yl]-9H-pyrido[2,3-b]indol-3-yl]-N-methoxy-pyridine-3-carboxamide;
5-[8-(ethylamino)-6-fluoro-4-[3-(trifluoromethyl)pyrazol-1-yl]-9H-pyrido[2,3-b]indol-3-yl]pyrimidine-2-carboxamide;
2-[5-[8-(ethylamino)-6-fluoro-4-[3-(trifluoromethyl)pyrazol-1-yl]-9H-pyrido[2,3-b]indol-3-yl]-3-pyridyl]acetamide;
5-[8-(ethylamino)-6-fluoro-4-[3-(trifluoromethyl)pyrazol-1-yl]-9H-pyrido[2,3-b]indol-3-yl]-N-(2-methoxyethyl)pyridine-3-carboxamide;
5-[8-(ethylamino)-6-fluoro-4-[3-(trifluoromethyl)pyrazol-1-yl]-9H-pyrido[2,3-b]indol-3-yl]-N-[1-(methoxymethyl)cyclopropyl]pyridine-3-carboxamide;
1-(3-(5-cyanopyridin-3-yl)-8-(ethylamino)-6-fluoro-9H-pyrido[2,3-b]indol-4-yl)-3-(trifluoromethyl)-1H-pyrazole-4-carboxamide;
5-[8-(ethylamino)-6-fluoro-4-[4-(hydroxymethyl)-3-(trifluoromethyl)pyrazol-1-yl]-9H-pyrido[2,3-b]indol-3-yl]pyridine-3-carbonitrile;
5-[[5-[8-(ethylamino)-6-fluoro-4-[3-(trifluoromethyl)pyrazol-1-yl]-9H-pyrido[2,3-b]indol-3-yl]-3-pyridyl]methyl]-3H-1,3,4-oxadiazol-2-one;
2-(5-(8-(ethylamino)-6-fluoro-4-(3-(trifluoromethyl)-1H-pyrazol-1-yl)-9H-pyrido[2,3-b]indol-3-yl)pyridin-3-yl)ethanol; and
3-(5-((1H-imidazol-2-yl)methyl)pyridin-3-yl)-N-ethyl-6-fluoro-4-(3-(trifluoromethyl)-1H-pyrazol-1-yl)-9H-pyrido[2,3-b]indol-8-amine;
or pharmaceutically acceptable salt thereof.

### SYNTHESIS

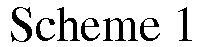

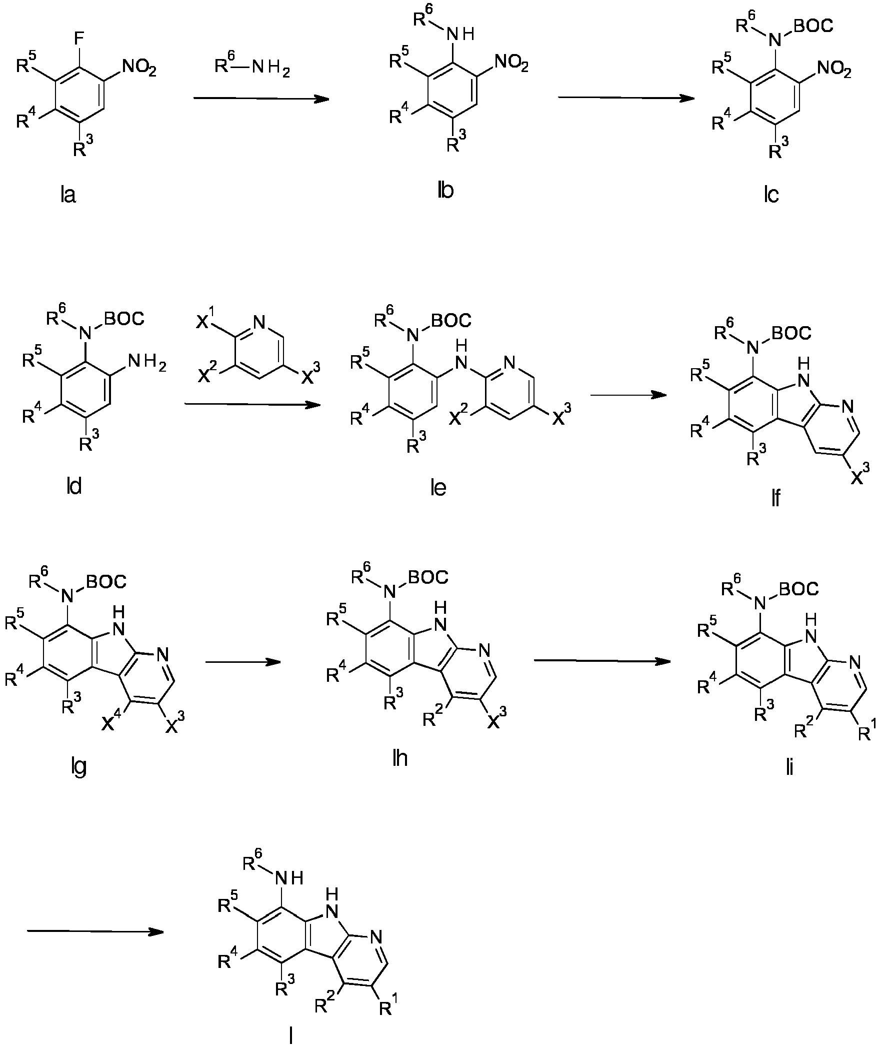

X¹, X², X³ and X⁴ are halogen.

Compound of formula (I) can be prepared according to Scheme 1. Nucleophilic substitution of ortho-fluoro nitrobenzene (Ia) with amine R⁶-NH₂ affords aniline (Ib). The aniline (Ib) can be protected with di-tert-butyl carbonate to give the protected aniline (Ic). The nitro group in aniline (Ic) can be reduced by a reducing agent, such as H₂ with palladium catalysts, to give the compound of formula (Id). Coupling of the compound of formula (Id) with trihalogenated pyridine can be achieved using palladium catalysts and phosphine ligands to give compound of formula (Ie). Cyclization of compound of formula (Ie) using palladium catalysts and phosphine ligands gives compound of formula (If). Compound of formula (Ig) can be obtained from formula (If) through oxidation of the pyridine followed by halogenation, such as treatment of POCl₃ or POBr₃. Coupling of compound of formula (Ig) to introduce R² can be achieved either through Buchwald-Hartwig Cross Coupling Reaction for certain C-N bond formation (with R² bearing a basic N), or a palladium catalyzed Suzuki coupling for C-C bond formation (with R² as a boronic ester or boronic acid), to give compound of formula (Ih). Further coupling of compound of formula (Ih) to introduce R¹ can be achieved using a palladium catalyzed Suzuki coupling to give compound of formula (Ii). Deprotection of compound of formula (Ii) in the presence of an acid, such as trifluoroacetic acid, affords compound of formula (I).

This invention also relates to a process for the preparation of a compound of formula (I) comprising the reaction of compound of formula (Ii), 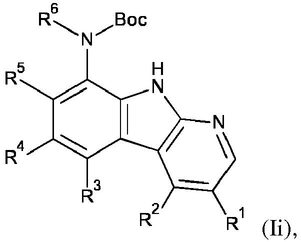
with an acid, which can be for example trifluoroacetic acid;
wherein R¹ to R⁶ are defined above. A compound of formula (I) when manufactured according to the above process is also an object of the invention.

### PHARMACEUTICAL COMPOSITIONS AND ADMINISTRATION

Another embodiment provides pharmaceutical compositions or medicaments containing the compounds of the invention and a therapeutically inert carrier, diluent or excipient, as well as methods of using the compounds of the invention to prepare such compositions and medicaments. In one example, compounds of formula (I) may be formulated by mixing at ambient temperature at the appropriate pH, and at the desired degree of purity, with physiologically acceptable carriers, i.e., carriers that are non-toxic to recipients at the dosages and concentrations employed into a galenical administration form. The pH of the formulation depends mainly on the particular use and the concentration of compound, but preferably ranges anywhere from about 3 to about 8. In one example, a compound of formula (I) is formulated in an acetate buffer, at pH 5. In another embodiment, the compounds of formula (I) are sterile. The compound may be stored, for example, as a solid or amorphous composition, as a lyophilized formulation or as an aqueous solution.

Compositions are formulated, dosed, and administered in a fashion consistent with good medical practice. Factors for consideration in this context include the particular disorder being treated, the particular mammal being treated, the clinical condition of the individual patient, the cause of the disorder, the site of delivery of the agent, the method of administration, the scheduling of administration, and other factors known to medical practitioners. The "effective amount" of the compound to be administered will be governed by such considerations, and is the minimum amount necessary to reduced bacterial load or improve host survival through the inhibition of bacterial DNA gyrase and/or Topoisomerase IV. For example, such amount may be below the amount that is toxic to normal cells, or the mammal as a whole.

In one example, the pharmaceutically effective amount of the compound of the invention administered parenterally per dose will be in the range of about 0.1 to 1000 mg/kg, alternatively about 1 to 100 mg/kg of patient body weight per day. In another embodiment, oral unit dosage forms, such as tablets and capsules, preferably contain from about 5 to about 5000 mg of the compound of the invention.

The compounds of the invention may be administered by any suitable means, including oral, topical (including buccal and sublingual), rectal, vaginal, transdermal, parenteral, subcutaneous, intraperitoneal, intrapulmonary, intradermal, intrathecal and epidural and intranasal, and, if desired for local treatment, intralesional administration. Parenteral infusions include intramuscular, intravenous, intraarterial, intraperitoneal, or subcutaneous administration.

The compounds of the present invention may be administered in any convenient administrative form, e.g., tablets, powders, capsules, solutions, dispersions, suspensions, syrups, sprays, suppositories, gels, emulsions, patches, etc. Such compositions may contain components conventional in pharmaceutical preparations, e.g., diluents, carriers, pH modifiers, sweeteners, bulking agents, and further active agents.

A typical formulation is prepared by mixing a compound of the present invention and a carrier or excipient. Suitable carriers and excipients are well known to those skilled in the art and are described in detail in, e.g., Ansel, Howard C., et al., Ansel's Pharmaceutical Dosage Forms and Drug Delivery Systems. Philadelphia: Lippincott, Williams & Wilkins, 2004; Gennaro, Alfonso R., et al. Remington: The Science and Practice of Pharmacy. Philadelphia: Lippincott, Williams & Wilkins, 2000; and Rowe, Raymond C. Handbook of Pharmaceutical Excipients. Chicago, Pharmaceutical Press, 2005. The formulations may also include one or more buffers, stabilizing agents, surfactants, wetting agents, lubricating agents, emulsifiers, suspending agents, preservatives, antioxidants, opaquing agents, glidants, processing aids, colorants, sweeteners, perfuming agents, flavoring agents, diluents and other known additives to provide an elegant presentation of the drug (i.e., a compound of the present invention or pharmaceutical composition thereof) or aid in the manufacturing of the pharmaceutical product (i.e., medicament).

An example of a suitable oral dosage form is a tablet containing about 10 to 500 mg of the compound of the invention compounded with about 40 to 400mg anhydrous lactose, about 5 to 50 mg sodium croscarmellose, about 5 to 50 mg polyvinylpyrrolidone (PVP) K30, and about 1 to 10 mg magnesium stearate. The powdered ingredients are first mixed together and then mixed with a solution of the PVP. The resulting composition can be dried, granulated, mixed with the magnesium stearate and compressed to tablet form using conventional equipment. An example of an aerosol formulation can be prepared by dissolving the compound, for example 5 to 1000 mg) of the invention in a suitable buffer solution, e.g. a phosphate buffer, adding a tonicifier, e.g. a salt such sodium chloride, if desired. The solution may be filtered, e.g., using a 0.2 micron filter, to remove impurities and contaminants.

An embodiment, therefore, includes a pharmaceutical composition comprising a compound of formula (I), or a stereoisomer or pharmaceutically acceptable salt thereof. In a further embodiment includes a pharmaceutical composition comprising a compound of formula (I), or a stereoisomer or pharmaceutically acceptable salt thereof, together with a pharmaceutically acceptable carrier or excipient.

Another embodiment includes a pharmaceutical composition comprising a compound of formula (I) for use in the treatment and/or prophylaxis of bacterial infections.

In some embodiments, the compounds of this invention may be administered, as part of a single or multiple dosage regimen, orally, parenterally, by inhalation spray, topically, rectally, nasally, buccally, vaginally, or via an implanted reservoir. The term parenteral as used includes subcutaneous, intracutaneous, intravenous, intramuscular, intra-articular, intrasynovial, intrasternal, intrathecal, intralesional and intracranial injection or infusion techniques. Typically, the pharmaceutical compositions of the invention will be administered from about 1 to 5 times per day or alternatively, as a continuous infusion upon improvement of a patient's condition.

### INDICATIONS AND METHODS OF TREATMENT

The compounds of the invention are useful for treatment and/or prophylaxis of bacterial infection in humans or other animals by administering to the subject in need of a therapeutically effective amount of compound of formula (I), or a pharmaceutically acceptable salt, or enantiomer or diastereomer thereof. The compounds and methods of the invention are particularly well suited for human patients infected by pathogens that include *Staphylococcus aureus, Escherichia coli, Klebsiella pneumoniae, Acinetobacter baumannii* and *Pseudomonas aeruginosa.* Examples of bacterial organisms that may also be controlled by the compounds of the invention include, but not limited to, the following Gram-Positive and Gram-Negative organisms: *Streptococcus pneumoniae, Streptococcus pyogenes, Enterococcus faecalis, Enterococcus faecium, Enterobacter spp.* species, *Proteus spp.* species, *Serratia marcescens, Staphylococcus aureus, Coag. Neg. Staphylococci, Haemophilus influenzae, Bacillus anthraces, Mycoplasma pneumoniae, Moraxella catarrhalis, Chlamydophila pneumoniae, Chlamydia trachomatis, Legionella pneumophila, Mycobacterium tuberculosis, Helicobacter pylori, Staphylococcus saprophyticus, Staphylococcus epidermidis, Francisella tularensis, Yersinia pestis, Clostridium difficile, Bacteroides spp.* species *Neisseria gonorrhoeae, Neisseria meningitidis, Burkholderia pseudomallei, Burkholderia mallei, Borrelia burgdorferi, Mycobacterium avium complex, Mycobacterium abscessus, Mycobacterium kansasii and Mycobacterium ulcerans.*

Examples of bacterial infections may include, but not limited to, upper respiratory infections, lower respiratory infections, ear infections, pleuropulmonary and bronchial infections, complicated urinary tract infections, uncomplicated urinary tract infections, intra-abdominal infections, cardiovascular infections, a blood stream infection, sepsis, bacteremia, CNS infections, skin and soft tissue infections, GI infections, bone and joint infections, genital infections, eye infections, or granulomatous infections. Examples of specific bacterial infections include, but not limited to, uncomplicated skin and skin structure infections (uSSSI), complicated skin and skin structure infections (cSSSI), catheter infections, pharyngitis, sinusitis, otitis extema, otitis media, bronchitis, empyema, pneumonia, community-acquired bacterial pneumoniae (CABP), hospital-acquired pneumonia (HAP), hospital-acquired bacterial pneumonia, ventilator-associated pneumonia (VAP), diabetic foot infections, vancomycin resistant enterococci infections, cystitis and pyelonephritis, renal calculi, prostatitis, peritonitis, complicated intra-abdominal infections (cIAI) and other inter-abdominal infections, dialysis-associated peritonitis, visceral abscesses, endocarditis, myocarditis, pericarditis, transfusion-associated sepsis, meningitis, encephalitis, brain abscess, osteomyelitis, arthritis, genital ulcers, urethritis, vaginitis, cervicitis, gingivitis, conjunctivitis, keratitis, endophthalmitisa, an infection in cystic fibrosis patients or an infection of febrile neutropenic patients.

Furthermore, the invention relates to the compound of formula (I) for use in the treatment and/or prophylaxis of bacterial infection. The invention relates to compound of formula (I) for the preparation of a medicament for use in the treatment and/or prophylaxis of bacterial infection. Also disclosed is a method for the treatment or prophylaxis of bacterial infection which method comprises administering an effective amount of a compound of formula (I), or pharmaceutically acceptable salt, or enantiomer or diastereomer thereof.

### EXAMPLES

The invention will be more fully understood by reference to the following examples. They should not, however, be construed as limiting the scope of the invention.

### ABBREVIATIONS

Abbreviations used herein are as follows:
- ACN or MeCN: acetonitrile
- AcOK: potassium acetate
- [α]D₂₀: optical rotation at 20 degrees Celsius
- B₂Pin₂: Bis(pinacolato)diboron
- BINAP: 2,2'-Bis(diphenylphosphino)-1,1'-binaphthalene
- BOMCl: benzylchloromethyl ester
- CAN: ceric ammonium nitrate
- CAMHB: Caution-Adjusted Mueller Hinton Broth
- calc'd: calculated
- CC₅₀: concentration results in the death of 50 percent of the cells
- CL: clearance
- Ct: cycle threshold
- d: day
- DBU: 1,8-Diazabicyclo[5.4.0]undec-7-ene
- DHP: dihydropyran
- DIAD: diisopropyl azodicarboxylate
- DIPEA: N,N-diisopropylethylamine
- EDTA-k2: edathamil
- EtOAc or EA: ethyl acetate
- FA: formic acid
- h(s) or hr(s): hour
- HATU:: 1-[Bis(dimethylamino)methylene]-1H-1,2,3-triazolo[4,5-b]pyridinium 3-oxid hexafluorophosphate
- HPLC:: high performance liquid chromatography
- HPLC-UV:: high performance liquid chromatography with ultraviolet detector
- IV: intravenous
- LAH: lithium aluminum hydride
- LDA: lithium diisopropylamide
- MIC: minimum inhibitory concentration
- Pd-Ad₂nBuP Biphenyl: Chloro[(di(1-adamantyl)-N-butylphosphine)-2-(2-aminobiphenyl)]palladium(II)
- PE: petroleum ether
- PBS: phosphate buffered saline
- Precat: precatalyst
- prep-HPLC: preparative high performance liquid chromatography
- qPCR: quantitative polymerase chain reaction
- qRT-PCR: quantitative real-time polymerase chain reaction
- rel: relative
- rt: room temperature
- rpm: revolutions per minute
- SFC: supercritical fluid chromatography
- SM: start material
- TLC: Thin Layer Chromatography
- UV: ultraviolet detector

### GENERAL EXPERIMENTAL CONDITIONS

Intermediates and final compounds were purified by flash chromatography using one of the following instruments: i) Biotage SP1 system and the Quad 12/25 Cartridge module, ii) ISCO combi-flash chromatography instrument. Silica gel Brand and pore size: i) KP-SIL 60 Å, particle size: 40-60 µm; ii) CAS registry NO: Silica Gel: 63231-67-4, particle size: 47-60 micron silica gel; iii) ZCX from Qingdao Haiyang Chemical Co., Ltd, pore: 200-300 or 300-400.

Intermediates and final compounds were purified by preparative HPLC on reversed phase column using X Bridge^{™} Perp C₁₈ (5 µm, OBD^{™} 30 × 100 mm) column or SunFire^{™} Perp C₁₈ (5 µm, OBD^{™} 30 × 100 mm) column.

Chiral Separation was conducted on Thar 350 preparative SFC using ChiralPak AD-10µ (200 × 50 mm I.D.) with mobile phase A for CO₂ and B for ethanol.LC/MS spectra were obtained using a Waters UPLC-SQD Mass. Standard LC/MS conditions were as follows (running time: 3 minutes):
Acidic condition: A: 0.1% formic acid and 1% acetonitrile in H₂O; B: 0.1% formic acid in acetonitrile;
Basic condition: A: 0.05% NH₃·H₂O in H₂O; B: acetonitrile.
Mass spectra (MS): generally only ions which indicate the parent mass are reported, and unless otherwise stated the mass ion quoted is the positive mass ion (M+H)⁺.
NMR Spectra were obtained using Bruker Avance 400MHz.

All reactions involving air-sensitive reagents were performed under an argon atmosphere. Reagents were used as received from commercial suppliers without further purification unless otherwise noted.

### PREPARATIVE EXAMPLES

### Intermediate A1

### tert-Butyl N-(3,4-dichloro-6-fluoro-9H-pyrido[2,3-b]indol-8-yl)-N-methyl-carbamate

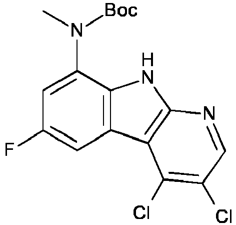

The titled compound was synthesized according to the following scheme:

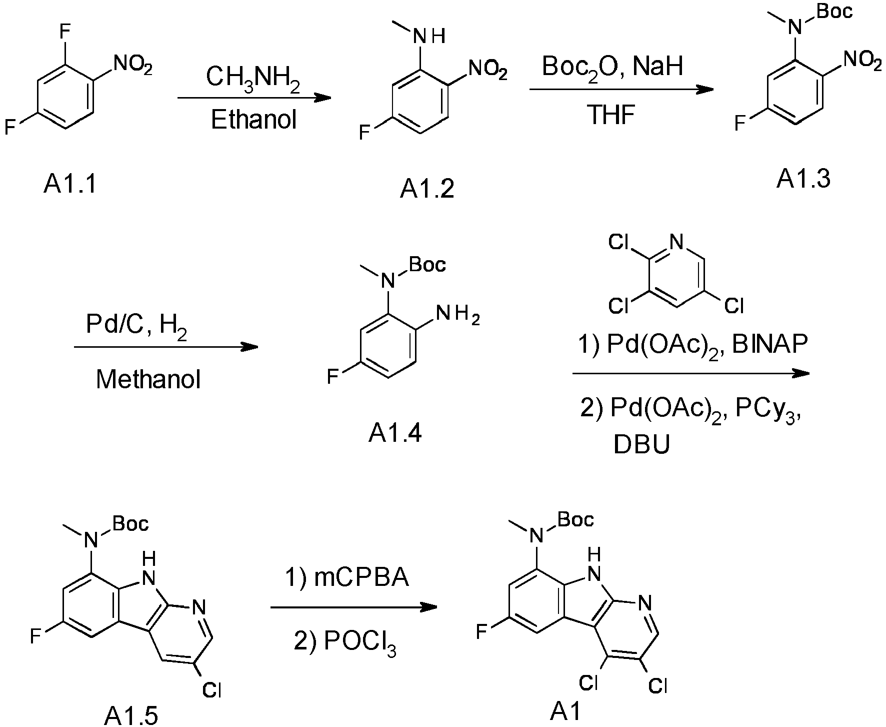

### Step (a) Preparation of 5-fluoro-N-methyl-2-nitro-aniline (compound A1.2)

Methylamine solution (355 g, 2.87 mol, 25% in ethanol) was added dropwise to 2,4-difluoronitrobenzene (147 g, 0.92 mol) at 0 °C over 15 min. After completion of the addition, the reaction mixture was stirred at 0 °C for 2 h. The solution was diluted with ethanol (500 mL) and poured into 2 L of ice-water. The resulting precipitates were collected by filtration and dried *in vacuo* to give 5-fluoro-N-methyl-2-nitro-aniline (143 g, 63% yield) as a yellow solid.

### Step (b) Preparation of tert-butyl N-(5-fluoro-2-nitro-phenyl)-N-methyl-carbamate (compound A1.3)

To the suspension of sodium hydride (141 g, 3.5 mol, 60 % dispersion in mineral oil) in dry tetrahydrofuran (2 L) was added 5-fluoro-N-methyl-2-nitro-aniline (60 g, 0.35 mol, compound A1.2) portion wise at 0 °C. The solution was stirred at 0 °C for 1 h. Then the solution of di-tert-butyl dicarbonate (115 g, 0.53 mol) in tetrahydrofuran (0.5 L) was added dropwise and the reaction continued for another 15 h at 15 °C. The reaction mixture was poured into 1.6 L of ice-water and extracted with EtOAc (1.6 L) two times. Combined organics was dried over anhy. Na₂SO₄, filtered, and concentrated *in vacuo.* The crude product was purified by flash chromatography (silica gel, 1-5% ethyl acetate in petroleum ether) to give *tert*-butyl N-(5-fluoro-2-nitro-phenyl)-N-methyl-carbamate (70 g, 73% yield) as a yellow solid. MS (ESI): 293.0 ([M+Na]+), 171.0 ([M-C₄H₈-CO₂+H]⁺).

### Step (c) Preparation of tert-butyl N-(2-amino-5-fluoro-phenyl)-N-methyl-carbamate (compound A1.4)

To the solution of *tert*-butyl N-(5-fluoro-2-nitro-phenyl)-N-methyl-carbamate (70 g, 259 mmol, compound A1.3) in MeOH (1 L) was added palladium on carbon (5 g, 10 wt. % loading). The reaction mixture was stirred at 16 °C for 18 h under H₂ atmosphere (50 psi). The remaining palladium catalyst was removed by filtration. The filtrate was concentrated *in vacuo* to give *tert-*butyl N-(2-amino-5-fluoro-phenyl)-N-methyl-carbamate (60 g, 96% yield) as a white solid. MS (ESI): 263.1 ([M+Na]+), 185.0 ([M-C₄H₈ +H]⁺), 141.0 ([M-C₄H₈-CO₂+H]⁺).

### Step (d) Preparation of tert-butyl N-(3-chloro-6-fluoro-9H-pyrido[2,3-b]indol-8-yl)-N-methylcarbamate (compound A1.5)

To the solution of *tert*-butyl N-(2-amino-5-fluoro-phenyl)-N-methyl-carbamate (80 g, 333 mmol, compound A1.4) and 2,3,5-trichloropyridine (66.8 g, 366 mmol, CAS: 16063-70-0) in dioxane (2 L) were added cesium carbonate (217 g, 666 mmol), palladium(II) acetate (3.74 g, 16.7 mmol), and BINAP ( 20.7 g, 33.3 mmol, CAS: 98327-87-8). Reaction continued at 120 °C for 16 h under nitrogen atmosphere. Then the reaction mixture was cooled to room temperature and diluted with EtOAc (800 mL). The precipitate was removed by filtration. The filtrate was concentrated *in vacuo* and the residue was purified by flash chromatography (silica gel, 0.2% to 5% EtOAc in petroleum ether) to give *tert*-butyl N-[2-[(3,5-dichloro-2-pyridyl)amino]-5-fluorophenyl]-N-methyl-carbamate (75 g, 58% yield). MS (ESI): 390.1 ([{³⁷Cl}M+H]⁺), 388.1 ([{³⁷Cl+³⁵Cl }M+H]⁺), 386.1 ([{³⁵Cl}M+H]⁺).

To the solution of *tert*-butyl N-[2-[(3,5-dichloro-2-pyridyl)amino]-5-fluoro-phenyl]-N-methyl-carbamate (5.0 g, 12.95 mmol) and DBU (3.94 g, 25.9 mmol, CAS: 6674-22-2) in the mixture of o-xylene (7.5mL) and N,N-Dimethylacetamide (7.5 mL) were added palladium(II) acetate (727 mg, 3.24 mmol) and tricyclohexylphosphine tetrafluoroborate (2.38 g, 6.48 mmol) under nitrogen atmosphere. The reaction mixture was stirred at 160 °C for 6 h, then cooled to room temperature and poured into water (100 mL). Then the mixture was extracted with EtOAc (200 mL) and the organic layer was collected and washed with water (50 mL) two times and brine (30 mL) two times, dried over anhy. Na₂SO₄ and filtered. The separated organic layer was concentrated *in vacuo* and the residue was purified by flash chromatography (silica gel, 0.5% to 20% EtOAc in dichloromethane) to give *tert*-butyl N-(3-chloro-6-fluoro-9H-pyrido[2,3-b]indol-8-yl)-N-methyl-carbamate (873 mg, 19.3% yield) as a yellow solid. MS (ESI): 352.1 ([{³⁷Cl}M+H]⁺), 350.1 ([{³⁵Cl}M+H]⁺).

### Step (e) Preparation of tert-butyl N-(3,4-dichloro-6-fluoro-9H-pyrido[2,3-b]indol-8-yl)-N-methyl-carbamate (compound A1)

To the solution of *tert*-butyl N-(3-chloro-6-fluoro-9H-pyrido[2,3-b]indol-8-yl)-N-methylcarbamate (4.8 g, 13.7 mmol, compound A1.5) in dichloromethane (200 mL) was added 3-chloroperbenzoic acid (9.47 g, 54.9 mmol) at 0 °C under nitrogen atmosphere. Reaction continued at 30 °C for 12 h. The reaction mixture was poured into sodium sulfite aqueous solution (10%, 150 mL) and stirred for 1 h followed by extraction with EtOAc (750 mL) three times. Combined organics was washed by sodium bicarbonate aqueous solution (5N, 200 mL) and brine (250 mL), then dried over anhy. Na₂SO₄, filtered, and concentrated *in vacuo* to give *tert*-butyl N-(3-chloro-6-fluoro-1-oxido-9H-pyrido[2,3-b]indol-1-ium-8-yl)-N-methyl-carbamate (4.8 g, yield: 96% yield) as a brown solid. MS (ESI): 368.1 ([{³⁷Cl}M+H]⁺), 366.1 ([{³⁵Cl}M+H]⁺).

To the solution of *tert*-butyl N-(3-chloro-6-fluoro-1-oxido-9H-pyrido[2,3-b]indol-1-ium-8-yl)-N-methyl-carbamate (4.8 g, 13.1 mmol) in dimethylformamide (100 mL) was added phosphorus(V) oxychloride (22.1 g, 144 mmol) dropwise at -5 °C. The mixture was stirred at - 5 °C to 0 °C for 1 h then poured into sodium bicarbonate aqueous solution (saturated, 350 mL) at 0 °C. The mixture was extracted by EtOAc (500 mL) three times and the combined organics were washed with water (200 mL) three times and brine (150 mL) two times, dried over anhy. Na₂SO₄, filtered, and concentrated *in vacuo.* The crude product was washed with MeOH (120 mL) to give tert-butyl N-(3,4-dichloro-6-fluoro-9H-pyrido[2,3-b]indol-8-yl)-N-methylcarbamate (2.16 g, 42.9% yield) as a pale yellow solid. MS (ESI): 388.1 ([{³⁷Cl+³⁷Cl }M+H]⁺), 386.1 ([{³⁷Cl+³⁵Cl }M+H]⁺), 384.1 ([{³⁵Cl+³⁵Cl }M+H]⁺). ¹H NMR (400 MHz, DMSO-*d6*) δppm: 12.55 (s, 1H), 8.65 (s, 1H), 8.05 (d, *J*=7.0 Hz, 1H) 7.49 (dd, *J*=10.3, 2.5 Hz, 1H) 3.26 (s, 3 H) 1.19 - 1.62 (m, 9H).

### Intermediate A2

### tert-Butyl N-(3,4-dichloro-6-fluoro-9H-pyrido[2,3-b]indol-8-yl)-N-ethyl-carbamate

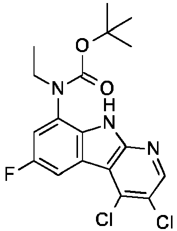

*tert-Butyl N-(3,4-dichloro-6-fluoro-9H-pyrido[2,3-b]indol-8-yl)-N-ethyl-carbamate* (**Intermediate A2**) was prepared in analogy to **Intermediate A1**, by replacing methylamine solution with ethylamine solution (30% in ethanol) in step (a). MS (ESI): 402.0 ([{³⁷Cl+³⁷Cl }M+H]⁺), 400.0 ([{³⁷Cl+³⁵Cl }M+H]⁺), 398.0 ([{³⁵Cl+³⁵Cl }M+H]⁺).¹H NMR (400 MHz, DMSO-d6) δ ppm: 12.57 (s, 1H), 8.65 (s, 1H), 8.08 (d, 1H) 7.42 (m, 1H) 3.67 (br, 2 H) 1.06 - 1.51 (m, 12H).

### Intermediate A3

### tert-Butyl N-(3,4-dichloro-5,6-difluoro-9H-pyrido[2,3-b]indol-8-yl)-N-methyl-carbamate

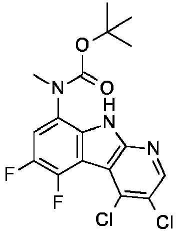

*tert*-Butyl N-(3,4-dichloro-5,6-difluoro-9H-pyrido[2,3-b]indol-8-yl)-N-methyl-carbamate (**Intermediate A3**) was prepared in analogy to **Intermediate A1**, by replacing 2,4-difluoronitrobenzene with 2,4,5-trifluoronitrobenzene in step (a). MS (ESI): 406.1 ([{³⁷Cl+³⁷Cl }M+H]⁺), 404.1 ([{³⁷Cl+³⁵Cl }M+H]⁺), 402.1 ([{³⁵Cl+³⁵Cl }M+H]⁺). ¹H NMR (400 MHz, DMSO-d6) δ ppm: 12.84 (br. s, 1H), 8.64 (s, 1H), 7.70 (m, 1H), 3.22 (s, 3H), 1.22-1.50 (m, 9H).

### Intermediate A4

### tert-Butyl N-(3,4-dichloro-6,7-difluoro-9H-pyrido[2,3-b]indol-8-yl)-N-methyl-carbamate

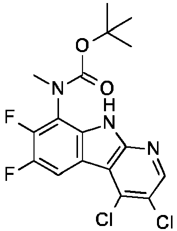

*tert*-Butyl N-(3,4-dichloro-6,7-difluoro-9H-pyrido[2,3-b]indol-8-yl)-N-methyl-carbamate (**Intermediate A4**) was prepared in analogy to **Intermediate A1**, by replacing 2,4-difluoronitrobenzene with 2,3,4-trifluoronitrobenzene in step (a). MS (ESI): 406.2

([{³⁷Cl+³⁷Cl }M+H]⁺), 404.2 ([{³⁷Cl+³⁵Cl }M+H]⁺), 402.1 ([{³⁵Cl+³⁵Cl }M+H]⁺). ¹H NMR (400 MHz, DMSO-*d6*) δ ppm: 12.61 (br. s, 1H), 8.59 (s, 1H), 8.26 (m, 1H), 3.04 (s, 3H), 1.22-1.50 (m, 9H).

### Intermediate A5

### tert-Butyl N-(3,4-dichloro-5,6-difluoro-9H-pyrido[2,3-b]indol-8-yl)-N-ethyl-carbamate

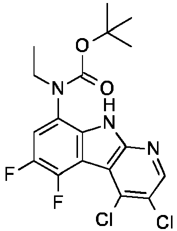

*tert*-Butyl N-(3,4-dichloro-5,6-difluoro-9H-pyrido[2,3-b]indol-8-yl)-N-ethyl-carbamate **(Intermediate A5**) was prepared in analogy to **Intermediate A1**, by replacing 2,4-difluoronitrobenzene with 2,4,5-trifluoronitrobenzene, and methylamine solution with ethylamine solution (30% in EtOH) in step (a). ¹H NMR (400 MHz, DMSO-*d6*) δ ppm: 12.86 (s, 1H), 8.67 (s, 1H), 7.65 (m, 1H) 3.62 (m, 2 H) 1.05 - 1.49 (m, 12 H). MS (ESI): 420.1 ([{³⁷Cl+³⁷Cl }M+H]⁺), 418.1 ([{³⁷Cl+³⁵Cl }M+H]⁺), 416.2 ([{³⁵Cl+³⁵Cl }M+H]⁺).

### Intermediate A6

### tert-Butyl N-(3-bromo-4,6-dichloro-9H-pyrido[2,3-b]indol-8-yl)-N-ethyl-carbamate

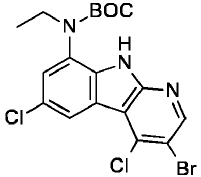

The title compound was prepared in analogy to **Intermediate A1**, by replacing 2,4-difluoronitrobenzene with 4-chloro-2-fluoro-1-nitrobenzene in step (a), replacing methylamine solution with ethylamine solution (30% in EtOH) in step (a), and replacing 2,3,5-trichloropyridine with 2,3,5-tribromopyridine in step (d). MS (ESI): 463.9 ([{³⁷Cl+³⁷Cl+⁸¹Br}M+H]⁺), 461.9 ([{³⁷Cl+³⁷Cl+⁷⁹Br}/{³⁵Cl+³⁷Cl+⁸¹Br}M+H]⁺), 459.9 ([{³⁵Cl+³⁷Cl+⁷⁹Br}/{³⁵Cl+³⁵Cl+⁸¹Br}M+H]⁺), 457.9 ([{³⁵Cl+³⁵Cl+⁷⁹Br }M+H]⁺).

### Intermediate B1

### [2-[(1-tert-Butoxycarbonyl-4-piperidyl)amino]pyrimidin-5-yl]boronic acid

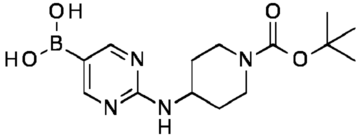

The titled compound was synthesized according to the following scheme:

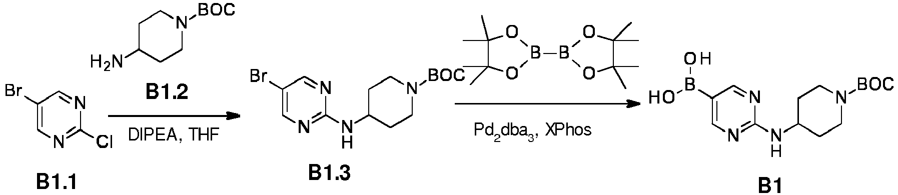

### Step (a) Preparation of tert-butyl 4-[(5-bromopyrimidin-2-yl)amino]piperidine-1-carboxylate (compound B1.3)

To the solution of 5-bromo-2-chloropyrimidine (3.0 g, 15.6 mmol, compound B1.1) and 4-amino-1-Boc-piperidine (4.7 g, 23.4 mmol, compound B1.2) in THF (5.0 mL) was added DIPEA (6.0 g, 48.9 mol). The reaction continued at 60 °C for 12 h. The mixture was cooled to room temperature and concentrated *in vacuo,* the residue was purified by column chromatography on silica gel (10% EtOAc in petroleum ether) to give *tert*-butyl 4-[(5-bromopyrimidin-2-yl)amino]piperidine-1-carboxylate (2.2 g, 39% yield) as a yellow oil. MS (ESI): 359.1 ([{⁸¹Br}M+H]⁺), 357.1 ([{⁷⁹Br}M+H]⁺).

### Step (b) Preparation of [2-[(1-tert-butoxycarbonyl-4-piperidyl)amino]pyrimidin-5-yl]boronic acid (compound B1)

To the solution of tert-butyl 4-[(5-bromopyrimidin-2-yl)amino]piperidine-1-carboxylate (2.0 g, 5.6 mmol, compound B1.4), bis(pinacolato)diboron (2.8 g, 11.2 mmol) in dioxane (8.0 mL) were added potassium acetate (1.45 g, 16.8 mmol), Pd₂dba₃ (514 mg, 0.56 mmol), and Xphos (267 mg, 0.56 mmol). The reaction continued under nitrogen atmosphere at 100 °C for 12 h. Then the mixture was cooled to room temperature, poured into water (100 mL), and extracted with EtOAc (100 mL) for three times. The organic layer was washed with water (50 mL), dried over Na₂SO₄, and concentrated *in vacuo* to obtain crude [2-[(1-*tert*-butoxycarbonyl-4-piperidyl)amino]pyrimidin-5-yl]boronic acid (1.2 g, 62% yield) as a white solid. MS (ESI): 323.2 ([M+H]⁺).

### Intermediate B2

### Methyl 2,2-dimethyl-3-[5-(4,4,5,5-tetramethyl-1,3,2-dioxaborolan-2-yl)pyrimidin-2-yl]oxy-propanoate

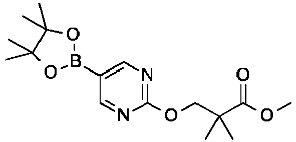

The titled compound was synthesized according to the following scheme:

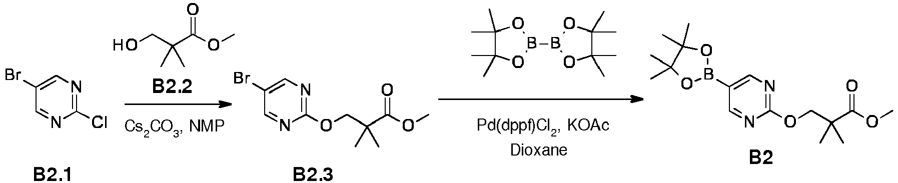

### Step (a) Preparation of methyl 3-(5-bromopyrimidin-2-yl)oxy-2,2-dimethyl-propanoate (compound B2.3)

To the solution of 5-bromo-2-chloropyrimidine (1.9 g, 10 mmol, compound B2.1) and methyl 2,2-dimethyl-3-hydroxypropionate (2.6 g, 20 mmol, compound B2.2) in NMP (10 mL) was added Cs₂CO₃ (4.2 g, 20 mmol). The reaction continued at 120 °C for 2 h in the microwave. The mixture was cooled to room temperature and poured into water (100 mL). The mixture was extracted by EtOAc (100 mL) for three times. The organic layer was dried over anhy. Na₂SO₄, filtered, and concentrated *in vacuo,* the residue was purified by column chromatography on silica gel (10% to 25% ethyl acetate in petroleum ether) to give methyl 3-(5-bromopyrimidin-2-yl)oxy-2,2-dimethyl-propanoate (0.88 g, 29% yield) as white solid. MS (ESI): 291.1 ([{⁸¹Br}M+H]⁺), 289.1 ([{⁷⁹Br}M+H]⁺).

### Step (b) Preparation of methyl 2,2-dimethyl-3-[5-(4,4,5,5-tetramethyl-1,3,2-dioxaborolan-2-yl)pyrimidin-2-yl]oxy-propanoate (compound B2)

To the solution of methyl 3-(5-bromopyrimidin-2-yl)oxy-2,2-dimethyl-propanoate (0.40 g, 1.4 mmol, compound B2.3), bis(pinacolato)diboron (0.42 g, 1.7 mmol) in dioxane (2 mL) were added potassium acetate (0.41 g, 4.2 mmol) and Pd(dppf)Cl₂ (51 mg, 0.07 mmol). The reaction continued under nitrogen atmosphere at 140 °C for 2 h in the microwave. Then the mixture was cooled to room temperature and concentrated *in vacuo,* the residue was purified by flash chromatography on silica gel (10% to 25% ethyl acetate in petroleum ether) to give methyl 2,2-dimethyl-3-[5-(4,4,5,5-tetramethyl-1,3,2-dioxaborolan-2-yl)pyrimidin-2-yl]oxy-propanoate (195 mg, 42% yield) as a white solid. MS (ESI): 337.3 ([M+H]⁺).

### Intermediate B3

### [2-[(1-Methoxycarbonylcyclopropyl)methylamino]pyrimidin-5-yl]boronic acid

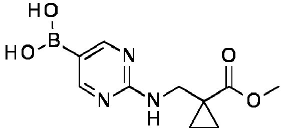

The title compound was prepared in analogy to **Intermediate B2,** by replacing methyl 2,2-dimethyl-3-hydroxypropionate (compound B2.2) with methyl 1-(aminomethyl) cyclopropanecarboxylatehydrochloride in step (a). MS (ESI): 252.0 ([M+H]⁺).

### Intermediate B4

### [2-[(3-methoxy-2,2-dimethyl-3-oxo-propyl)amino]pyrimidin-5-yl]boronic acid

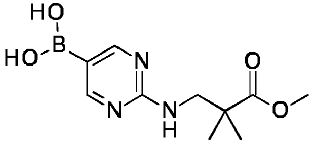

The title compound was prepared in analogy to **Intermediate B2,** by replacing methyl 2,2-dimethyl-3-hydroxypropionate (compound B2.2) with methyl 3-amino-2,2-dimethyl-propanoate in step (a). MS (ESI): 254.2 ([M+H]⁺).

### Intermediate B5

### [2-(1-tert-Butoxycarbonylpyrrolidin-3-yl)oxypyrimidin-5-yl]boronic acid

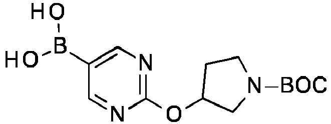

The title compound was prepared in analogy to **Intermediate B2,** by replacing methyl 2,2-dimethyl-3-hydroxypropionate with 1-Boc-3-hydroxypyrrolidine. MS (ESI): 310.1 ([M+H]⁺).

### Intermediate B6

### [2-(1-tert-Butoxycarbonylazetidin-3-yl)oxypyrimidin-5-yl]boronic acid

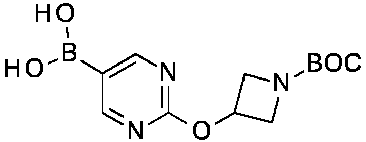

The title compound was prepared in analogy to **Intermediate B2,** by replacing methyl 2,2-dimethyl-3-hydroxypropionate with 1-Boc-3-hydroxyazetidine, Cs₂CO₃ with NaH, NMP with DMF, and the reaction was conducted for 3 h at room temperature instead of 120 °C for 2 h in step (a). MS (ESI): 296.1 ([M+H]⁺).

### Intermediate B7

### [2-[(1-tert-Butoxycarbonylpyrrolidin-3-yl)amino]pyrimidin-5-yl]boronic acid

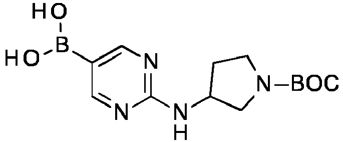

The title compound was prepared in analogy to **Intermediate B2,** by replacing methyl 2,2-dimethyl-3-hydroxypropionate with 1-Boc-3-aminopyrrolidine, Cs₂CO₃ with DIPEA, NMP with THF, and the reaction was conducted at 65 °C for 12 h instead of 120 °C for 2 h in step (a). MS (ESI): 309.2 ([M+H]⁺).

### Intermediate B8

### [2-(Tetrahydrofuran-3-ylamino)pyrimidin-5-yl]boronic acid

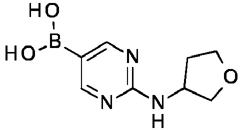

The title compound was prepared in analogy to **Intermediate B2,** by replacing methyl 2,2-dimethyl-3-hydroxypropionate with 3-aminotetrahydrofuran, Cs₂CO₃ with DIPEA, NMP with THF, and the reaction was conducted at 65 °C for 12 h instead of 120 °C for 2 h in step (a). MS (ESI): 210.2 ([M+H]⁺).

### Intermediate B9

### [2-[2-(tert-Butoxycarbonylamino)-2-methyl-propoxy]pyrimidin-5-yl]boronic acid

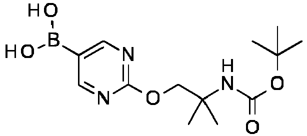

The title compound was prepared in analogy to **Intermediate B2,** by replacing methyl 2,2-dimethyl-3-hydroxypropionate with N-Boc-2-amino-2-methyl-1-propanol in step (a). MS (ESI): 312.2 ([M+H]⁺).

### Intermediate B10

### [2-[(tert-Butoxycarbonylamino)methyl]pyrimidin-5-yl]boronic acid

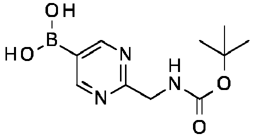

The titled compound was prepared in analogy to **Intermediate B4**, by replacing ethyl 1-(5-bromopyrimidin-2-yl)cyclopropanecarboxylate (compound B4.1) with N-Boc-5-bromo-2-pyrimidinemethanamine (Bepharm, catalog number: B220284). MS (ESI): 254.1 ([M+H]⁺).

### Intermediate B11

### [2-(Methoxymethoxymethyl)pyrimidin-5-yl]boronic acid

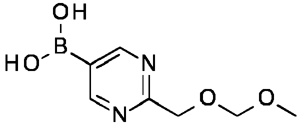

The titled compound was synthesized according to the following scheme:

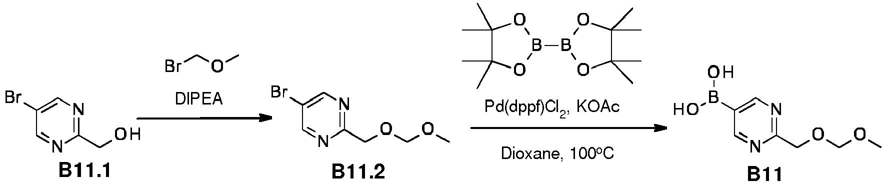

### Step (a) Preparation of 5-bromo-2-(methoxymethoxymethyl)pyrimidine (compound B11.2)

The mixture of DIPEA(1 mL), (5-bromopyrimidin-2-yl)methanol (compound B11.1, 125 mg, 661 µmol) and bromo(methoxy)methane (413 mg, 3.31 mmol) in 10 mL DCM was stirred at r.t. overnight. The reaction mixture was diluted with EtOAc (50 mL) and washed with water (50 mL). The organic layer was dried over anhy. Na₂SO₄, filtered, and concentrated in vacuo to give crude 5-bromo-2-(methoxymethoxymethyl)pyrimidine (160 mg, 99% yield) as light yellow oil.

### Step (b) Preparation of [2-(methoxymethoxymethyl)pyrimidin-5-yl]boronic acid (compound B11)

The titled compound was prepared in analogy to **Intermediate B4**, by replacing ethyl 1-(5-bromopyrimidin-2-yl)cyclopropanecarboxylate (compound B4.1) with 5-bromo-2-(methoxymethoxymethyl)pyrimidine (crude, prepared from step (a)). MS (ESI): 199.1 ([M+H]⁺).

### Intermediate C1

### tert-Butyl N-[[3-(trifluoromethyl)-1H-pyrazol-4-yl]methyl]carbamate

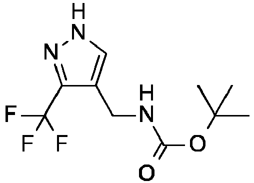

The titled compound was synthesized according to the following scheme:

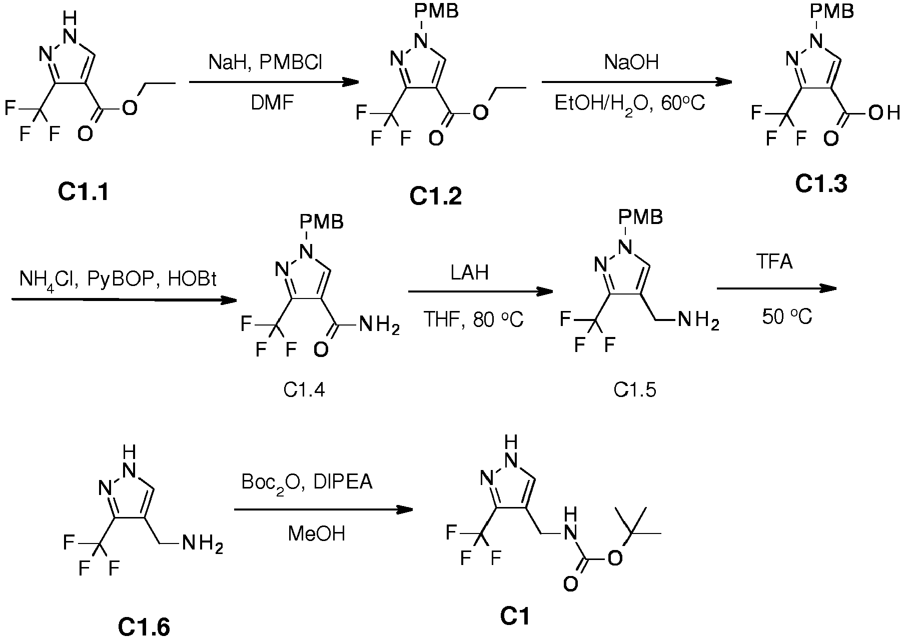

### Step (a) Preparation of ethyl 1-[(4-methoxyphenyl)methyl]-3-(trifluoromethyl)pyrazole-4-carboxylate (compound C1.2)

To a solution of ethyl 3-(trifluoromethyl)-1H-pyrazole-4-carboxylate (4 g, 19.2 mmol, compound C1.1) in DMF (50 mL) was added NaH (1.5 g, 38.5 mmol) at 0 °C. The mixture was stirred for 1 h before a solution of PMBCl (3.6 g, 23.1 mmol) was added. After the reaction was continued at room temperature for 3 h, it was poured into water (100 mL), and extracted with EtOAc (200 mL) twice. The combined organics were dried over anhy. Na₂SO₄,filtered, and concentrated *in vacuo,* and the residue was purified by flash chromatography on silica gel (petroleum ether : EtOAc = 20:1- 5:1) to give ethyl 1-[(4-methoxyphenyl)methyl]-3-(trifluoromethyl)pyrazole-4-carboxylate (3.8 g, 60% yield) as a yellow solid. MS (ESI): 329.2 ([M+H]⁺).

### Step (b) Preparation of 1-[(4-methoxyphenyl)methyl]-3-(trifluoromethyl)pyrazole-4-carboxylic acid (compound C1.3)

The solution of ethyl 1-[(4-methoxyphenyl)methyl]-3-(trifluoromethyl)pyrazole-4-carboxylate (3.8 g, 11.3 mmol) and NaOH (9 g, 225 mmol) in EtOH (30 mL) and water (15 mL) was stirred at 60 °C for 2 h. After the solution was cooled back to room temperature, aq. HCl solution (28% in water) was added to adjust pH to about 3. The mixture was then concentrated *in vacuo* and the residue was re-dissolved in EtOAc (100 mL), and washed with water (100 mL). The organic layer was dried over anhy. Na₂SO₄, filtered, and concentrated *in vacuo* to give 1-[(4-methoxyphenyl)methyl]-3-(trifluoromethyl)pyrazole-4-carboxylic acid (3.3 g, 97% yield) as a yellow solid. MS (ESI): 301.2 ([M+H]⁺).

### Step (c) Preparation of 1-[(4-methoxyphenyl)methyl]-3-(trifluoromethyl)pyrazole-4-carboxamide (compound C1.4)

The solution of 1-[(4-methoxyphenyl)methyl]-3-(trifluoromethyl)pyrazole-4-carboxylic acid (3.2 g, 10.7 mmol), NH₄Cl (2.85 g, 53.3 mmol), HOBt (1.73 g, 12.8 mmol), PyBOP (6.66 g, 12.8 mmol), and DIPEA (8.25 g, 64.0 mmol) in DMF (30.0 mL) was stirred at 30 °C under N₂ atmosphere for 1 h. Then the mixture was poured into water (100 mL) and extracted with EtOAc (100 mL) three times. The combined organics was dried over anhy. Na₂SO₄, filtered, and concentrated *in vacuo,* and the residue was purified by prep-HPLC to give 1-[(4-methoxyphenyl)methyl]-3-(trifluoromethyl)pyrazole-4-carboxamide (3.0 g, 94% yield) as yellow oil. MS (ESI): 300.2 ([M+H]⁺).

### Step (d) Preparation of [1-[(4-methoxyphenyl)methyl]-3-(trifluoromethyl)pyrazol-4-yllmethanamine (compound C1.5)

To a solution of 1-[(4-methoxyphenyl)methyl]-3-(trifluoromethyl)pyrazole-4-carboxamide (2.9 g, 9.7 mmol) in THF (25 mL) was added LAH (1.84 g, 48.5 mmol) at 0 °C. The reaction was stirred at 80 °C for 2 h under N₂ atmosphere. After the mixture was cooled to 0 °C, water (1.8 mL), 15% aqueous NaOH (3.6 mL), and then water (1.8 mL) were added sequentially. Then the mixture was diluted with EtOAc (200 mL) and filtered. The filtrate was dried over anhy. Na₂SO₄, filtered, and concentrated *in vacuo* to give [1-[(4-methoxyphenyl)methyl]-3-(trifluoromethyl)pyrazol-4-yl]methanamine (2.6 g, 94% yield) as clear oil. MS (ESI): 286.2 ([M+H]⁺).

### Step (e) Preparation of [3-(trifluoromethyl)-1H-pyrazol-4-yl]methanamine (compound C1.6)

To a solution of [1-[(4-methoxyphenyl)methyl]-3-(trifluoromethyl)pyrazol-4-yl]methanamine (2.5 g, 8.8 mmol) in MeCN (40 mL) and water (10 mL) was added ceric ammonium nitrate (19.2 g, 35.0 mmol). The reaction was stirred at room temperature for 2 h. The mixture was basified to pH ~ 9 with NaOH solid and then diluted with MeOH (400 mL). The mixture was filtered and the filtrate was concentrated *in vacuo* to give crude [3-(trifluoromethyl)-1H-pyrazol-4-yl]methanamine (1.5 g) as a yellow solid.

### Step (f) Preparation of tert-butyl N-[[3-(trifluoromethyl)-1H-pyrazol-4-yl]methyl]carbamate (compound C1)

The mixture of crude [3-(trifluoromethyl)-1H-pyrazol-4-yl]methanamine (1.5 g, 8.5 mmol), Boc₂O (2.78 g, 12.7 mmol) and DIPEA (3.28 g, 25.4 mmol) in MeOH (50 mL) was stirred at room temperature for 1 h. After the mixture was concentrated *in vacuo,* the residue was resuspended in water (100 mL), and extracted with EtOAc (100 mL) three times. The combined organics was washed with brine (50 mL) twice, dried over anhy. Na₂SO₄, filtered, and concentrated *in vacuo,* and the residue was purified by prep-HPLC to give *tert*-butyl N-[[3-(trifluoromethyl)-1H-pyrazol-4-yl]methyl]carbamate (1.05 g, 47% yield) as a white solid. MS (ESI): 266.3 ([M+H]⁺).

### Intermediate C2

### tert-Butyl 3-(trifluoromethyl)-4,6-dihydro-1H-pyrrolo[3,4-c]pyrazole-5-carboxylate

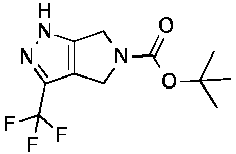

The titled compound was synthesized according to the following scheme:

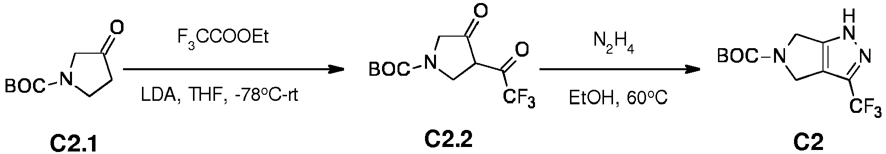

### Step (a) Preparation of tert-butyl 3-oxo-4-(2,2,2-trifluoroacetyl)pyrrolidine-1-carboxylate (compound C2.2)

To the solution of N-Boc-3-pyrrolidinone (1.0 g, 5.4 mmol) in THF (20 mL) was added LDA (6.5 mL, 1 M in THF/hexanes, 6.5 mmol) and ethyl trifluoroacetate (1.0 g, 7.0 mmol) at - 78 °C. Reaction was allowed to warm up to room temperature slowly and stirred at room temperature for 12 h. Then the mixture was poured into water (100 mL) and extracted with EtOAc (100 mL) three times. The combined organics was washed with brine (100 mL), dried over anhy. Na₂SO₄, filtered, and concentrated *in vacuo* to give crude *tert*-butyl 3-oxo-4-(2,2,2-trifluoroacetyl)pyrrolidine-1-carboxylate (1.7 g, quantitive yield) as yellow oil, which was used in the next step without purification.

### Step (b) Preparation of tert-butyl 3-(trifluoromethyl)-4,6-dihydro-1H-pyrrolo[3,4-c]pyrazole-5-carboxylate (compound C2)

The solution of tert-butyl 3-oxo-4-(2,2,2-trifluoroacetyl)pyrrolidine-1-carboxylate (1.7 g, 5.4 mmol, prepared from step (a)) and hydrazine monohydrate (0.35 g, 7.0 mmol) in EtOH (20 mL) was stirred at 60 °C for 12 h. Then the mixture was cooled to room temperature, concentrated *in vacuo,* and then dissolved in EtOAc (100 mL). The solution was washed with brine (100 mL), dried over anhy. Na₂SO₄, filtered, and concentrated *in vacuo,* the residue was purified by prep-HPLC to give *tert*-butyl 3-(trifluoromethyl)-4,6-dihydro-1H-pyrrolo[3,4-c]pyrazole-5-carboxylate (160 mg, 11% yield).
¹H NMR (400 MHz, DMSO-*d6*) δ ppm: 13.65 (s, 1H), 4.42-4.53 (m, 4H), 1.50 (s, 9H). MS (ESI): 278.1 ([M+H]⁺).

### Intermediate C3

### tert-Butyl 3-cyano-4,6-dihydro-1H-pyrrolo[3,4-c]pyrazole-5-carboxylate

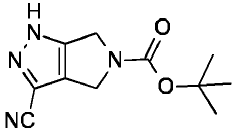

The titled compound was synthesized according to the following scheme:

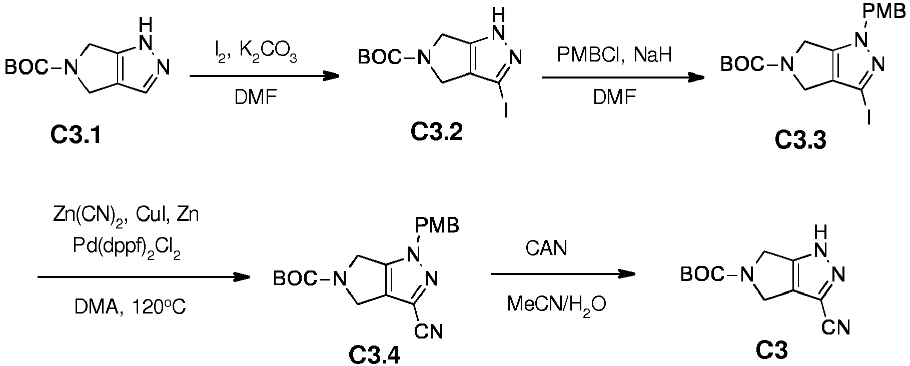

### Step (a) Preparation of tert-butyl 3-iodo-4,6-dihydro-1H-pyrrolo[3,4-c]pyrazole-5-carboxylate (compound C3.2)

To a solution of *tert*-butyl 4,6-dihydropyrrolo[3,4-c]pyrazole-5(1H)-carboxylate (1 g, 4.8 mmol), K₂CO₃ (1.4 g, 10.6 mmol) in DMF (20 mL) was added I₂ (2.4 g, 9.6 mmol) in DMF (10 mL) at room temperature. Reaction continued for 4 h, then another portion of K₂CO₃ (1.4 g, 10.6 mmol) and I₂ (2.4 g, 9.6 mmol) was added. Reaction continued for another 14 h, and was quenched with saturated Na₂S₂O₃ solution (20 mL). The mixture was diluted with water (100 mL), extracted with EtOAc (80 mL) three times. The combined organics was washed with brine (50 mL) twice, dried over anhy. Na₂SO₄, filtered, and concentrated *in vacuo,* the residue was purified by flash chromatography to give *tert*-butyl 3-iodo-4,6-dihydro-1H-pyrrolo[3,4-c]pyrazole-5-carboxylate (800 mg, 50% yield) as yellow solid. MS (ESI): 336.0 ([M+H]⁺).

### Step (b) Preparation of tert-butyl 3-iodo-1-[(4-methoxyphenyl)methyl]-4,6-dihydropyrrolo[3,4-clpyrazole-5-carboxylate (compound C3.3)

To a solution of *tert*-butyl 3-iodo-4,6-dihydro-1H-pyrrolo[3,4-c]pyrazole-5-carboxylate (700 mg, 2.1 mmol) in DMF (20 mL) were added NaH (125 mg, 3.1 mmol) and PMBCl (391 mg, 2.5 mmol) at 0 °C. Reaction continued at room temperature for 18 h. The solution was quenched with saturated NH₄Cl (20 mL), diluted with water (100 mL), and extracted with EtOAc (50 mL) three times. The combined organics was washed with brine (30 mL), dried over anhy. Na₂SO₄, filtered, and concentrated *in vacuo.* The crude product was purified by silica gel chromatography to give *tert*-butyl 3-iodo-1-[(4-methoxyphenyl)methyl]-4,6-dihydropyrrolo[3,4-c]pyrazole-5-carboxylate (900 mg, 95% yield) as yellow oil. MS (ESI): 456.1 ([M+H]⁺).

### Step (c) Preparation of tert-butyl 3-cyano-1-[(4-methoxyphenyl)methyl]-4,6-dihydropyrrolo[3,4-clpyrazole-5-carboxylate (compound C3.4)

To the solution of *tert*-butyl 3-iodo-1-[(4-methoxyphenyl)methyl]-4,6-dihydropyrrolo[3,4-c]pyrazole-5-carboxylate (900 mg, 1.98 mmol), Zn(CN)₂ (459 mg, 3.96 mmol), Zn (32 mg, 0.5 mmol), CuI (564 mg, 2.97 mmol) in DMA (20 mL) was added Pd(dppf)₂Cl₂ (163 mg, 0.20 mmol) at room temperature. Reaction continued at 120 °C for 18 h under N₂ atmosphere. The mixture was cooled to room temperature and poured into saturated NH₄Cl (50 mL), diluted with water (100 mL), and extracted with EtOAc (50 mL) three times. The combined organics was washed with brine (50 mL), dried over anhy. Na₂SO₄, and concentrated *in vacuo.* The crude product was purified by silica gel chromatography to give tert-butyl 3-cyano-1-[(4-methoxyphenyl)methyl]-4,6-dihydropyrrolo[3,4-c]pyrazole-5-carboxylate (500 mg, 71% yield) as colorless oil. MS (ESI): 355.2 ([M+H]⁺).

### Step (d) Preparation of tert-butyl 3-cyano-4,6-dihydro-1H-pyrrolo[3,4-c]pyrazole-5-carboxylate (compound C3)

To the solution of *tert*-butyl 3-cyano-1-[(4-methoxyphenyl)methyl]-4,6-dihydropyrrolo[3,4-c]pyrazole-5-carboxylate (500 mg, 1.4 mmol) in the mixture of MeCN (20 mL) and water (4 mL) was added CAN (3.8 g, 7.0 mmol) at room temperature. The solution was stirred for 4 h before diluted with water (50 mL) and extracted with EtOAc (50 mL) three times. The combined organics was washed with brine (50 mL), dried over anhy. Na₂SO₄, filtered, and concentrated *in vacuo.* The crude product was purified by silica gel chromatography to give *tert-*butyl 3-cyano-4,6-dihydro-1H-pyrrolo[3,4-c]pyrazole-5-carboxylate (150 mg, 46% yield) as a white solid. MS (ESI): 235.1 ([M+H]⁺).

### Intermediate C4

### tert-Butyl 3-cyano-1,4,5,7-tetrahydropyrazolo[3,4-c]pyridine-6-carboxylate

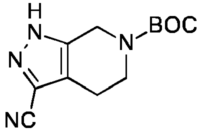

The titled compound was synthesized in analogy to **Intermediate C3**, by replacing *tert-*butyl 4,6-dihydropyrrolo[3,4-c]pyrazole-5(1H)-carboxylate with *tert*-Butyl 4,5-dihydro-1H-pyrazolo[3,4-c]pyridine-6(7H)-carboxylate-6(7H)-carboxylate in step (a). MS (ESI): 249.1 ([M+H]⁺).

### Example 1.01

### 5-[8-(Ethylamino)-6-fluoro-4-[3-(trifluoromethyl)pyrazol-1-yl]-9H-pyrido[2,3-b]indol-3-yl]pyridine-3-carbonitrile

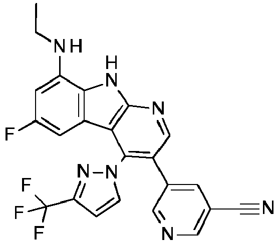

### Step (a) Preparation of tert-butyl N-[3-chloro-6-fluoro-4-[3-(trifluoromethyl)pyrazol-1-yl]-9H-pyrido[2,3-b]indol-8-yl]-N-ethyl-carbamate

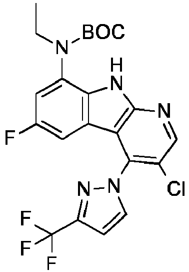

To the solution of *tert*-butyl N-(3,4-dichloro-6-fluoro-9H-pyrido[2,3-b]indol-8-yl)-N-ethyl-carbamate (**Intermediate A2,** 80 mg, 0.2 mmol, as the **"CORE"** in table 1) in DMSO (1 mL) were added 3-(trifluoromethyl)pyrazole (54.4 mg, 0.40 mmol, as the **"NUCLEOPHILE"** in table 1) and K₂CO₃ (82.9 mg, 0.60 mmol). The mixture was stirred at 120 °C for 12 h. The reaction mixture was cooled to room temperature, filtered through thin celite pad, and concentrated *in vacuo.* The residue was purified by prep-HPLC to give *tert*-butyl N-[3-chloro-6-fluoro-4-[3-(trifluoromethyl)pyrazol-1-yl]-9H-pyrido[2,3-b]indol-8-yl]-N-ethyl-carbamate (85 mg, 86% yield) as a white solid. MS (ESI): 500.2 ([{³⁷Cl}M+H]⁺), 498.2 ([{³⁵Cl}M+H]⁺).

### Step (b) Preparation of tert-butyl N-[3-(5-cyano-3-pyridyl)-6-fluoro-4-[3-(trifluoromethyl)prazol-1-yl]-9H-pyrido[2,3-b]indol-8-yl]-N-ethyl-carbamate

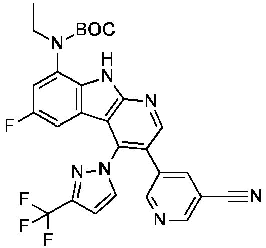

A solution of tert-butyl N-[3-chloro-6-fluoro-4-[3-(trifluoromethyl)pyrazol-1-yl]-9H-pyrido[2,3-b]indol-8-yl]-N-ethyl-carbamate (60 mg, 0.12 mmol), 3-cyanopyridine-5-boronic acid pinacol ester (55.7 mg, 0.24 mmol, as the **"BORONIC REAGENT"** in table 1), Pd₂dba₃ (21.9 mg, 0.024 mmol) and XPhos (11.4 mg, 0.024 mmol), as the **"CATALYST"** in table 1, and potassium phosphate tribasic (144.7 mg, 0.69 mmol) in dioxane (4 mL) and water (0.4 mL) was stirred at 100 °C for 12 h under N₂ atmosphere. The mixture was cooled to room temperature and poured into water (50 mL). Then the mixture was extracted with EtOAc (50 mL) three times. The combined organics was dried over anhy. Na₂SO₄, filtered, and concentrated *in vacuo.* The residue was purified by prep-HPLC to give *tert*-butyl N-[3-(5-cyano-3-pyridyl)-6-fluoro-4-[3-(trifluoromethyl)pyrazol-1-yl]-9H-pyrido[2,3-b]indol-8-yl]-N-ethyl-carbamate (45.3 mg, 66% yield) as a yellow oil. MS (ESI): 566.3 ([M+H]⁺).

### Step (c) Preparation of 5-[8-(ethylamino)-6-fluoro-4-[3-(trifluoromethyl)pyrazol-1-yl]-9H-pyrido[2,3-b]indol-3-yl]pyridine-3-carbonitrile

To the solution of *tert*-butyl N-[3-(5-cyano-3-pyridyl)-6-fluoro-4-[3-(trifluoromethyl)pyrazol-1-yl]-9H-pyrido[2,3-b]indol-8-yl]-N-ethyl-carbamate (45 mg, 0.08 mmol) in DCM (3 mL) was added TFA (2 mL). The solution was stirred at room temperature for 2 h before it was concentrated *in vacuo.* The residue was purified by prep-HPLC to give 5-[4-[(3*R*)-3-aminopyrrolidin-1-yl]-6-fluoro-8-(methylamino)-9H-pyrido[2,3-b]indol-3-yl]pyridine-3-carbonitrile (30 mg, 81% yield) as a yellow solid. ¹H NMR (400MHz, DMSO-*d6*) δ ppm: 12.19 (s, 1H), 8.98 (d, J=2.0Hz, 1H), 8.76 (s, 1H), 8.57 (d, J=2.0Hz, 1H), 8.33 (s, 1H), 8.12 (s, 1H), 7.13 (d, J=2.4Hz, 1H), 6.48 (m, 1H), 5.92 (m, 1H), 5.81 (m, 1H), 3.25 (m, 2H), 1.32 (t, J=6.8Hz, 3H). MS (ESI): 466.1 ([M+H]⁺).

The following examples were prepared in analogy to **Example 1.01,** replacing *tert*-butyl N-(3,4-dichloro-6-fluoro-9H-pyrido[2,3-b]indol-8-yl)-N-ethyl-carbamate (**Intermediate A2**) as the **"CORE"** in step (a), 3-(trifluoromethyl)pyrazole as the **"NUCLEOPHILE"** in step (a), and 3-cyanopyridine-5-boronic acid pinacol ester as the **"BORONIC REAGENT"** in step (b), Pd₂dba₃ and XPhos as the **"CATALYST"** in step (b) with the reagents indicated in Table 1 respectively.

**Table 1. Compound synthesis and characterization**

| **No.** | **Compound Name and Structure** | **CORE, NUCLEOPHILE, BORONIC REAGENT, and CATALYST** | **¹H NMR and MS (ESI)** |
|---|---|---|---|
| 1.02 | **6-Fluoro-3-(2-methoxypyrimidin-5-yl)-N-methyl-4-[3-(trifluoromethyl)pyrazol-1-yl]-9H-pyrido[2,3-b]indol-8-amine** | **CORE:** Intermediate A1 | **¹H NMR** (400 MHz, MeOH-*d4*) δ ppm: 8.63 (s, 1H), 8.40 (s, 2H), 8.06 (s, 1H), 6.97 (s, 1H), 6.5 (m, 1H), 5.95 (m, 1H), 4.04 (s, 3H), 2.99 (s, 3H). |
| | | **NUCLEOPHILE:** 3-(Trifluoromethyl)pyrazol e | |
| | | **BORONIC REAGENT:** 2-Methoxypyrimidine-5-boronic acid | |
| | | | **MS** (ESI): 458.1 ([M+H]⁺). |
| | 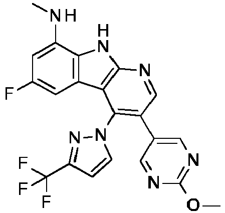 | **CATALYST:** Pd₂dba₃ and XPhos | |
| 1.03 | **5-[8-(Ethylamino)-6-fluoro-4-[4-(trifluoromethyl)pyrazol-1-yl]-9H-pyrido[2,3-b]indol-3-yl]pyridine-3-carbonitrile** | **CORE:** Intermediate A2 | **¹H NMR** (400 MHz, DMSO-*d6*) δ ppm: 12.19 (s, 1H), 9.00 (d, J=2.0Hz, 1H), 8.82 (s, 1H), 8.77 (s, 1H), 8.58 (s, 1H), 8.44 (s, 1H), 8.12 (d, J=2.0 Hz, 1H), 6.51 (m, 1H), 5.84 (m, 1H), 3.26 (q, J=7.2 Hz, 2H), 1.32 (t, J=7.2 Hz, 3H). |
| | | **NUCLEOPHILE:** 4-(Trifluoromethyl)-1H-pyrazole | |
| | 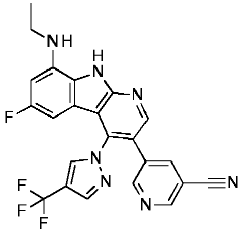 | **BORONIC REAGENT:** 3-Cyanopyridine-5-boronic acid pinacol ester | |
| | | **CATALYST:** Pd₂dba₃ and XPhos | **MS** (ESI): 466.1 ([M+H]⁺). |
| 1.04 | **N-Ethyl-6-fluoro-3-(2-methoxypyrimidin-5-yl)-4-[3-(trifluoromethyl)pyrazol-1-yl]-9H-pyrido[2,3-b]indol-8-amine** | **CORE:** Intermediate A2 | **¹H NMR** (400 MHz, DMSO-d6) δ ppm: 8.70 (s, 1H), 8.38 (s, 2H), 8.29 (s, 1H), 7.10 (s, 1H), 6.46 (m, 1H), 5.72 (m, 1H), 3.92 (s, 3H), 3.24 (m, 2H), 1.32 (m, 3H). |
| | | **NUCLEOPHILE:** 3-(Trifluoromethyl)pyrazol e | |
| | 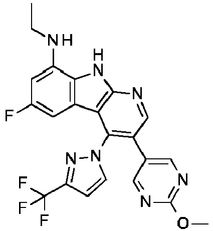 | **BORONIC REAGENT:** 2-Methoxypyrimidine-5-boronic acid | |
| | | | **MS** (ESI): 472.2 ([M+H]⁺) |
| | | **CATALYST:** Pd₂dba₃ and XPhos | |
| 1.05 | **5-[4-(3-Cyanopyrazol-1-yl)-8-(ethylamino)-6-fluoro-9H-pyrido[2,3-b]indol-3-** | **CORE:** Intermediate A2 | **¹H NMR** (400 MHz, DMSO-*d6*) δ ppm: 12.22 (s, 1H), 9.01 (d, J=1.2 Hz, 1H), |
| | | **NUCLEOPHILE:** 1H- | |
| | **yl]pyridine-3-carbonitrile** | Pyrazole-3 -carbonitrile | 8.77 (s, 1H), 8.56 (d, J=2.4Hz, 1H), 8.43 (d, J=2.4Hz, 1H), 8.21 (s, 1H), 7.37 (d, J=2.4Hz, 1H), 6.52 (m, 1H), 5.76 (m, 1H), 3.27 (q, J=7.2 Hz, 2H), 1.33 (t, J=7.2 Hz, 3H). |
| | 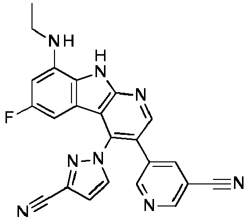 | **BORONIC REAGENT:** 3-Cyanopyridine-5-boronic acid pinacol ester | |
| | | **CATALYST:** Pd₂dba₃ and XPhos | |
| | | | **MS** (ESI): 423.2 ([M+H]⁺). |
| 1.06 | **3-(2-Aminopyrimidin-5-yl)-N-ethyl-6-fluoro-4-[3-(trifluoromethyl)pyrazol-1-yl]-9H-pyrido[2,3-b]indol-8-amine** | **CORE:** Intermediate A2 | **¹H NMR** (400 MHz, DMSO-d6) δ ppm: 12.01 (s, 1H), 8.66 (s, 1H), 8.30 (d, 1H), 8.01 (s, 2H), 7.13 (d, 1H), 6.80 (s, 2H), 6.47 (m, 1H), 5.86 (m, 1H), 5.67 (m, 1H), 3.22-3.28 (m, 2H), 1.32 (m, 3H). |
| | | **NUCLEOPHILE:** 3-(Trifluoromethyl)pyrazol e | |
| | 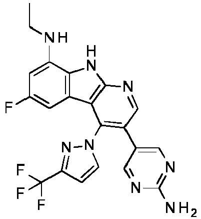 | **BORONIC REAGENT:** 2-(*tert-*Butoxycarbonylamino)py rimidine-5-boronic acid pinacol ester | |
| | | | **MS** (ESI): 457.2 ([M+H]⁺). |
| | | **CATALYST:** Pd₂dba₃ and XPhos | |
| 1.07 | **N-ethyl-6-fluoro-3-(6-methyl-3-pyridyl)-4-[3-(trifluoromethyl)pyrazol-1-yl]-9H-pyrido[2,3-b]indol-8-amine** | **CORE:** Intermediate A2 | **¹H NMR** (400 MHz, DMSO-d6) δ ppm: 12.08 (s, 1H), 8.67 (s, 1H), 8.19-8.30 (m, 2H), 7.46 (m, 1H), 7.24 (d, 1H), 7.08 (d, 1H), 6.48 (m, 1H), 5.85-5.91 (m, 1H), 5.69 (m, 1H), 3.25-3.28 (q, 2H), 2.47 (s, 3H), 1.32 (t, 3H). |
| | | **NUCLEOPHILE:** 3-(Trifluoromethyl)pyrazol e | |
| | | **BORONIC REAGENT:** 6-Methylpyridine-3-boronic acid | |
| | | **CATALYST:** Pd₂dba₃ and XPhos | **MS** (ESI): 455.2 ([M+H]⁺). |
| | 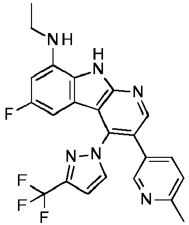 | | |
| 1.08 | **5-[6-Fluoro-8-(methylamino)-4- [4-(trifluoromethyl)pyrazol-1-yl]-9H-pyrido[2,3-b]indol-3-yl]pyridine-3-carbonitrile** | **CORE:** Intermediate A1 | **¹H NMR** (400 MHz, DMSO-d6) δ ppm: 12.10 (s, 1H), 8.99 (s, 1H), 8.81 (s, 1H), 8.77 (s, 1H), 8.58 (d, J=2.0Hz, 1H), 8.43 (s, 1H), 8.12 (d, J=2.0Hz, 1H), 6.49 (m, 1H), 6.03 (d, J=4.4 Hz, 1H), 5.83 (m, 1H), 2.92 (d, J=4.0Hz, 3H). |
| | | **NUCLEOPHILE:** 4-(Trifluoromethyl)-1H-pyrazole | |
| | 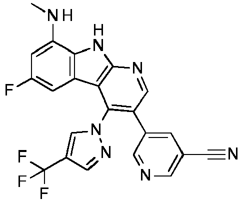 | **BORONIC REAGENT:** 3-Cyanopyridine-5-boronic acid pinacol ester | |
| | | **CATALYST:** Pd₂dba₃ and XPhos | **MS** (ESI): 452.2 ([M+H]⁺). |
| 1.09 | **5-[4-(4-Cyanopyrazol-1-yl)-6-fluoro-8-(methylamino)-9H-pyrido[2,3-b]indol-3-yl]pyridine-3-carbonitrile** | **CORE:** Intermediate A1 | **¹H NMR** (400 MHz, DMSO-d6) δ ppm: 12.18 (s, 1H), 9.01 (s, 1H), 8.77 (s, 1H), 8.56 (s, 1H), 8.43 (d, J=2.0Hz, 1H), 8.21 (s, 1H), 7.37 (d, J=2.4Hz, 1H), 6.51 (d, J=12.0Hz, 1H), 6.09 (br, 1H), 5.77 (d, J=8.8Hz, 1H), 2.93 (s, 3H). |
| | | **NUCLEOPHILE:** 1H Pyrazole-4-carbonitrile | |
| | 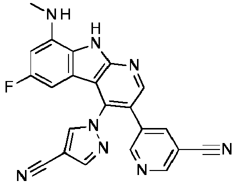 | **BORONIC REAGENT:** 3-Cyanopyridine-5-boronic acid pinacol ester | |
| | | **CATALYST:** Pd₂dba₃ and XPhos | |
| | | | **MS** (ESI): 409.2 ([M+H]⁺). |
| 1.10 | **N-Ethyl-6-fluoro-3-[2-(4-piperidylamino)pyrimidin-5-yl]-4-[3-(trifluoromethyl)pyrazol-1-yl]-9H-pyrido[2,3-b]indol-8-amine** | **CORE:** Intermediate A2 | **¹H NMR** (400 MHz, DMSO-d6) δ ppm: 12.05 (br. s., 1H), 8.66 (s, 1H), 8.34 (d, 1H), 8.06 (br. s., 1H), 7.42 (d, 1H), 7.15 (d, 1H), 6.47 (m, 1H), 5.87 (br. s., 1H), 5.66 (m, 1H), 5.27-5.36 (m, 1H), 3.25 (br. s., 2H), 3.10 (br. s., 1H), 2.65-2.76 (m, 2H), 1.97-2.06 (m, 2H), 1.87 (d, 2H), 1.46 (d, 2H), 1.27-1.34 (m, 3H). |
| | | **NUCLEOPHILE:** 3-(Trifluoromethyl)pyrazole | |
| | 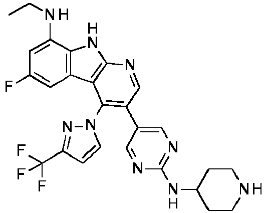 | **BORONIC REAGENT:** Intermediate B1 | |
| | | **CATALYST:** Pd₂dba₃ and XPhos | |
| | | | **MS** (ESI): 540.3 ([M+H]⁺). |
| 1.11 | **N-Ethyl-6-fluoro-3-(1,5-naphthyridin-3-yl)-4- [3-(trifluoromethyl)pyrazol-1-yl]-9H-pyrido[2,3-b]indol-8-amine** | **CORE:** Intermediate A2 | **¹H NMR** (400 MHz, DMSO-d6) δ ppm: 12.17 (s, 1H), 9.04 (m, 1H), 8.91 (s, 1H), 8.70 (d, 1H), 8.44 (d, 1H), 8.29 (m, 2H), 7.82 (m, 1H), 7.07 (d, 1H), 6.51 (m, 1H), 5.90 (br. s., 1H), 5.80 (m, 1H), 3.28 (d, 2H), 1.34 (t, 3H). |
| | | **NUCLEOPHILE:** 3-(Trifluoromethyl)pyrazol e | |
| | 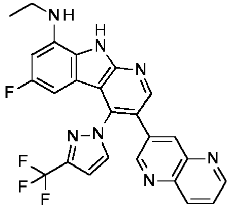 | **BORONIC REAGENT:** 3-(4,4,5,5-Tetramethyl-1, 3,2-dioxaborolan-2-yl)-1, 5-naphthyridine (Matrix Scientific: Catalog # 059382) | |
| | | | MS (ESI): 492.2 ([M+H]⁺). |
| | | **CATALYST:** Pd₂dba₃ and XPhos | |
| 1.12 | **N-Ethyl-6-fluoro-3-pyrimidin-5-yl-4-[3-(trifluoromethyl)pyrazol-1-yl]-9H-pyrido[2,3-b]indol-8-amine** | **CORE:** Intermediate A2 | **¹H NMR** (400 MHz, DMSO-d6) δ ppm: 12.18 (br. s., 1H), 9.14 (s, 1H), 8.78 (s, 1H), 8.60 (s, 2H), 8.37 (d, 1H), 7.14 (d, 1H), 6.51 (m, 1H), 5.90 (br. s., 1H), 5.78 (m, 1H), 3.22-3.29 (m, 2H), 1.33 (m, 3H). |
| | | **NUCLEOPHILE:** 3-(Trifluoromethyl)pyrazol e | |
| | | **BORONIC REAGENT:** Pyrimidine-5-boronic acid | |
| | | **CATALYST:** Pd₂dba₃ | **MS** (ESI): 442.2 ([M+H]⁺). |
| | 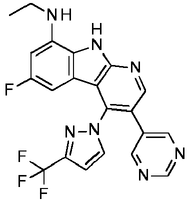 | and XPhos | |
| 1.13 | **N-Ethyl-6-fluoro-3-[2-(2-methoxyethylamino)pyrimi din-5-yl]-4-[3-(trifluoromethyl)pyrazol-1-yl]-9H-pyrido[2,3-b]indol-8-amine** | **CORE:** Intermediate A2 | **¹H NMR** (400 MHz, DMSO-d6) δ ppm: 12.01 (s, 1H), 8.66 (s, 1H), 8.31 (d, 1H), 8.06 (br. s., 1H), 7.33 (br. s., 1H), 7.14 (d, 1H), 6.47 (m, 1H), 5.85 (br. s., 1H), 5.67 (m, 1H), 5.33 (m, 1H), 3.44 (s, 3H), 3.28 (br. s., 2H), 1.32 (m, 3H). |
| | | **NUCLEOPHILE:** 3-(Trifluoromethyl)pyrazol e | |
| | 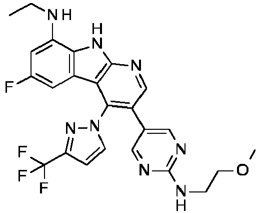 | **BORONIC REAGENT:** N-(2-Methoxyethyl)-5-(4,4,5,5-tetramethyl-1,3,2-dioxaborolan-2-yl)pyrimidin-2-amine (BePharm: Catalog # B239854) | |
| | | | **MS** (ESI): 515.3 ([M+H]⁺). |
| | | **CATALYST:** Pd₂dba₃ and XPhos | |
| 1.14 | **5-[4-[4-(Aminomethyl)-3-(trifluoromethyl)pyrazol-1-yl]-6-fluoro-8-(methylamino)-9H-pyrido[2,3-b]indol-3-yl]pyridine-3-carbonitrile** | **CORE:** Intermediate A1 | **¹H NMR** (400 MHz, DMSO-d6) δ ppm: 12.19 (br. s, 1 H), 9.01 (d, J= 1.6 Hz, 1H), 8.75 (s, 1 H), 8.56 (d, J= 2.0 Hz, 1H), 8.30 (m, 3H), 8.20 (s, 1H), 6.50 (m, 1H), 6.04 (m, 1H), 4.10 (m, 2H), 2.93 (s, 3H). |
| | | **NUCLEOPHILE:** Intermediate C1 | |
| | | **BORONIC REAGENT:** 3-Cyanopyridine-5-boronic acid pinacol ester | |
| | | **CATALYST:** Pd₂dba₃ and Xphos | **MS** (ESI): 481.2 ([M+H]⁺). |
| | | Amino: Intermediate C1 | |
| | 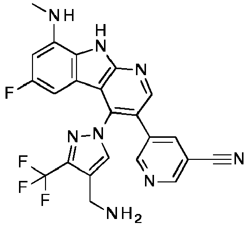 | | |
| 1.15 | **5-[8-(Ethylamino)-6-fluoro-4-[3-(trifluoromethyl)pyrazol-1-yl]-9H-pyrido[2,3-b]indol-3-yl]pyridine-3-carboxamide** | **CORE:** Intermediate A2 | **¹H NMR** (400 MHz, DMSO-d6) δ ppm: 12.13 (s, 1H), 8.95 (d, 1H), 8.70-8.79 (m, 1H), 8.42 (d, 1H), 8.27 (d, 1H), 8.15 (s, 1H), 8.08 (m, 1H), 7.63 (br. s., 1H), 7.08 (d, 1H), 6.50 (m, 1H), 5.89 (br. s., 1H), 5.76 (m, 1H), 3.27 (m, 2H), 1.31-1.36 (m, 3H). |
| | | **NUCLEOPHILE:** 3-(Trifluoromethyl)pyrazol e | |
| | 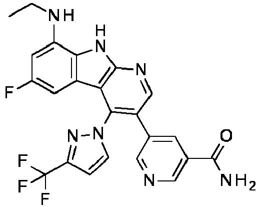 | **BORONIC REAGENT:** 5-Carbamoylpyridine-3-boronic acid pinacol ester | |
| | | **CATALYST:** Pd₂dba₃ and XPhos | |
| | | | **MS** (ESI): 484.2 ([M+H]⁺). |
| 1.16 | **N-Ethyl-6-fluoro-3-(2-morpholinopyrimidin-5-yl)-4-[3-(trifluoromethyl)pyrazol-1-yl]-9H-pyrido[2,3-b]indol-8-amine** | **CORE:** Intermediate A2 | **¹H NMR** (400 MHz, DMSO-d6) δ ppm: 12.06 (br. s., 1H), 8.67 (s, 1H), 8.33 (d, 1H), 8.18 (s, 2H), 7.17 (d, 1H), 6.47 (m, 1H), 5.86 (br. s., 1H), 5.65 (m, 1H), 3.53-3.74 (m, 6H), 3.14-3.30 (m, 2H), 1.96-2.05 (m, 2H), 1.32 (m, 3H). |
| | | **NUCLEOPHILE:** 3-(Trifluoromethyl)pyrazol e | |
| | 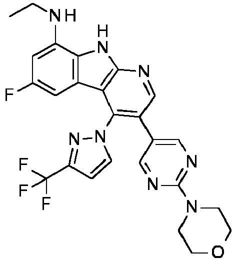 | **BORONIC REAGENT:** 2-(4-Morpholino) pyrimidine-5-boronic acid pinacol ester | |
| | | **CATALYST:** Pd₂dba₃ and XPhos | **MS** (ESI): 527.4 ([M+H]⁺). |
| 1.17 | **N-Ethyl-6-fluoro-3-[2-** | **CORE:** Intermediate A2 | **¹H NMR** (400 MHz, |
| | **(methylamino)pyrimidin-5-yl]-4-[3-(trifluoromethyl)pyrazol-1-yl]-9H-pyrido[2,3-b]indol-8-amine** | **NUCLEOPHILE:** 3-(Trifluoromethyl)pyrazol e | DMSO-*d6*) δ ppm: 12.03 (s, 1H), 8.66 (s, 1H), 8.31 (s, 1H), 7.22 (br. s., 1H), 7.14 (d, 1H), 6.68 (br. s., 1H), 6.47 (m, 1H), 5.86 (br. s., 1H), 5.66 (m, 1H), 5.33 (m, 1H), 3.19-3.29 (m, 2H), 2.74 (d., 3H), 1.29-1.35 (t, 3H). |
| | | **BORONIC REAGENT:** (2-(Methylamino) pyrimidin-5-yl)boronic acid | |
| | 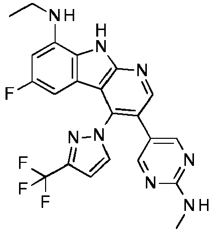 | | |
| | | **CATALYST:** Pd₂dba₃ and XPhos | **MS** (ESI): 471.3 ([M+H]⁺). |
| 1.18 | **N-Ethyl-6-fluoro-3-(5-fluoro-3-pyridyl)-4-[3-(trifluoromethyl)pyrazol-1-yl]-9H-pyrido[2,3-b]indol-8-amine** | **CORE:** Intermediate A2 | **¹H NMR** (400 MHz, DMSO-d6) δ ppm: 12.20 (br. s., 1H), 8.76 (s, 1H), 8.56 (d, 1H), 8.33 (s, 1H), 8.22 (s, 1H), 7.57 (d, 1H), 7.22 (br. s., 1H), 6.66-6.72 (m, 1H), 6.50 (d, 1H), 5.76 (m, 1H), 3.21-3.30 (m, 2H), 1.29-1.35 (m, 3H). |
| | | **NUCLEOPHILE:** 3-(Trifluoromethyl)pyrazol e | |
| | 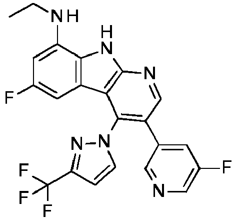 | **BORONIC REAGENT:** 5-Fluoropyridine-3-boronic acid | |
| | | **CATALYST:** Pd₂dba₃ and XPhos | **MS** (ESI): 459.2 ([M+H]⁺) |
| 1.19 | **N-Ethyl-6-fluoro-3-pyrazolo[1,5-a]pyrimidin-6-yl-4-[3-(trifluoromethyl)pyrazol-1-yl]-9H-pyrido[2,3-b]indol-8-amine** | **CORE:** Intermediate A2 | **¹H NMR** (400 MHz, DMSO-d6) δ ppm: 12.16 (s, 1H), 9.15 (d, 1H), 8.84 (s, 1H), 8.40 (d, 1H), 8.28 (d, 1H), 8.10 (d, 1H), 7.13 (d, 1H), 6.76 (d, 1H), 6.51 (m, 1H), 5.90 (br. s., 1H), 5.81 (m, 1H), 3.22-3.31 (m, 2H), |
| | | **NUCLEOPHILE:** 3-(Trifluoromethyl)pyrazol e | |
| | | **BORONIC REAGENT:** 6-(4,4,5,5-Tetramethyl-1,3,2-dioxaborolan-2-yl)pyrazolo[1,5- | |
| | 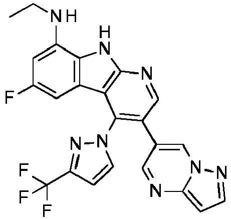 | a]pyrimidine | 1.30-1.37 (t, 3H). |
| | | **CATALYST:** Pd₂dba₃ and XPhos | MS (ESI): 481.3 ([M+H]⁺) |
| 1.20 | **N-Ethyl-6-fluoro-3-(2-pyrrolidin-3-yloxypyrimidin-5-yl)-4-** [3-**(trifluoromethyl)pyrazol-1-yl]-9H-pyrido[2,3-b]indol-8-amine** | **CORE:** Intermediate A2 | **¹H NMR** (400 MHz, MeoH-d4) δ ppm: 8.51 (s, 1H), 8.33 (s, 2H), 7.98 (s, 1H), 6.86 (s, 1H), 6.40 (m, 1H), 5.80 (m, 1H), 5.60 (s, 1H), 3.40 (m, 2H), 3.31 (m, 2H), 3.20 (m, 2H), 2.20 (m, 3H), 1.32 (m, 3H). |
| | | **NUCLEOPHILE:** 3-(Trifluoromethyl)pyrazol e | |
| | 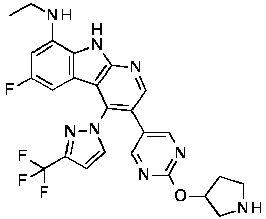 | **BORONIC REAGENT:** Intermediate B5 | |
| | | **CATALYST:** Pd₂dba₃ and XPhos | MS (ESI): 527.3 ([M+H]⁺) |
| 1.21 | **4-(5,6-Dihydro-4H-pyrrolo[3,4-c]pyrazol-1-yl)-N-ethyl-6-fluoro-3-(2-methoxypyrimidin-5-yl)-9H-pyrido[2,3-b]indol-8-amine** | **CORE:** Intermediate A2 | **¹H NMR** (400 MHz, DMSO-d6) δ ppm: 12.13 (s, 1H), 10.00 (br. s., 2H), 8.70 (s, 1H), 8.38 (s, 2H), 7.84 (s, 1H), 6.51 (m, 1H), 6.02 (m, 1H), 4.35-4.42 (m, 2H), 4.10 (s, 1H), 3.95 (s, 3H) 3.80 (m, 1H), 3.26 (m, 2H), 1.32 (t, J=7.2 Hz, 3H). |
| | | **NUCLEOPHILE:** *tert-*Butyl 4,6-dihydropyrrolo[3,4-c] pyrazole-5(1H)-carboxylate | |
| | 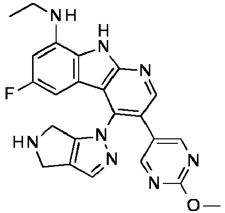 | **BORONIC REAGENT:** 2-Methoxypyrimidine-5-boronic acid | |
| | | **CATALYST:** Pd₂dba₃ and XPhos | MS (ESI): 445.3 ([M+H]⁺) |
| 1.22 | **3-(2-Ethoxypyrimidin-5-yl)-N-ethyl-6-fluoro-4-[3-(trifluoromethyl)pyrazol-1-yl]-9H-pyrido[2,3-b]indol-8-amine** | **CORE:** Intermediate A2 | **¹H NMR** (400 MHz, MeOH-d4) δ ppm: 8.50 (s, 1H), 8.26 (s, 2H), 7.94 (d, 1H), 6.85 (d, 1H), 6.38 (m, 1H), 5.79 (m, 1H), 4.34 (m, 2H), 3.21 (d, 2H), 1.26-1.35 (m, 6H). |
| | | **NUCLEOPHILE:** 3-(Trifluoromethyl)pyrazol e | |
| | 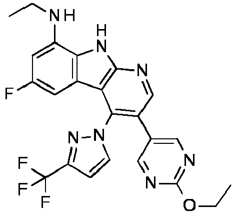 | **BORONIC REAGENT:** 2-Ethoxypyrimidine-5-boronic acid | |
| | | **CATALYST:** Pd₂dba₃ and XPhos | MS (ESI): 486.5 ([M+H]⁺) |
| 1.23 | **3-[2-(Azetidin-3-yloxy)pyrimidin-5-yl]-N-ethyl-6-fluoro-4-[3-(trifluoromethyl)pyrazol-1-yl]-9H-pyrido[2,3-b]indol-8-amine** | **CORE:** Intermediate A2 | **¹H NMR** (400 MHz, MeOH-d4) δ ppm: 8.61 (s, 1H), 8.38 (s, 2H), 8.07 (s, 1H), 6.97 (s, 1H), 6.51 (m, 1H), 5.90 (m, 1H), 5.50 (m, 1H), 4.00 (m, 2H), 3.75 (m, 2H), 3.51 (m, 1H), 3.31 (m, 2H), 1.42 (m, 3H). |
| | | **NUCLEOPHILE:** 3-(Trifluoromethyl)pyrazol e | |
| | 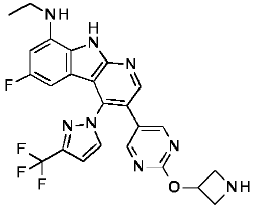 | **BORONIC REAGENT:** Intermediate B6 | |
| | | **CATALYST:** Pd₂dba₃ and XPhos | MS (ESI): 513.2 ([M+H]⁺) |
| 1.24 | **N-Ethyl-6-fluoro-3-[2-(pyrrolidin-3-ylamino)pyrimidin-5-yl]-4-[3-(trifluoromethyl)pyrazol-1-yl]-9H-pyrido[2,3-b]indol-8-amine** | **CORE:** Intermediate A2 | **¹H NMR** (400 MHz, MeOH-d4) δ ppm: 8.54 (s, 1H), 8.17 (s, 2H), 8.02 (s, 1H), 6.96 (s, 1H),6.47 (d, J=11.8Hz, 1H), 5.86 (d, J=9.2 Hz, 1H), 4.59 (m, 1H), 3.57 (m, 2H), 3.01 (m, 1H), 2.88 (m, 1H), 2.41 (m, 1H), 2.25 (m, 1H), 1.40- |
| | | **NUCLEOPHILE:** 3-(Trifluoromethyl)pyrazol e | |
| | | **BORONIC REAGENT:** Intermediate B7 | |
| | | **CATALYST:** Pd₂dba₃ and XPhos | |
| | 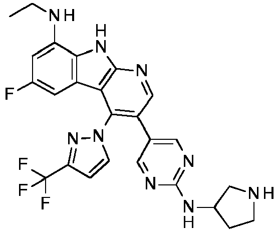 | | 1.43 (m, 5H). |
| | | | MS (ESI): 526.2 ([M+H]⁺) |
| 1.25 | **N-Ethyl-6-fluoro-3-[2-(tetrahydrofuran-3-ylamino)pyrimidin-5-yl]-4-[3-(trifluoromethyl)pyrazol-1-yl]-9H-pyrido[2,3-b]indol-8-amine** | **CORE:** Intermediate A2 | **¹H NMR** (400 MHz, DMSO-*d6*) δ ppm: 12.03 (br. s, 1H), 8.67 (s, 1H), 8.31 (s, 1H), 8.08 (br. s, 2H),7.66 (s,1H), 7.14 (s, 1H), 6.46 (d, J=11.6 Hz, 1H), 5.68 (d, J=8.8Hz, 1H), 3.85 (m, 3H), 3.72 (m, 1H), 3.53 (m, 1H), 3.24 (m, 2H), 2.13 (m, 1H), 1.86 (m, 1H),1.31 (t, J= 6.8Hz, 3H). |
| | | **NUCLEOPHILE:** 3-(Trifluoromethyl)pyrazol e | |
| | 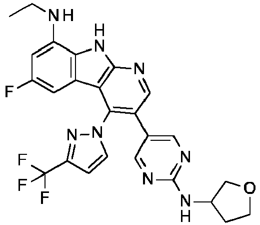 | **BORONIC REAGENT:** Intermediate B8 | |
| | | **CATALYST:** Pd₂dba₃ and XPhos | |
| | | | MS (ESI): 527.3 ([M+H]⁺) |
| 1.26 | **5-[4-[4-(Aminomethyl)-3-(trifluoromethyl)pyrazol-1-yl]-8-(ethylamino)-6-fluoro-9H-pyrido[2,3-b]indol-3-yl]pyridine-3-carbonitrile** | **CORE:** Intermediate A2 | **¹H NMR** (400 MHz, DMSO-*d6*) δ ppm: 12.28 (s, 1H), 9.00 (d, J=1.6Hz, 1H), 8.74 (s, 1H), 8.55 (d, J=2.0Hz, 1H), 8.36 (br. s, 3H), 8.30 (s, 1 H), 8.19 (s, 1H), 6.53 (m, 1H), 6.03 (m, 1H), 4.10 (s, 2H), 3.26 (m, 2H), 1.32 (t, J=6.8 Hz, 3H). |
| | | **NUCLEOPHILE:** Intermediate C1 | |
| | 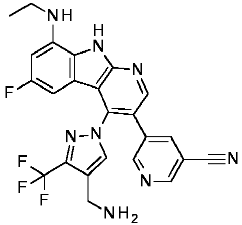 | **BORONIC REAGENT:** 3-Cyanopyridine-5-boronic acid pinacol ester | |
| | | **CATALYST:** Pd₂dba₃ and XPhos | |
| | | | MS (ESI): 495.3 ([M+H]⁺) |
| 1.27 | **4-[4-(Aminomethyl)-3-** | **CORE:** Intermediate A2 | **¹H** NMR (400 MHz, |
| | **(trifluoromethyl)pyrazol-1-yl]-N-ethyl-6-fluoro-3-(2-methoxypyrimidin-5-yl)-9H-pyrido[2,3-b]indol-8-amine** | **NUCLEOPHILE:** Intermediate C1 | DMSO-*d6*) δ ppm: 12.23 (s, 1H), 8.71 (s, 1H), 8.31-8.40 (m, 6H), 6.48 (m, 1H), 5.94 (m, 1H), 4.12 (s, 2H), 3.94 (s, 3H), 3.25 (q, J=7.2Hz, 2H), 1.32 (t, J=7.2Hz, 3H). |
| | | **BORONIC REAGENT:** 2-Methoxypyrimidine-5-boronic Acid | |
| | 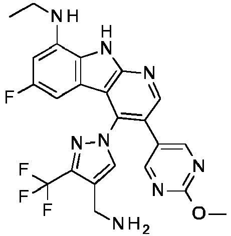 | **CATALYST:** Pd₂dba₃ and XPhos | MS (ESI): 501.1 ([M+H]⁺) |
| 1.28 | **5- [8-(Ethylamino)-5,6-difluoro-4-[3-(trifluoromethyl)pyrazol-1-yl]-9H-pyrido[2,3-b]indol-3-yl]pyridine-3-carbonitrile** | **CORE:** Intermediate A5 | **¹H NMR** (400 MHz, DMSO-*d6*) δ ppm: 12.51 (br. s, 1H), 8.98 (s, 1H) 8.79 (s, 1H), 8.60 (s, 1H), 8.31 (s, 1H), 8.03 (s, 1H), 6.96 (s, 1H),6.67 (m, 1H), 5.69 (s, 1H), 3.23 (m, 2H), 1.31 (t, J=7.8 Hz, 3H). |
| | | **NUCLEOPHILE:** 3-(Trifluoromethyl)pyrazol e | |
| | 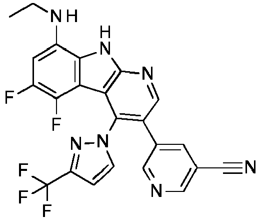 | **BORONIC REAGENT:** 3-cyanopyridine-5-boronic acid pinacol ester | |
| | | **CATALYST:** Pd₂dba₃ and XPhos | MS (ESI): 484.2 ([M+H]⁺) |
| 1.29 | **N-Ethyl-5,6-difluoro-3-(2-methoxypyrimidin-5-yl)-4-[3-(trifluoromethyl)pyrazol-1-yl]-9H-pyrido[2,3-b]indol-8-amine** | **CORE:** Intermediate A5 | **¹H NMR** (400 MHz, DMSO-*d6*) δ ppm: 12.43 (s, 1H), 8.74 (s, 1H), 8.32-8.35 (m, 3H), 6.97 (d, J=2.0Hz, 1H), 6.65 (m, 1H), 5.65 (br. s, 1H), 3.91 (s, 3H), 3.23 (m, 2H), 1.31 (t, J=7.2Hz, 3H). |
| | | **NUCLEOPHILE:** 3-(Trifluoromethyl)pyrazol e | |
| | | **BORONIC REAGENT:** 2-Methoxypyrimidine-5-boronic Acid | |
| | 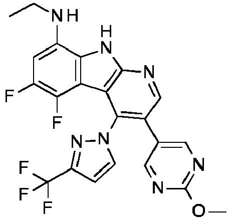 | **CATALYST:** Pd₂dba₃ and XPhos | MS (ESI): 490.2 ([M+H]⁺) |
| 1.30 | **3-[2-(2-Amino-2-methyl-propoxy)pyrimidin-5-yl]-N-ethyl-6-fluoro-4-[3-(trifluoromethyl)pyrazol-1-yl]-9H-pyrido[2,3-b]indol-8-amine** | **CORE:** Intermediate A2 | **¹H NMR** (400 MHz, MeOH-*d4*) δ ppm: 8.51 (s, 1H), 8.42-8.47 (m, 1H), 8.29-8.37 (m, 2H), 7.97 (d, 1H), 6.85 (d, 1H), 6.39 (m, 1H), 5.77 (m, 1H), 4.26 (s, 2H), 1.27-1.35 (m, 9H). |
| | | **NUCLEOPHILE:** 3-(Trifluoromethyl)pyrazol e | |
| | 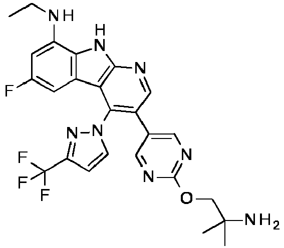 | **BORONIC REAGENT:** Intermediate B9 | |
| | | **CATALYST:** Pd₂dba₃ and XPhos | MS (ESI): 529.3 ([M+H]⁺) |
| 1.31 | **2-[5-[8-(Ethylamino)-6-fluoro-4-[3-(trifluoromethyl)pyrazol-1-yl]-9H-pyrido[2,3-b]indol-3-yl]pyrimidin-2-yl]propan-2-ol** | **CORE:** Intermediate A2 | **¹H NMR** (400 MHz, MeOH-d4) δ ppm: 8.66 (s, 1H), 8.60 (s, 2H), 8.10 (d, 1H), 6.97 (d, 1H), 6.51 (m, 1H), 5.96 (m, 1H), 1.60 (s, 6H), 1.43 (t, 3H). |
| | | **NUCLEOPHILE:** 3-(Trifluoromethyl)pyrazol e | |
| | | **BORONIC REAGENT:** 2-[5-(4,4,5,5-Tetramethyl-1,3,2-dioxaborolan-2-yl)pyrimidin-2-yl]propan-2-ol | |
| | | | MS (ESI): 500.3 ([M+H]⁺) |
| | | **CATALYST:** Pd₂dba₃ | |
| | 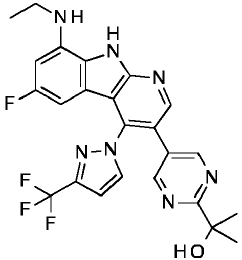 | and XPhos | |
| 1.32 | **5-[8-(Ethylamino)-4-(3-ethylpyrazol-1-yl)-6-fluoro-9H-pyrido[2,3-b]indol-3-yl]pyridine-3-carbonitrile** | **CORE:** Intermediate A2 | **¹H NMR** (400 MHz, DMSO-*d6*) δ ppm: 12.01 (s, 1H), 8.94 (d, J=1.6Hz, 1H), 8.71 (s, 1H), 8.56 (d, J=2.0Hz, 1H), 7.97 (t, J=2.0Hz, 1H), 7.83 (d, J=2.4Hz, 1H), 6.43-6.49 (m, 2H), 6.10 (m, 1H), 5.84 (br. s, 1H), 3.26 (q, J=7.2Hz, 2H), 2.64 (m, 2H), 1.32 (t, J=7.2Hz, 3H), 1.19 (t, J=7.6Hz, 3H). |
| | | **NUCLEOPHILE:** 3-Ethyl-1H-pyrazole | |
| | 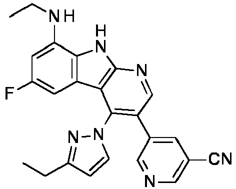 | **BORONIC REAGENT:** 3-Cyanopyridine-5-boronic acid pinacol ester | |
| | | **CATALYST:** Pd₂dba₃ and XPhos | |
| | | | MS (ESI): 426.1 ([M+H]⁺) |
| 1.33 | **6-Chloro-N-ethyl-3-(2-methoxypyrimidin-5-yl)-4-[3-(trifluoromethyl)pyrazol-1-yl]-9H-pyrido[2,3-b]indol-8-amine** | **CORE:** Intermediate A6 | **¹H NMR** (400 MHz, MeOH-*d4*) δ ppm: 8.72 (s, 1H), 8.38 (s, 2H), 8.32 (s, 1H), 7.14 (d, J=2.0 Hz, 1H), 6.58 (s, 1H), 6.05 (s, 1H), 5.87 (d, J=4.4Hz, 1H), 3.92 (s, 3H), 3.24 (m, 2H), 1.31 (t, J=7.2Hz, 3H). |
| | | **NUCLEOPHILE:** 3-(Trifluoromethyl)pyrazol e | |
| | 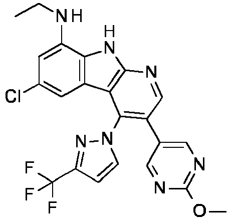 | **BORONIC REAGENT:** 2-Methoxypyrimidine-5-boronic acid | |
| | | **CATALYST:** Pd₂dba₃ and XPhos | MS (ESI): 488.0 ([{³⁵Cl}M+H]⁺), 490.0 ([{³⁷Cl}M+H]⁺) |
| 1.34 | **5-[8-(Ethylamino)-6-fluoro-4-[3-(trifluoromethyl)-5,6-dihydro-4H-pyrrolo[3,4-c]pyrazol-1-yl]-9H-pyrido[2,3-b]indol-3-yl]pyridine-3-carbonitrile** | **CORE:** Intermediate A2 | **¹H NMR** (400 MHz, DMSO-*d6*) δ ppm: 12.30 (s, 1H), 10.22 (br. s, 2H), 9.05 (d, J=1.6Hz, 1H), 8.78 (s, 1H), 8.56 (d, J=2.0Hz, 1H), 8.19 (s, 1H), 6.56 (m, 1H), 6.10 (m, 1H), 4.45-4.61 (m, 2H), 4.22 (d, J=14.8Hz, 1H), 3.84 (d, J=14.8Hz, 1H), 3.28 (q, J=7.2Hz, 2H), 1.33 (t, J=7.2Hz, 3H). |
| | | **NUCLEOPHILE:** Intermediate C2 | |
| | | **BORONIC REAGENT:** 3-Cyanopyridine-5-boronic acid pinacol ester | |
| | 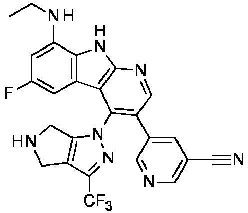 | **CATALYST:** Pd₂dba₃ and Xphos | |
| | | | MS (ESI): 507.1 ([M+H]⁺) |
| 1.35 | **N-Ethyl-6-fluoro-3-(2-methoxypyrimidin-5-yl)-4-[3-(trifluoromethyl)-5,6-dihydro-4H-pyrrolo[3,4-c]pyrazol-1-yl]-9H-pyrido[2,3-b]indol-8-amine** | **CORE:** Intermediate A2 | **¹H NMR** (400 MHz, DMSO-*d6*) δ ppm: 12.25 (s, 1H), 10.26 (br. s, 2H), 8.73 (s, 1H), 8.43 (s, 2H), 6.53 (m, 1H), 6.04 (m, 1H), 4.62 (d, J=13.2Hz, 1H), 4.52 (d, J=13.2Hz, 1H), 4.24 (d, J=14.8Hz, 1H), 3.94 (d, J= 14.8Hz, 1H), 3.27 (q, J=7.2Hz, 2H), 1.32 (t, J=7.2Hz, 3H). |
| | | **NUCLEOPHILE:** Intermediate C2 | |
| | | **BORONIC REAGENT:** 2-Methoxypyrimidine-5-boronic acid | |
| | 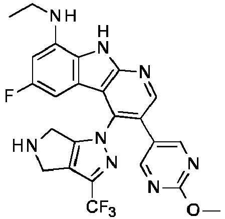 | **CATALYST:** Pd₂dba₃ and Xphos | |
| | | | MS (ESI): 513.1 ([M+H]⁺) |
| 1.36 | **[5-[8-(Ethylamino)-6-fluoro-4-[3-(trifluoromethyl)pyrazol-1-yl]-9H-pyrido[2,3-b]indol-3-yl]-3-pyridyl]methanol** | **CORE:** Intermediate A2 | **¹H NMR** (400 MHz, MeOH-*d4*) δ ppm: 8.64 (s, 1H), 8.55 (s, 1H), 8.31 (s, 1H), 7.98 (d, J=1.5 Hz, 1H), 7.82 (s, 1H), 6.92 (d, J=2.4 Hz, 1H), 6.49 (dd, J=12.0, 2.2 Hz, 1H), 5.93 (dd, |
| | | **NUCLEOPHILE:** 3-(Trifluoromethyl)pyrazol e | |
| | | **BORONIC REAGENT:** | |
| | 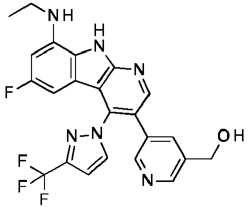 | B11 | J=9.0, 2.2 Hz, 1H), 4.68 (s, 2H), 3.34-3.45 (m, 2H), 1.42 ppm (t, J=7.2 Hz, 3H). |
| | | CATALYST: Pd₂dba₃ and XPhos | |
| | | | MS (ESI): 471.1 ([M+H]⁺) |
| 1.37 | **3-(5-Amino-3-pyridyl)-N-ethyl-6-fluoro-4-[3-(trifluoromethyl)pyrazol-1-yl]-9H-pyrido[2,3-b]indol-8-amine** | **CORE:** Intermediate A2 | **¹H NMR** (400 MHz, MeOH-*d4*) δ ppm: 8.62 (s, 1H), 8.08 (d, J=1.6 Hz, 1H), 7.95 (br s, 1H), 7.63 (s, 1H), 7.32-7.45 (m, 1H), 6.99 (d, J=2.4 Hz, 1H), 6.50 (dd, J=11.9, 2.2 Hz, 1H), 5.98 (dd, J=9.1, 2.3 Hz, 1H), 3.34-3.47 (m, 2H), 1.42 ppm (t, J=7.2 Hz, 3H). |
| | | **NUCLEOPHILE:** 3-(Trifluoromethyl)pyrazol e | |
| | 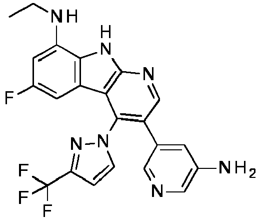 | **BORONIC REAGENT:** 3-Aminopyridine-5-boronic acid pinacol ester | |
| | | **CATALYST:** Pd₂dba₃ and XPhos | |
| | | | MS (ESI): 456.1 ([M+H]⁺) |
| 1.38 | **1-[6-Fluoro-3-(2-methoxypyrimidin-5-yl)-8-(methylamino)-9H-pyrido[2,3-b]indol-4-yl]-5,6-dihydro-4H-pyrrolo[3,4-c]pyrazole-3-carbonitrile** | **CORE:** Intermediate A1 | **¹H NMR** (400 MHz, MeOH-*d4*) δ ppm: 8.67 (s, 1H), 8.49-8.52 (m, 2H), 6.52-6.59 (m, 1H), 6.02 (dd, J=2.20, 8.60 Hz, 1H), 4.71 (d, J=16.44 Hz, 1H), 4.56-4.63 (m, 1H), 4.057-4.079 (m, 1H), 4.04-4.11 (m, 4H), 3.03 (s, 3H). |
| | | **NUCLEOPHILE:** Intermediate C3 | |
| | | **BORONIC REAGENT:** 2-Methoxypyrimidine-5-boronic acid | |
| | 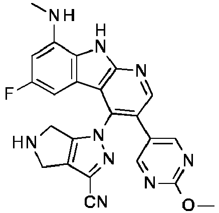 | **CATALYST:** Pd₂dba₃ and XPhos | |
| | | | MS (ESI): 456.2 ([M+H]⁺) |
| 1.39 | **1-[8-(Ethylamino)-6-fluoro-3-(2-methoxypyrimidin-5-yl)-9H-pyrido[2,3-b]indol-4-** | **CORE:** Intermediate A2 | **¹H NMR** (400 MHz, MeOH-*d4*) δ ppm: 8.68 (s, 1H), 8.49 (s, 2H), 6.55-6.51 |
| | | **NUCLEOPHILE:** | |
| | **yl]-4,5,6,7-tetrahydropyrazolo[3,4-c]pyridine-3-carbonitrile** | Intermediate C4 | (d, J=14Hz, 1H), 5.75-5.72 (d, J=10.8Hz, 1H), 4.04 (s, 3H), 3.97-3.89 (m, 2H), 3.47-3.46 (m, 2H), 3.31-3.02 (m, 4H), 1.42-1.38 (m, 3H). |
| | | **BORONIC REAGENT:** 2-Methoxypyrimidine-5-boronic acid | |
| | 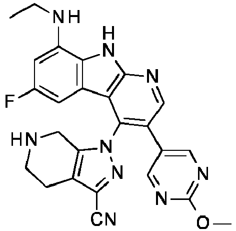 | | |
| | | **CATALYST:** Pd₂dba₃ and XPhos | MS (ESI): 484.2 ([M+H]⁺) |
| 1.40 | **3-(6-Amino-3-pyridyl)-N-ethyl-6-fluoro-4-[3-(trifluoromethyl)pyrazol-1-yl]-9H-pyrido[2,3-b]indol-8-amine** | **CORE:** Intermediate A2 | **¹H NMR** (400 MHz, MeOH-*d4*) δ ppm: 8.53 (s, 1H), 8.24 (d, 1H), 7.89 (d, 1H), 7.74 (s, 1H), 7.31 (m, 1H), 6.91 (d, 1H), 6.58 (m, 1H), 6.46 (m, 1H), 5.86 (m, 1H), 3.30 (m, 2H), 1.40 (t, 3H). |
| | | **NUCLEOPHILE:** 3-(Trifluoromethyl)pyrazol e | |
| | 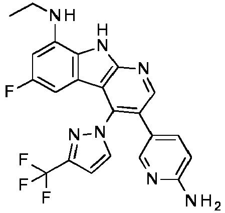 | **BORONIC REAGENT:** 2-Aminopyridine-5-boronic acid pinacol ester | |
| | | **CATALYST:** Pd₂dba₃ and XPhos | MS (ESI): 456.2 ([M+H]⁺) |
| 1.41 | **5-[4-[3-(Difluoromethyl)pyrazol-1-yl]-8-(ethylamino)-6-fluoro-9H-pyrido[2,3-b]indol-3-yl]pyridine-3-carbonitrile** | **CORE:** Intermediate A2 | **¹H NMR** (400 MHz, MeOH-*d4*) δ ppm: 8.84 (d, 1H), 8.64 (s, 1H), 8.58 (d, 1H), 8.02 (m, 1H), 7.96 (d, 1H), 6.83 (d, 1H), 6.49 (m, 1H), 5.99 (m, 1H), 5.17 (s, 1H), 4.58 (m, 2H), 1.41 (t, 3H). |
| | | **NUCLEOPHILE:** 3-(Difluoromethyl)-1H-pyrazole | |
| | | **BORONIC REAGENT:** 3-Cyanopyridine-5-boronic acid pinacol ester | |
| | | **CATALYST:** Pd₂dba₃ and XPhos | MS (ESI): 448.3 ([M+H]⁺) |
| | 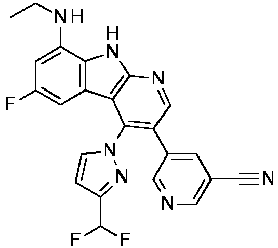 | | |
| 1.42 | **N-Ethyl-6-fluoro-3-[6-(morpholinomethyl)-3-pyridyl]-4-[3-(trifluoromethyl)pyrazol-1-yl]-9H-pyrido[2,3-b]indol-8-amine** | **CORE:** Intermediate A2 | **¹H NMR** (400 MHz, MeOH-*d4*) δ ppm: 8.62 (s, 1H), 8.46 (d, J=1.8 Hz, 1H), 8.02 (d, J=1.5 Hz, 1H), 7.77 (dd, J=8.0, 2.3 Hz, 1H), 7.51 (d, J=7.9 Hz, 1H), 6.92 (d, J=2.3 Hz, 1H), 6.50 (dd, J=12.0, 2.2 Hz, 1H), 5.92 (dd, J=9.0, 2.2 Hz, 1H), 4.39 (s, 2H), 3.83-3.99 (m, 4H), 3.12-3.29 (m, 6H), 1.42 (t, J=7.2 Hz, 3H). |
| | | **NUCLEOPHILE:** 3-(Trifluoromethyl)pyrazol e | |
| | 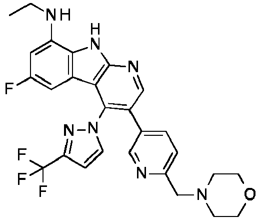 | **BORONIC REAGENT:** (6-(Morpholinomethyl)pyri din-3-yl)boronic acid (Bepharm, catalog number: B232176) | |
| | | **CATALYST:** Pd₂dba₃ and XPhos | MS (ESI): 540.2 ([M+H]⁺) |
| 1.43 | **5-[8-(Ethylamino)-6-fluoro-4-[3-(trifluoromethyl)pyrazol-1-yl]-9H-pyrido[2,3-b]indol-3-yl]-N-methyl-pyridine-3-carboxamide** | **CORE:** Intermediate A2 | **¹H NMR** (400 MHz, MeOH-*d4*) δ ppm: 8.78 (d, 1H), 8.56 (s, 1H), 8.31 (d, 1H), 8.04 (m, 1H), 7.87 (d, 1H), 6.79 (d, 1H), 6.39 (m, 1H), 5.79-5.86 (m, 1H), 3.15-3.20 (m, 2H), 2.84 (s, 3H), 1.26-1.35 (t, 3H). |
| | | **NUCLEOPHILE:** 3-(Trifluoromethyl)pyrazol e | |
| | | **BORONIC REAGENT:** N-Methyl-5-(4,4,5,5-tetramethyl-1,3,2-dioxaborolan-2-yl)nicotinamide (Bepharm, catalog | |
| | | | MS (ESI): 498.1 ([M+H]⁺) |
| | 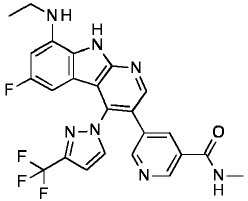 | number: B250344) | |
| | | **CATALYST:** Pd₂dba₃ and XPhos | |
| 1.44 | **N-Ethyl-6-fluoro-3-(5-morpholino-3-pyridyl)-4-[3-(trifluoromethyl)pyrazol-1-yl]-9H-pyrido[2,3-b]indol-8-amine** | **CORE:** Intermediate A2 | **¹H NMR** (400 MHz, MeOH-*d4*) δ ppm: 8.59-8.70 (m, 1H), 8.21-8.36 (m, 1H), 7.89-8.06 (m, 2H), 7.20-7.37 (m, 1H), 6.96 (d, J=2.4 Hz, 1H), 6.50 (dd, J=11.9, 2.1 Hz, 1H), 5.90 (dd, J=9.0, 2.1 Hz, 1H), 3.75-3.87 (m, 4H), 3.03-3.24 (m, 4H), 2.84-3.02 (m, 2H), 1.27-1.49 (m, 3H). |
| | | **NUCLEOPHILE:** 3-(Trifluoromethyl)pyrazol e | |
| | 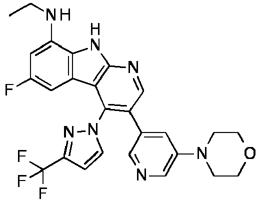 | **BORONIC REAGENT:** (5-Morpholinopyridin-3-yl)boronic acid (Bepharm, catalog number: B165830) | |
| | | **CATALYST:** Pd₂dba₃ and XPhos | MS (ESI): 526.3 ([M+H]⁺) |
| 1.45 | **3-[5-(Aminomethyl)-3-pyridyl]-N-ethyl-6-fluoro-4-[3-(trifluoromethyl)pyrazol-1-yl]-9H-pyrido[2,3-b]indol-8-amine** | **CORE:** Intermediate A2 | **¹H NMR** (400 MHz, MeOH-*d4*) δ ppm: 8.57-8.68 (m, 2H), 8.34 (d, J=1.8 Hz, 1H), 7.93-8.09 (m, 2H), 6.95 (d, J=2.4 Hz, 1H), 6.43-6.57 (m, 1H), 5.93 (dd, J=9.0, 2.2 Hz, 1H), 4.23 (s, 2H), 3.21-3.24 (m, 2H),1.33-1.50 ppm (m, 3H). |
| | | **NUCLEOPHILE:** 3-(Trifluoromethyl)pyrazol e | |
| | 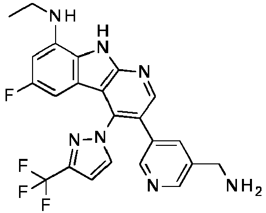 | **BORONIC REAGENT:** (5-(((*tert-*Butoxycarbonyl)amino) methyl)-pyridin-3-yl)boronic acid (Matrix Scientific), catalog number: 097316) | |
| | | | MS (ESI): 470.4 ([M+H]⁺) |
| | | **CATALYST:** Pd₂dba₃ and XPhos | |
| 1.46 | **3-[2-(Aminomethyl)pyrimidin-5-yl]-N-ethyl-6-fluoro-4-[3-(trifluoromethyl)pyrazol-1-yl]-9H-pyrido[2,3-b]indol-8-amine** | **CORE:** Intermediate A2 | **¹H NMR** (400 MHz, MeOH-*d4*) δ ppm: 8.55-8.64 (m, 3H), 7.99 (d, J=1.6 Hz, 1H), 6.85 (d, J=2.4 Hz, 1H), 6.41 (dd, J=11.9, 2.2 Hz, 1H), 5.77 (dd, J=9.0, 2.3 Hz, 1H), 4.32 (s, 2H), 3.22 (m, 2H), 1.32 ppm (t, J=7.2 Hz, 3H). |
| | | **NUCLEOPHILE:** 3-(Trifluoromethyl)pyrazol e | |
| | 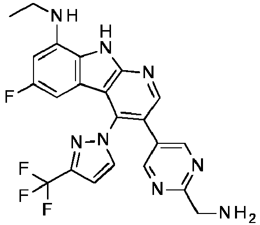 | **BORONIC REAGENT:** Intermediate B10 | |
| | | **CATALYST:** Pd₂dba₃ and XPhos | |
| | | | MS (ESI): 471.3 ([M+H]⁺) |
| 1.47 | **3-(2-Ethylpyrimidin-5-yl)-6-fluoro-N-methyl-4-[3-(trifluoromethyl)pyrazol-1-yl]-9H-pyrido[2,3-b]indol-8-amine** | **CORE:** Intermediate A1 | **¹H NMR** (400 MHz, MeOH-*d4*) δ ppm: 8.66 (s, 1H), 8.54 (s, 2H), 8.09 (s, 1H), 6.98 (s, 1H), 6.51 (m, 1H), 5.96 (m, 1H), 3.01 (s, 3H), 2.97 (m, 2H), 1.34 (m, 3H). |
| | | **NUCLEOPHILE:** 3-(Trifluoromethyl)pyrazol e | |
| | 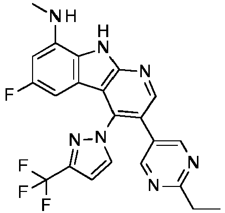 | **BORONIC REAGENT:** 2-Ethyl-5-(4,4,5,5-tetramethyl-1,3,2-dioxaborolan-2-yl)pyrimidine (Bepharm, Catalog number: B249228) | |
| | | | MS (ESI): 456.3 ([M+H]⁺) |
| | | **CATALYST:** Pd₂dba₃ and XPhos | |
| 1.48 | **[5-[8-(Ethylamino)-6-fluoro-4-[3-(trifluoromethyl)pyrazol-1-yl]-9H-pyrido[2,3-b]indol-3-** | **CORE:** Intermediate A2 | **¹H NMR** (400 MHz, MeOH-*d4*) δ ppm: 8.55-8.64 (m, 3H), 7.99 (d, J=1.6 Hz, 1H), 6.85 (d, J=2.4 Hz, |
| | | **NUCLEOPHILE:** 3-(Trifluoromethyl)pyrazole | |
| | **yl]pyrimidin-2-yl]methanol** | | 1H), 6.41 (dd, J=11.9, 2.2 Hz, 1H), 5.77 (dd, J=9.0, 2.3 Hz, 1H), 4.32 (s, 2H), 3.22 (m, 2H), 1.32 ppm (t, J=7.2 Hz, 3H). |
| | 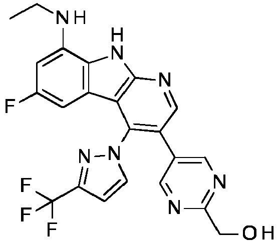 | **BORONIC REAGENT:** Intermediate B11 | |
| | | **CATALYST:** Pd₂dba₃ and XPhos | MS (ESI): 471.3 ([M+H]⁺) |
| 1.49 | **5-[4-[4-(Aminomethyl)-3-(trifluoromethyl)pyrazol-1-yl]-6,7-difluoro-8-(methylamino)-9H-pyrido[2,3-b]indol-3-yl]pyridine-3-carbonitrile** | **CORE:** Intermediate A4 | **¹H NMR** (400 MHz, DMSO-*d6*) δ ppm: 12.40 (s, 1H), 9.01 (s, 1H), 8.75 (s, 1H), 8.54 (d, 1H), 8.33 (m, 4H), 8.20 (s, 1H), 6.28 (m, 1H), 4.09 (m, 2H), 3.14 (d, 3H). |
| | | **NUCLEOPHILE:** Intermediate C1 | |
| | | **BORONIC REAGENT:** 3-Cyanopyridine-5-boronic acid pinacol ester | |
| | 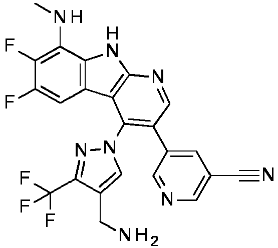 | **CATALYST:** Pd₂dba₃ and XPhos | MS (ESI): 499.1 ([M+H]⁺) |
| 1.50 | **N-ethyl-6-fluoro-3-(2-methylpyrimidin-5-yl)-4-[3-trifluoromethyl)pyrazol-1-yl]-9H-pyrido[2,3-b]indol-8-amine** | **CORE:** Intermediate A2 | **¹H NMR** (400 MHz, DMSO-*d6*): δ = 12.15 (s, 1H), 8.75 (s, 1H), 8.48 (s, 2H), 8.33 (s, 1H), 7.14 (d, *J*=2.3 Hz, 1H), 6.44-6.54 (m, 1H), 5.88 (br s, 1H), 5.73 (dd, *J*=9.0, 2.0 Hz, 1H), 3.22-3.30 (m, 2H), 2.62 (s, 3H), 1.32 ppm (t, *J*=7.2 Hz, 3H). |
| | | **NUCLEOPHILE:** 3-(Trifluoromethyl)pyrazol e | |
| | | **BORONIC REAGENT:** 2-Methyl-5-(4,4,5,5-tetramethyl-1,3,2-dioxaborolan-2- | |
| | 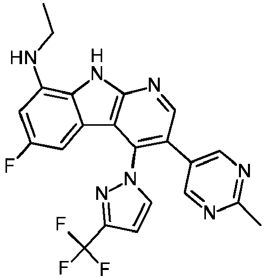 | yl)pyrimidine | |
| | | **CATALYST:** ) Pd₂(dba)₃ | **MS** (ESI): 456.2 ([M+H]⁺). |
| | | **SOLVENT:** 1,4-dioxane/H₂O | |
| 1.51 | **5- [8-(ethylamino) -6-fluoro-4-thiazol-5-yl-9H-pyrido[2,3-b]indol-3-yl]pyridine-3-carbonitrile** | **CORE:** Intermediate A2 | **¹H NMR** (400 MHz, DMSO-*d6*) δ ppm: 9.31 (s, 1H), 8.95 (d, *J*=2.0 Hz, 1H), 8.76 (d, J=2.0 Hz, 1H), 8.57 (s, 1H), 8.32 (t, *J*=2.0 Hz, 1H), 8.10 (s, 1H), 6.45 (dd, *J*=2.3, 12.3 Hz, 1H), 5.8-5.9 (m, 2H), 3.2-3.3 (m, 2H), 1.32 (t, *J*=7.2 Hz, 3H). |
| | | **NUCLEOPHILE:** Thiazol-5-ylboronic acid | |
| | 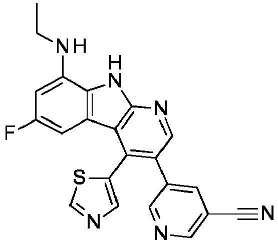 | **BORONIC REAGENT:** 3-Cyanopyridine-5-boronic acid pinacol ester | |
| | | **CATALYST:** Pd₂dba₃ and XPhos | |
| | | | MS (ESI): 415.1 ([M+H]⁺). |
| 1.52 | **N-ethyl-6-fluoro-3-(2-piperazin-1-ylpyrimidin-5-yl)-4-[3-(trifluoromethyl)pyrazol-1-yl]-9H-pyrido[2,3-b]indol-8-amine** | **CORE:** Intermediate A2 | **¹H NMR** (400 MHz, DMSO-*d6*) δ 8.6-8.7 (m, 1H), 8.3-8.4 (m, 1H), 8.13 (s, 1H), 7.6-7.7 (m, 1H), 7.16 (d, 1H, *J*=2.3 Hz), 6.4-6.5 (m, 1H), 5.8-5.9 (m, 1H), 5.6-5.7 (m, 1H), 5.3-5.4 (m, 1H), 3.6-3.7 (m, 2H), 3.2-3.3 (m, 2H), 2.7-2.8 (m, 2H), 1.9-2.1 (m, 2H), 1.6-1.7 (m, 1H), 1.4-1.5 (m, 1H), 1.3-1.3 (m, 3H). |
| | | **NUCLEOPHILE:** 3-(Trifluoromethyl)pyrazol e | |
| | 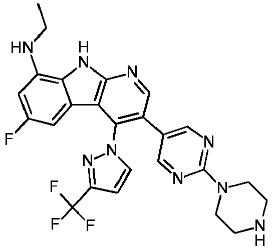 | **BORONIC REAGENT:** *tert*-Butyl 4-[5-(4,4,5,5-tetramethyl-1,3,2-dioxaborolan-2-yl)pyrimidin-2-yl]piperazine-1-carboxylate | |
| | | **CATALYST:** Pd₂(dba)₃ | |
| | | | **MS** (ESI): 526.4 ([M+H]⁺). |
| | | **SOLVENT:** 1,4-dioxane/H₂O | |
| 1.53 | **N-ethyl-6-fluoro-3-[2-(4-piperidyloxy)pyrimidin-5-yl]-4-[3-(trifluoromethyl)pyrazol-1-yl]-9H-pyrido[2,3-b]indol-8-amine** | **CORE:** Intermediate A2 | **¹H NMR** (400 MHz, MeOH-*d4*) δ 8.5-8.5 (m, 1H), 8.3-8.3 (m, 2H), 8.0-8.0 (m, 1H), 7.2-7.3 (m, 1H), 6.8-6.9 (m, 1H), 6.4-6.4 (m, 1H), 5.8-5.8 (m, 1H), 5.2-5.3 (m, 1H), 3.3-3.3 (m, 1H), 3.1-3.1 (m, 1H), 3.0-3.0 (m, 1H), 2.1-2.2 (m, 2H), 1.9-2.0 (m, 3H), 1.7-1.9 (m, 1H), 1.3-1.3 (m, 3H). |
| | | **NUCLEOPHILE:** 3-(Trifluoromethyl)pyrazol e | |
| | 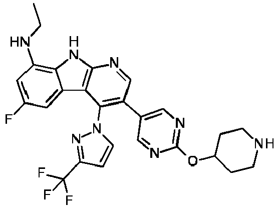 | **BORONIC REAGENT:** *tert*-Butyl 4-[5-(4,4,5,5-tetramethyl-1,3,2-dioxaborolan-2-yl)pyrimidin-2-yl]oxypiperidine-1-carboxylate | |
| | | **CATALYST:** Pd₂(dba)₃ | **MS** (ESI): 541.3 ([M+H]⁺). |
| | | **SOLVENT:** 1,4-dioxane/H₂O | |
| 1.54 | **1-[5-[8-(ethylamino)-6-fluoro-4-[3-(trifluoromethyl)pyrazol-1-yl]-9H-pyrido[2,3-b]indol-3-yl]pyrimidin-2-yl]ethanol** | **CORE:** Intermediate A2 | **¹H NMR** (400 MHz, MeOH-*d4*) δ 8.5-8.5 (m, 1H), 8.5-8.5 (m, 2H), 7.9-8.0 (m, 1H), 6.8-6.9 (m, 1H), 6.3-6.4 (m, 1H), 5.8-5.9 (m, 1H), 4.8-4.9 (m, 1H), 3.18 (m, 2H), 1.41 (d, 3H, *J*=6.6 Hz), 1.3-1.4 (m, 3H). |
| | | **NUCLEOPHILE:** 3-(Trifluoromethyl)pyrazol e | |
| | | **BORONIC REAGENT:** [2-[1-[*tert-*Butyl(dimethyl)silyl]oxy ethyl]pyrimidin-5-yl]boronic acid | |
| | | | **MS** (ESI): 486.3 ([M+H]⁺). |
| | 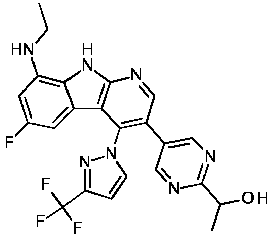 | **CATALYST:** Pd₂(dba)₃ | |
| | | **SOLVENT:** 1,4-dioxane/H₂O | |
| 1.55 | **N-ethyl-6-fluoro-3-[2-[(3*R*)-pyrrolidin-3-yl]oxypyrimidin-5-yl]-4-[3-(trifluoromethyl)pyrazol-1-yl]-9H-pyrido[2,3-b]indol-8-amine** | **CORE:** Intermediate A2 | **¹H NMR** (400 MHz, MeOH-d4) δ 8.63 (s, 1H), 8.41 (s, 2H), 8.08 (d, 1H, *J*=1.6 Hz), 6.98 (d, 1H, *J*=2.3 Hz), 6.51 (dd, 1H, *J*=2.2, 11.9 Hz), 5.92 (dd, 1H, *J*=2.3, 9.0 Hz), 5.6-5.6 (m, 1H), 3.3-3.4 (m, 3H), 3.2-3.3 (m, 3H), 3.08 (br d, 1H, J=8.2 Hz), 2.2-2.3 (m, 1H), 2.1-2.2 (m, 1H) 1.43 (t, 3H, *J*=7.2 Hz). |
| | | **NUCLEOPHILE:** 3-(Trifluoromethyl)pyrazol e | |
| | 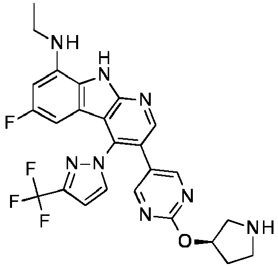 | **BORONIC REAGENT:** Intermediate B5 | |
| | | **CATALYST:** Pd₂(dba)₃ | |
| | | **SOLVENT:** 1,4-dioxane/H₂O | |
| | | | MS (ESI): 527.1 ([M+H]⁺). |
| 1.56 | **N-ethyl-6-fluoro-3-[2-[(3*S*)-pyrrolidin-3-yl]oxypyrimidin-5-yl]-4-[3-(trifluoromethyl)pyrazol-1-yl]-9H-pyrido[2,3-b]indol-8-amine** | **CORE:** Intermediate A2 | **¹H** NMR (400 MHz, MeOH-d4) δ 8.62 (s, 1H), 8.43 (s, 1H), 8.09 (d, 1H, 7=1.6 Hz), 6.98 (d, 1H, 7=2.4 Hz), 6.51 (dd, 1H, 7=2.3, 11.9 Hz), 5.91 (dd, 1H, 7=2.3, 9.0 Hz), 5.65 (br t, 1H, J=4.8 Hz), 3.3-3.6 (m, 3H), 3.2-3.3 (m, 4H), 2.2-2.4 (m, 2H), 1.43 (t, 3H, 7=7.2 Hz). |
| | | **NUCLEOPHILE**: 3-(Trifluoromethyl)pyrazol e | |
| | 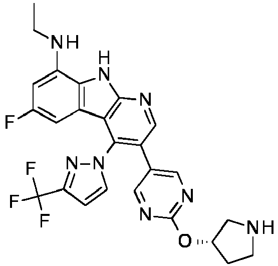 | **BORONIC REAGENT**: Intermediate B5 | |
| | | **CATALYST**: Pd₂(dba)₃ | |
| | | **SOLVENT**: 1,4-dioxane/H₂O | |
| | | | MS (ESI): 527.1 ([M+H]⁺). |
| | | | |
| 1.57 | **(1*S*)-1-[5-[8-(ethylamino)-6-fluoro-4-[3-(trifluoromethyl)pyrazol-1-yl]-9H-pyrido[2,3-b]indol-3-yl]pyrimidin-2-yl]ethanol** | **CORE:** Intermediate A2 | **¹H NMR** (400 MHz, MeOH-*d4*) δ 8.6-8.7 (m, 1H), 8.5-8.6 (m, 2H), 8.0-8.1 (m, 1H), 6.9-7.0 (m, 1H), 6.4-6.5 (m, 1H), 5.9-6.0 (m, 1H), 4.9-5.0 (m, 1H), 3.3-3.3 (m, 2H), 1.5-1.5 (m, 3H), 1.4-1.4 (m, 3H). |
| | | **NUCLEOPHILE:** 3-(Trifluoromethyl)pyrazol e | |
| | 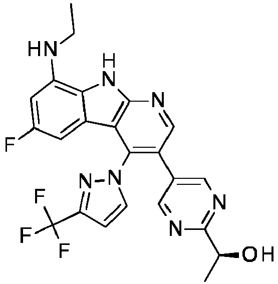 | **BORONIC REAGENT:** [2-[1-[t*ert-*Butyl(dimethyl)silyl]oxy ethyl]pyrimidin-5-yl]boronic acid | |
| | | **CATALYST:** Pd₂(dba)₃ | **MS** (ESI): 486.2 ([M+H]⁺). |
| | | **SOLVENT:** 1,4-dioxane/H₂O | |
| 1.58 | **N-ethyl-6-fluoro-3-[5-[(5-methyl-1,3,4-oxadiazol-2-yl)methyl]-3-pyridyl] -4- [3-(trifluoromethyl)pyrazol-1-yl]-9H-pyrido[2,3-b]indol-8-amine** | **CORE:** Intermediate A2 | **¹H NMR** (400 MHz, MeOH-*d4*) δ ppm: 8.51 (s, 1H), 8.40 (s, 1H), 8.21 (s, 1H), 7.85 (s, 1H), 7.55 (s, 1H), 6.77 (d, 1H), 6.35-6.39 (d, 1H), 5.79-5.82 (d, 1H), 4.15(s, 1H), 3.21 (m, 2H),2.41 (s, 6H), 1.28-1.32 (m, 3H). |
| | | **NUCLEOPHILE:** 3-(Trifluoromethyl)pyrazol e | |
| | 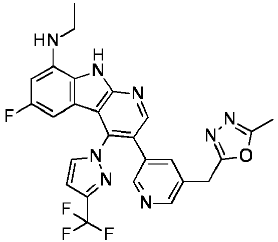 | **BORONIC REAGENT:** Intermediate B12 | |
| | | **CATALYST:** Pd₂(dba)₃ | |
| | | **SOLVENT:** 1,4-dioxane/H₂O | MS (ESI): 537.3 ([M+H]⁺) |
| 1.59 | **6-[8-(ethylamino)-6-fluoro-4-[3-(trifluoromethyl)pyrazol-1-yl]-9H-pyrido[2,3-b]indol-3-yl]pyrazolo[1,5-** | **CORE:** Intermediate A2 | **¹H NMR** (400 MHz, MeOH-*d4*) δ 9.0-9.0 (m, 1H), 8.6-8.7 (m, 1H), 8.43 (s, 1H), 8.36 (d, 1H, *J*=2.1 Hz), 8.0-8.1 (m, 1H), 6.7- |
| | | **NUCLEOPHILE:** 3-(Trifluoromethyl)pyrazol e | |
| | **a]pyrimidine-3-carbonitrile** | **BORONIC REAGENT:** 6-(4,4,5,5-Tetramethyl-1,3,2-dioxaborolan-2-yl)pyrazolo[1,5-a]pyrimidine-3-carbonitrile | 6.8 (m, 1H), 6.4-6.5 (m, 1H), 5.9-5.9 (m, 1H), 2.9-3.0 (m, 2H), 1.3-1.4 (m, 3H). |
| | 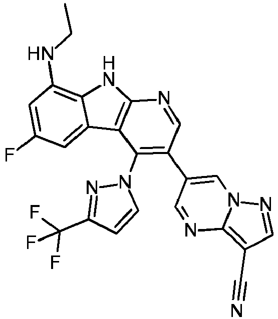 | | |
| | | | **MS** (ESI): 505.2 ([M+H]⁺). |
| | | **CATALYST:** Pd₂(dba)₃ | |
| | | **SOLVENT:** 1,4-dioxane/H₂O | |
| 1.60 | **4-[4-(Aminomethyl)-3-(trifluoromethyl)pyrazol-1-yl]-N-ethyl-6-fluoro-3-pyrazolo[1,5-a]pyrimidin-6-yl-9H-pyrido[2,3-b]indol-8-amine** | **CORE:** Intermediate A2 | **¹H NMR** (400 MHz, MeOH-*d4*) δ 8.9-9.0 (m, 1H), 8.7-8.8 (m, 1H), 8.2-8.3 (m, 2H), 8.15 (s, 1H), 6.74 (dd, 1H, J=0.8, 2.4 Hz), 6.53 (dd, 1H, J=2.3, 12.1 Hz), 6.03 (dd, 1H, J=2.3, 8.9 Hz), 4.04 (s, 2H), 3.3-3.4 (m, 2H), 1.43 (t, 3H, J=7.2 Hz). |
| | | **NUCLEOPHILE:** Intermediate C1 | |
| | | **BORONIC REAGENT:** 6-(4,4,5,5-Tetramethyl-1,3,2-dioxaborolan-2-yl)pyrazolo[1,5-a]pyrimidine | |
| | 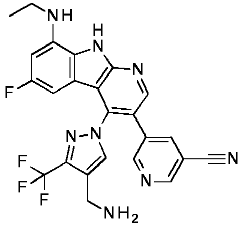 | | |
| | | **CATALYST:** Pd₂dba₃ and XPhos | **MS** (ESI): 510.1 ([M+H]⁺). |

### Example 2.01

### 1-[5-[8-(Ethylamino)-6-fluoro-4-[3-(trifluoromethyl)pyrazol-1-yl]-9H-pyrido[2,3-b]indol-3-yl]pyrimidin-2-yl]cyclopropanecarboxylic acid

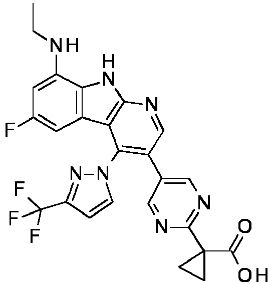

### Step (a) Preparation of tert-butyl (3-chloro-6-fluoro-4-(3-(trifluoromethyl)-1H-p_{.}^{y}razol-1-yl)-9H-pyrido[2,3-b]indol-8-yl)(ethyl)carbamate

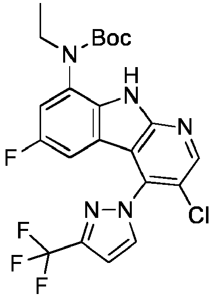

A mixture of *tert-*butyl (3,4-dichloro-6-fluoro-9H-pyrido[2,3-b]indol-8-yl)(ethyl)carbamate (1.1 g, 2.77 mmol, as the **"CORE"** in table 2), 3-(trifluoromethyl)-1H-pyrazole (753.6 mg, 5.54 mmol, as the **"NUCLEOPHILE"** in table 2) and K₂CO₃ (1.1 g, 8.31 mmol) in DMSO (15 mL) was stirred at 110 °C for 12 h. After cooled back to r.t., the mixture was poured into water (100 mL), and extracted with EtOAc (100 mL) three times. The combined organics were dried over anhy. Na₂SO₄, filtered, and concentrated *in vacuo* to give the crude product, which was purified by column chromatography on silica gel eluted with (petroleum ether: EtOAc= 5:1, v/v) to give the *tert*-butyl (3-chloro-6-fluoro-4-(3-(trifluoromethyl)-1H-pyrazol-1-yl)-9H-pyrido[2,3-b]indol-8-yl)(ethyl)carbamate(1.2 g, 85.7% yield) as a white solid, MS (ESI) : 498.1 ([{³⁵Cl}M+H]⁺).

### Step (b) Preparation of ethyl 1-(5-(8-((tert-butoxycarbonyl)(ethyl)amino)-6-fluoro-4-(3-(trifluoromethyl)-1H-pyrazol-1-yl)-9H-pyrido[2,3-b]indol-3-yl)pyrimidin-2-yl)cyclopropanecarboxylate

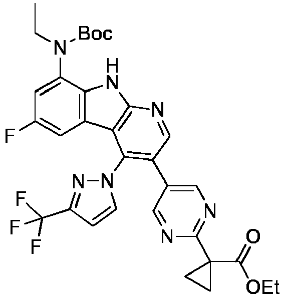

A solution of *tert*-butyl (3-chloro-6-fluoro-4-(3-(trifluoromethyl)-1H-pyrazol-1-yl)-9H-pyrido[2,3-b]indol-8-yl)(ethyl)carbamate (100 mg, 0.2 mmol), (2-(1-(ethoxycarbonyl)cyclopropyl)pyrimidin-5-yl)boronic acid (94.8 mg, 0.4 mmol, as the **"BORONIC REAGENT"** in table 2) and K₃PO₄ (127.8 mg, 0.6 mmol) in THF (4 mL) and H₂O (0.4 mL) was degassed with N₂ for five times before Pd-Ad₂nBuP Biphenyl (13.3 mg, 0.02 mmol, as the **"CATALYST"** in table 2) was added to the mixture at 20 °C under N₂. After the reaction solution was degassed with N₂ for five times again, it was stirred at 60 °C for 12 h under N₂. The mixture was cooled back to r.t., poured into water (100 mL), and extracted with EtOAc (100 mL) three times. The combined organics were dried over anhy. Na₂SO₄, filtered, and concentrated *in vacuo* to give the crude product, which was purified by Prep-TLC (petroleum ether: EtOAc= 2:1) to give ethyl 1-(5-(8-((*tert*-butoxycarbonyl)(ethyl)amino)-6-fluoro-4-(3-(trifluoromethyl)-1H-pyrazol-1-yl)-9H-pyrido[2,3-b]indol-3-yl)pyrimidin-2-yl)cyclopropanecarboxylate(132.3 mg, 50.3% yield) as a yellow solid, MS (ESI) : 654.2([M+H]⁺).

### Step (c) Preparation of 1-(5-(8-((tert-butoxycarbonyl)(ethyl)amino)-6-fluoro-4-(3-(trifluoromethyl)-1H-pyrazol-1-yl)-9H-pyrido[2,3-b]indol-3-yl)pyrimidin-2-yl)cyclopropanecarboxylic acid

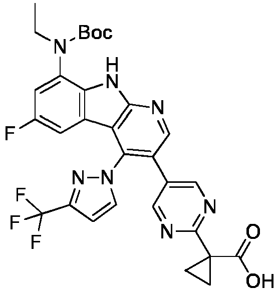

A solution of ethyl 1-(5-(8-((*tert*-butoxvcarbonyl)(ethyl)amino))-6-fluoro-4-(3-(trifluoromethyl)-1H-pyrazol-1-yl)-9H-pyrido[2,3-b]indol-3-yl)pyrimidin-2-yl)cyclopropanecarboxylate (100 mg, 0.153 mmol) and NaOH (61.3 mg, 1.53 mmol) in EtOH (3 mL) and H₂O (1 mL) was stirred at 20 °C for 2 h. Then the mixture was poured into water (50.0 mL), and adjusted to PH = 3 with aq. HCl solution (2N), and extracted with DCM (100 mL) three times. The combined organics were dried over anhy. Na₂SO₄, filtered, and concentrated *in vacuo* to give the crude product, which was used in the next step directly (92.3 mg, 96.4% yield). MS (ESI): 626.1 ([M+H]⁺).

### Step (d) Preparation of 1-[5-[8-(ethylamino)-6-fluoro-4-[3-(trifluoromethyl)pyrazol-1-yl1]-9H-pyrido[2,3-b]indol-3-y1]pyrimidin-2-yl]cyclopropanecarboxylic acid

To the solution of 1-(5-(8-((*tert*-butoxycarbonyl)(ethyl)amino)-6-fluoro-4-(3-(trifluoromethyl)-1H-pyrazol-1-yl)-9H-pyrido[2,3-b]indol-3-yl)pyrimidin-2-yl)cyclopropanecarboxylic acid (0.08 mmol) in DCM (3 mL) was added TFA (2 mL), and the solution was stirred at room temperature for 2 h before it was concentrated *in vacuo.* The residue was purified by prep-HPLC to give 1-[5-[8-(ethylamino)-6-fluoro-4-[3-(trifluoromethyl)pyrazol-1-yl]-9H-pyrido[2,3-b]indol-3-yl]pyrimidin-2-yl]cyclopropanecarboxylic acid (30 mg, 75% yield) as a yellow solid. ¹H NMR (MeOH-*d4*, 400 MHz) δ 8.68 (s, 1H), 8.58 (s, 2H), 8.1-8.1 (m, 1H), 6.99 (d, 1H, *J*=2.3 Hz), 6.52 (dd, 1H, *J*=2.1, 12.0 Hz), 5.98 (dd, 1H, *J*=2.2, 9.0 Hz), 3.3-3.4 (m, 2H), 1.8-1.9 (m, 2H), 1.7-1.8 (m, 2H), 1.43 (t, 3H, *J*=7.2 Hz). MS: 526.1 ([M+H]⁺).

The following examples were prepared in analogy to **Example 2.01,** replacing *tert*-butyl N-(3,4-dichloro-6-fluoro-9H-pyrido[2,3-b]indol-8-yl)-N-ethyl-carbamate **(Intermediate A2)** as the **"CORE"** in step (a), 3-(trifluoromethyl)pyrazole as the **"NUCLEOPHILE"** in step (a), (2-(1-(ethoxycarbonyl)cyclopropyl)pyrimidin-5-yl)boronic acid as the **"BORONIC REAGENT"** in step (b), and Pd-Ad₂nBuP Biphenyl as the **"CATALYST"** in step (b) with the reagents indicated in Table 2 respectively.

**Table 2. Compound synthesis and characterization**

| **No.** | **Compound Name and Structure** | **CORE, NUCLEOPHILE, BORONIC REAGENT, and CATALYST** | **¹H NMR and MS (ESI)** |
|---|---|---|---|
| 2.02 | **1-[5-[6-Fluoro-8-(methylamino)-4-[3-(trifluoromethyl)pyrazol-1-yl]-9H-pyrido[2,3-b]indol-3-** | **CORE:** Intermediate A1 | **¹H NMR** (400 MHz, MeOH-*d4*) δ 8.68 (s, 1H), 8.58 (s, 2H), 8.12 (s, 1H), 6.99 (d, 1H, *J*=2.0 Hz), 6.51 |
| | | **NUCLEOPHILE:** 3-(Trifluoromethyl)pyrazol | |
| | **yl]pyrimidin-2-yl]cyclopropanecarboxylic acid** | e | (dd, 1H, *J*=2.1, 11.8 Hz), 5.98 (dd, 1H, *J*=2.3, 9.0 Hz), 3.01 (s, 3H), 1.85 (m, 2H), 1.77 (m, 2H). |
| | | **BORONIC REAGENT:** (2-(1-(Ethoxycarbonyl)cyclopr opyl)pyrimidin-5-yl)boronic acid | |
| | 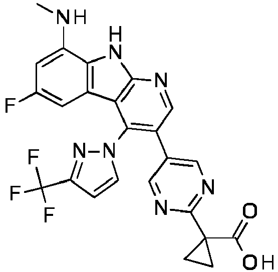 | | |
| | | | **MS** (ESI): 512.1 ([M+H]⁺). |
| | | **CATALYST:** Pd-Ad₂nBuP Biphenyl | |
| | | **SOLVENT:** 1,4-dioxane/H₂O | |
| 2.03 | **3-[5-[8-(Ethylamino)-6-fluoro-4-[3-(trifluoromethyl)pyrazol-1-yl]-9H-pyrido[2,3-b]indol-3-yl]pyrimidin-2-yl]oxy-2,2-dimethyl-propanoic acid** | **CORE:** Intermediate A2 | **¹H NMR** (400 MHz, MeOH-*d4*) δ 8.63 (s, 1H), 8.39 (s, 2H), 8.05 (d, 1H, *J*=1.5 Hz), 6.98 (d, 1H, *J*=2.5 Hz), 6.50 (dd, 1H, *J*=2.3, 12.0 Hz), 5.91 (dd, 1H, *J*=2.3, 9.0 Hz), 4.43 (s, 2H), 3.3-3.4 (m, 1H), 3.29 (m, 2H), 1.43 (t, 3H, *J*=7.0 Hz), 1.34 (s, 6H). |
| | | **NUCLEOPHILE:** 3-(Trifluoromethyl)pyrazol e | |
| | | **BORONIC REAGENT:** Intermediate B2 | |
| | 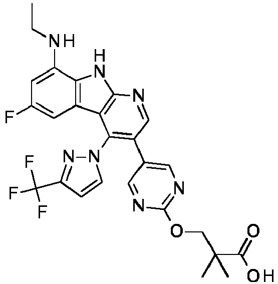 | | |
| | | **CATALYST:** Pd-Ad₂nBuP Biphenyl | |
| | | **SOLVENT:** 1,4-dioxane/H₂O | **MS** (ESI): 558.1 ([M+H]⁺). |
| 2.04 | **3-[5-[8-(Ethylamino)-6-fluoro-4-[3-(trifluoromethyl)pyrazol-1-yl]-9H-pyrido[2,3-b]indol-3-yl]pyrimidin-2-yl]oxy-2,2-dimethyl-propanoic** | **CORE:** Intermediate A2 | **¹H NMR** (400 MHz, MeOH-*d4*) δ 8.57 (s, 1H), 8.09 (s, 2H), 8.02 (d, 1H, *J*=1.5 Hz), 6.98 (d, 1H, *J*=2.3 Hz), 6.49 (dd, 1H, *J*=2.3, 12.0 Hz), 5.87 (dd, |
| | | **NUCLEOPHILE:** 3-(Trifluoromethyl)pyrazol e | |
| | **acid** | **BORONIC** REAGENT: Intermediate B3 | 1H, *J*=2.3, 9.0 Hz), 3.66 (s, 2H), 3.3-3.3 (m, 2H), 1.42 (t, 3H, *J*=7.0 Hz), 1.2-1.2 (m, 2H), 1.0-1.0 (m, 2H). |
| | 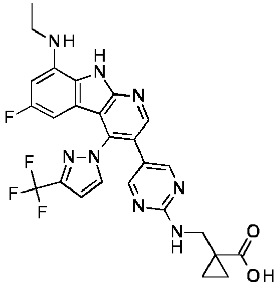 | | |
| | | **CATALYST:** Pd-Ad₂nBuP Biphenyl | |
| | | | **MS** (ESI): 555.1 ([M+H]⁺). |
| | | **SOLVENT:** 1,4-dioxane/H₂O | |
| 2.05 | **3-[[5-[8-(Ethylamino)-6-fluoro-4-[3-(trifluoromethyl)pyrazol-1-yl]-9H-pyrido[2,3-b]indol-3-yl]pyrimidin-2-yl]amino]-2,2-dimethyl-propanoic acid** | **CORE:** Intermediate A2 | **¹H NMR** (400 MHz, MeOH-*d4*) δ 8.57 (s, 1H), 8.11 (s, 2H), 8.0-8.0 (m, 1H), 6.97 (d, 1H, *J*=2.4 Hz), 6.49 (dd, 1H, *J*=2.3, 11.9 Hz), 5.88 (dd, 1H, *J*=2.3, 9.0 Hz), 3.63 (s, 2H), 3.3-3.3 (m, 2H), 1.42 (t, 3H, *J*=7.2 Hz), 1.23 (s, 6H). |
| | | **NUCLEOPHILE:** 3-(Trifluoromethyl)pyrazol e | |
| | | **BORONIC REAGENT:** Intermediate B4 | |
| | 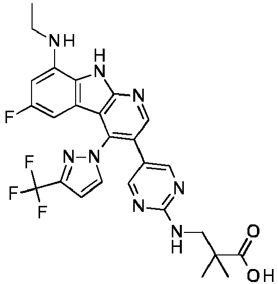 | | |
| | | **CATALYST:** Pd-Ad₂nBuP Biphenyl | |
| | | | **MS** (ESI): 557.1 ([M+H]⁺). |
| | | **SOLVENT:** 1,4-dioxane/H₂O | |

### Example 3.01

### 1-(5-(8-(Ethylamino)-6-fluoro-4-(3-(trifluoromethyl)-1H-pyrazol-1-yl)-9H-pyrido[2,3-b]indol-3-yl)pyrimidin-2-yl)-N-methylcyclopropanecarboxamide

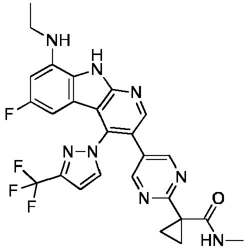

### Step (a) Preparation of tert-butyl ethyl(6-fluoro-3-(2-(1-(methylcarbamoyl)cyclopropyl)pyrimidin-5-yl)-4-(3-(trifluoromethyl)-1H-pyrazol-1-yl)-9H-pyrido[2,3-b]indol-8-yl)carbamate

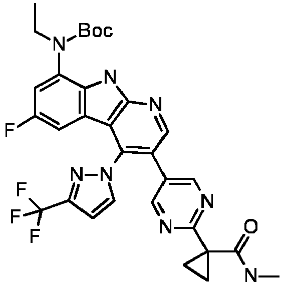

A solution of 1-(5-(8-((*tert*-butoxycarbonyl)(ethyl)amino)-6-fluoro-4-(3-(trifluoromethyl)-1H-pyrazol-1-yl)-9H-pyrido[2,3-b]indol-3-yl)pyrimidin-2-yl)cyclopropanecarboxylic acid (40 mg, 0.06 mmol, Example 2.01/step c), methanamine hydrochloride (21.4 mg, 0.32 mmol, as the **"AMINE"** in table 3), HATU (29.2 mg, 0.08 mmol) and DIPEA (24.7 mg, 0.19 mmol) in DMF (3 mL) was stirred at 20 °C for 12 h. After LC-MS showed the starting material was consumed, the mixture was poured into water (50 mL), and extracted with EtOAc (100 mL) three times. The combined organics were dried over anhy. Na₂SO₄, filtered, and concentrated *in vacuo* to give the crude *tert-*butyl ethyl(6-fluoro-3-(2-(1-(methylcarbamoyl)cyclopropyl)pyrimidin-5-yl)-4-(3-(trifluoromethyl)-1H-pyrazol-1-yl)-9H-pyrido[2,3-b]indol-8-yl)carbamate (40 mg, 97.6% yield) as a yellow solid, which was used in the next step directly. MS (ESI): 639.3 ([M+H]⁺).

### Step (b) Preparation of 1-(5-(8-(ethylamino)-6-fluoro-4-(3-(trifluoromethyl)-1H-pyrazol-1-yl)-9H-pyrido[2,3-b]indol-3-yl)pyrimidin-2-yl)-N-methylcyclopropanecarboxamide

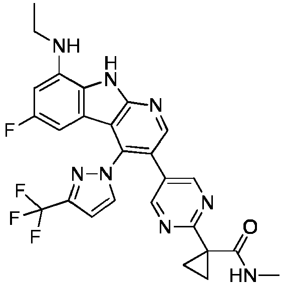

To a solution of *tert-*butyl ethyl(6-fluoro-3-(2-(1-(methylcarbamoyl)cyclopropyl)pyrimidin-5-yl)-4-(3-(trifluoromethyl)-1H-pyrazol-1-yl)-9H-pyrido[2,3-b]indol-8-yl)carbamate (30 mg, 0.05 mmol) in DCM (2 mL) was added TFA (1 mL), and the reaction mixture was stirred at 20 °C for 1 h. After LC-MS showed the starting material was consumed, the mixture was concentrated *in vacuo* to give the crude product, which was purified by Prep-HPLC (0.5% TFA in water) to give the 1-(5-(8-(ethylamino)-6-fluoro-4-(3-(trifluoromethyl)-1H-pyrazol-1-yl)-9H-pyrido[2,3-b]indol-3-yl)pyrimidin-2-yl)-N-methylcyclopropanecarboxamide (12 mg, 47.4% yield) as a yellow solid. MS (ESI): 539.2 ([M+H]⁺), ¹H NMR (400 MHz, DMSO-*d6*): δ 12.18 (s, 1H), 8.92-8.93 (d, J= 4 Hz, 1H), 8.74 (s, 1H), 8.51 (s, 2H), 8.37-8.75 (d, J= 1.6 Hz, 1H), 7.16-7.17 (d, J= 2.4 Hz, 1H), 6.48-6.52 (m, 1H), 5.91 (br, 1H), 5.76-5.78 (m, 1H), 3.17-3.27 (m, 2H), 2.71-2.72 (m, 3H), 1.53-1.54 (m, 2H), 1.41-1.42 (m, 2H), 1.30-1.34 (m, 3H).

The following examples were prepared in analogy to **Example 3.01,** replacing methyl amine as the **"AMINE"** in step (a) with the reagents indicated in Table 3.

**Table 3. Compound synthesis and characterization**

| **No.** | **Compound Name and Structure** | **AMINE** | **¹H NMR and MS (ESI)** |
|---|---|---|---|
| 3.02 | **1-(5-(8-(Ethylamino)-6-fluoro-4-(3-(trifluoromethyl)-1H-pyrazol-1-yl)-9H-pyrido[2,3-b]indol-3-yl)pyrimidin-2-yl)-N-methoxycyclopropanecarbo xamide** | **AMINE:** O-methylhydroxylamine hydrochloride | ¹H NMR (400 MHz, DMSO-*d6*): δ 12.18 (s, 1H), 11.51 (s, 1H), 8.74 (s, 1H), 8.50 (s, 1H), 8.36 (s, 1H), 7.16 (s, 1H), 6.48-6.51 (m, 1H), 5.73-5.75 (m, 1H), 3.63 (s, 3H), 3.25-3.27 (m, 2H), 1.46 (m, 2H), 1.41 (m, |
| | 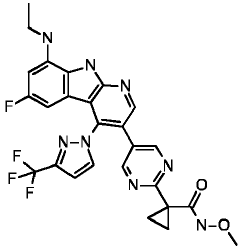 | | 2H), 1.30-1.34 (m, 3H). |
| | | | MS (ESI): 555.3 ([M+H]⁺). |
| 3.03 | **5-[8-(Ethylamino)-6-fluoro-4-[3-(trifluoromethyl)pyrazol-1-yl]-9H-pyrido[2,3-b]indol-3-yl]-N-methoxy-pyridine-3-carboxamide** | **AMINE:** O-methylhydroxylamine hydrochloride | **¹H NMR** (400 MHz, MeOH-*d4*) δ 8.7-8.8 (m, 1H), 8.5-8.6 (m, 1H), 8.4-8.4 (m, 1H), 7.9-8.0 (m, 1H), 7.8-7.9 (m, 1H), 6.7-6.8 (m, 1H), 6.3-6.4 (m, 1H), 5.8-5.9 (m, 1H), 3.7-3.8 (m, 3H), 1.3-1.3 (m, 3H). |
| | 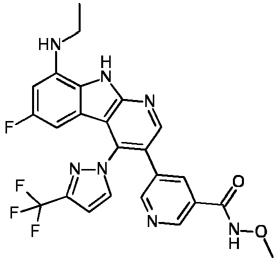 | | |
| | | | MS (ESI): 514.1 ([M+H]⁺). |
| 3.04 | **5-[8-(Ethylamino)-6-fluoro-4-[3-(trifluoromethyl)pyrazol-1-yl]-9H-pyrido[2,3-b]indol-3-yl]pyrimidine-2-carboxamide** | **AMINE:** Ammonia | **¹H NMR** (400 MHz, MeOH-*d4*) δ 8.7-8.8 (m, 3H), 8.0-8.1 (m, 1H), 6.9-7.0 (m, 1H), 6.5-6.6 (m, 1H), 5.9-6.0 (m, 1H), 3.3-3.3 (m, 2H), 1.3-1.5 (m, 3H). |
| | | | **MS** (ESI): 485.2 ([M+H]⁺). |
| | 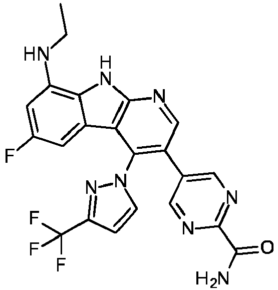 | | |
| 3.05 | **2-[5-[8-(Ethylamino)-6-fluoro-4-[3-(trifluoromethyl)pyrazol-1-yl]-9H-pyrido[2,3-b]indol-3-yl]-3-pyridyl]acetamide** | **AMINE:** Ammonia | **¹H NMR** (400 MHz, DMSO-d₆) δ ppm: 12.08 (s, 1 H) 8.68 (s, 1 H) 8.40 (s, 1 H) 8.24 (dd, *J*=4.77, 1.71 Hz, 2 H) 7.44 - 7.55 (m, 2 H) 7.02 - 7.13 (m, 1 H) 6.96 (s, 1 H) 6.43 - 6.55 (m, 1 H) 5.80 - 5.95 (m, 1 H) 5.69 (dd, *J*=9.17, 2.20 Hz, 1 H) 3.37 (s, 2 H) 3.26 (br dd, *J*=6.97, 4.89 Hz, 2 H) 1.28 - 1.36 (m, 3 H). |
| | 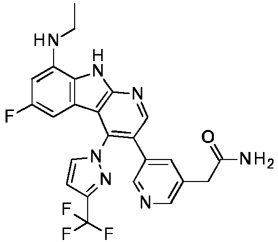 | | |
| | | | **MS** (ESI): 498.2 ([M+H]⁺) |
| 3.06 | **5-[8-(ethylamino)-6-fluoro-4-[3-(trifluoromethyl)pyrazol-1-yl]-9H-pyrido[2,3-b]indol-3-yl]-N-(2-methoxyethyl)pyridine-3-carboxamide** | **AMINE:** 2-Methoxyethanamine | **¹H NMR** (400 MHz, DMSO-*d*₆), *δ* ppm: 12.22 (s, 1H), 8.91 (d, *J*=2.0Hz, 1H), 8.38 (d, *J*=2.0Hz, 1H), 8.26 (s, 1H), 8.09 (s, 1H), 7.07 (d, *J*=2.4Hz, 1H), 6.49 (m, 1H), 5.95 (s, 1H), 5.76 (m, 1H), 3.47-3.42 (m, 4H), 3.27-3.22 (m, 5H), 1.32 (t, |
| | 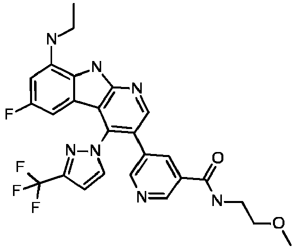 | | *J*=7.2Hz, 3H). |
| | | | **MS** (ESI): 542.1 ([M+H]+). |
| 3.07 | **5-[8-(ethylamino)-6-fluoro-4-[3-(trifluoromethyl)pyrazol-1-yl]-9H-pyrido[2,3-b]indol-3-yl]-N-[1-(methoxymethyl)cyclopropy l]pyridine-3-carboxamide** | **AMINE:** 1 (Methoxymethyl)cyclopr opanamine | **¹H NMR** (400 MHz, DMSO-*d6*) *δ* ppm: 12.16 (s, 1H), 8.93 (s, 1H), 8.89 (d, *J*=2.0Hz, 1H), 8.77 (s, 1H), 8.36 (d, *J*=2.0Hz, 1H), 8.24 (d, J=2.0Hz, 1H), 8.07 (t, *J*=2.0Hz, 1H), 7.06 (d, *J*=2.0Hz, 1H), 6.49 (m, 1H), 5.91 (brs, 1H), 5.76 (m, 1H), 3.47 (s, 2H), 3.28-3.25(m, 5H), 1.32 (t, *J*=7.2Hz, 3H), 0.81-0.75(m, 4H). |
| | 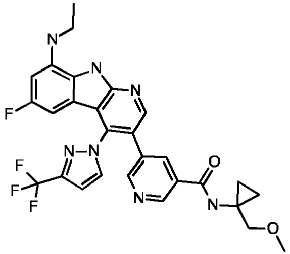 | | |
| | | | **MS** (ESI): 568.1 ([M+H]⁺). |

### Example 4.01

### 1-(3-(5-Cyanopyridin-3-yl)-8-(ethylamino)-6-fluoro-9H-pyrido[2,3-b]indol-4-yl)-3-(trifluoromethyl)-1H-pyrazole-4-carboxamide

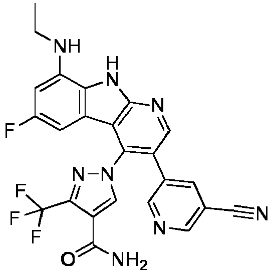

The titled compound was synthesized according to the following scheme:

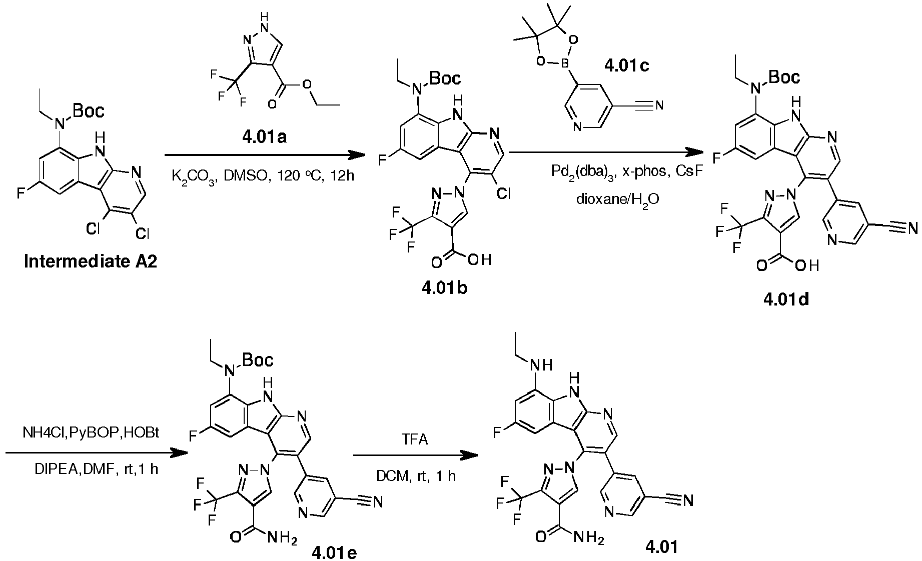

### Step (a) Preparation of 1-(8-((tert-butoxvcarbonyl)(ethyl)amino)-3-chloro-6-fluoro-9H-pyrido[2,3-b]indol-4-yl)-3-(trifluoromethyl)-1H-prazole-4-carboxylic acid (compound 4.01b)

To a solution of *tert-*butyl (3,4-dichloro-6-fluoro-9H-pyrido[2,3-b]indol-8-yl)(ethyl)carbamate (440 mg, 1.1 mmol, **Intermediate A2)** and K₂CO₃ (610 mg, 4.42 mmol) in DMSO (6 mL) was added ethyl 3-(trifluoromethyl)-1H-pyrazole-4-carboxylate (920 mg, 4.42 mmol, compound 4.01a), the reaction solution was stirred at 120 °C for 12 h. After cooled back to r.t., the mixture was poured into water and extracted by EtOAc. The organic layer was washed by brine, dried with anhy. Na₂SO₄, filtered, and concentrated *in vacuo* to give the crude product, which was purified by Prep-TLC and further purified by silica gel flash chromatography (TFA as additive) to give 1-(8-((*tert*-butoxycarbonyl)(ethyl)amino)-3-chloro-6-fluoro-9H-pyrido[2,3-b]indol-4-yl)-3-(trifluoromethyl)-1H-pyrazole-4-carboxylic acid (200 mg, 33.4% yield). MS (ESI): 542.2 ({³⁵Cl}M+H)⁺, 544.2 ({³⁷Cl}M+H)⁺, 564.2 ({³⁵Cl}M+Na)⁺.

### Step (b) Preparation of 1-(8-((tert-butoxycarbonyl)(ethyl)amino)-3-(5-cyanopyridin-3-yl)-6-fluoro-9H-pyrido[2,3-b]indol-4-yl)-3-(trifluoromethyl)-1H-pyrazole-4-carboxylic acid (compound 4.01d)

A solution of 1-(8-((*tert*-butoxycarbonyl)(ethyl)amino)-3-chloro-6-fluoro-9H-pyrido[2,3-b]indol-4-yl)-3-(trifluoromethyl)-1H-pyrazole-4-carboxylic acid (compound 4.01b) (200 mg, 0.369 mmol), 5-(4,4,5,5-tetramethyl-1,3,2-dioxaborolan-2-yl)nicotinonitrile (compound 4.01c) (170 mg, 0.738 mmol) and CsF (224 mg, 1.476 mmol) in dioxane/H₂O (6.0 mL, v/v=10:1) was degassed with N₂ for five times, and then Pd₂(dba)₃ (34 mg, 0.037 mmol) and X-phos (36 mg, 0.074 mmol) were added into the mixture at 0 °C under N₂. The reaction solution was degassed with N₂ for five times again, and then stirred for 12 h at 110 °C for 12 h under N₂. After cooled back to r.t., the mixture was poured into water and extracted by EtOAc. The organic layer was washed by brine and dried with anhy. Na₂SO₄ and filtered. The organic layer was concentrated *in vacuo* to give the crude product, which was purified by silica gel flash chromatography (TFA as additive) to give 1-(8-((*tert*-butoxycarbonyl)(ethyl)amino)-3-(5-cyanopyridin-3-yl)-6-fluoro-9H-pyrido[2,3-b]indol-4-yl)-3-(trifluoromethyl)-1H-pyrazole-4-carboxylic acid (200 mg, 88.9% yield) as a yellow solid. MS (ESI): 610.2 (M+H)⁺, 632.1 (M+Na)⁺.

### Step (c) Preparation of tert-butyl (4-(4-carbamoyl-3-(trifluoromethyl)-1H-pyrazol-1-yl)-3-(5-yvanopyridin-3-yl)-6-fluoro-9H-pyrido[2,3-b]indol-8-yl)(ethyl)carbamate (compound 4.01e)

To a solution of 1-(8-((*tert*-butoxycarbonyl)(ethyl)amino)-3-(5-cyanopyridin-3-yl)-6-fluoro-9H-pyrido[2,3-b]indol-4-yl)-3-(trifluoromethyl)-1H-pyrazole-4-carboxylic acid_(100 mg, 0.16 mmol, compound 4.01d), NH₄C1 (43 mg, 0.8 mmol), HOBt (65 mg, 0.48 mmol), and PyBOP (250 mg, 0.48 mmol) in DMF (5.0 mL) was added DIPEA (124 mg, 0.96 mmol) under N₂. The reaction mixture was stirred at 25 °C for 1 h, then purified by silica gel flash chromatography (TFA as additive) to give *tert-*butyl (4-(4-carbamoyl-3-(trifluoromethyl)-1H-pyrazol-1-yl)-3-(5-cyanopyridin-3-yl)-6-fluoro-9H-pyrido[2,3-b]indol-8-yl)(ethyl)carbamate (90 mg, 90.2% yield) as a yellow solid.MS (ESI): 609.3 (M+H)⁺, 631.2 (M+Na)⁺.

### Step (d) Preparation of 1-(3-(5-cyanopyridin-3-yl)-8-(ethylamino)-6-fluoro-9H-pyridor2,3-b]lindol-4-yl)-3-(trifluoromethyl)-1H-pyrazole-4-carboxamide (compound 4.01)

To a solution of *tert-*butyl (4-(4-carbamoyl-3-(trifluoromethyl)-1H-pyrazol-1-yl)-3-(5-cyanopyridin-3-yl)-6-fluoro-9H-pyrido[2,3-b]indol-8-yl)(ethyl)carbamate (80.0 mg, 0.131 mmol, compound 4.01e) in DCM (3 mL) was added TFA (1.5 mL), and the resulting mixture was stirred at 25 °C for 1 h. The reaction mixture was concentrated *in vacuo* to give a crude product, which was purified by Prep-HPLC (0.5% TFA in water) to give 1-(3-(5-cyanopyridin-3-yl)-8-(ethylamino)-6-fluoro-9H-pyrido[2,3-b]indol-4-yl)-3-(trifluoromethyl)-1H-pyrazole-4-carboxamide. (50 mg, 74.8% yield) as a yellow solid. ¹H NMR (400 MHz, DMSO-d₆) δ:ppm 12.23 (s, 1 H) 9.01 (d, J=1.76 Hz, 1 H), 8.77 (s, 1 H), 8.56 ~ 8.69 (m, 2 H), 8.24 (t, J=1.88 Hz, 1 H), 7.77 (br. s., 1 H), 7.42 (br. s., 1 H), 6.53 (dd, J=12.17, 1.88 Hz, 1 H), 5.79 ~ 6.00 (m, 2 H), 3.27 (q, J=7.03 Hz, 2 H), 1.33 (t, J=7.15 Hz, 3 H). MS (ESI) : 509.2 (M+H)⁺.

### Example 5.01

### 5-[8-(ethylamino)-6-fluoro-4-[4-(hydroxymethyl)-3-(trifluoromethyl)pyrazol-1-yl]-9H-pyrido[2,3-b]indol-3-yl]pyridine-3-carbonitrile

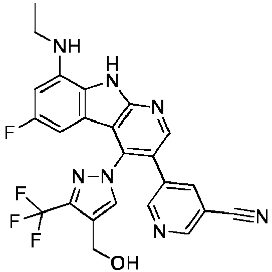

The titled compound was synthesized according to the following scheme:

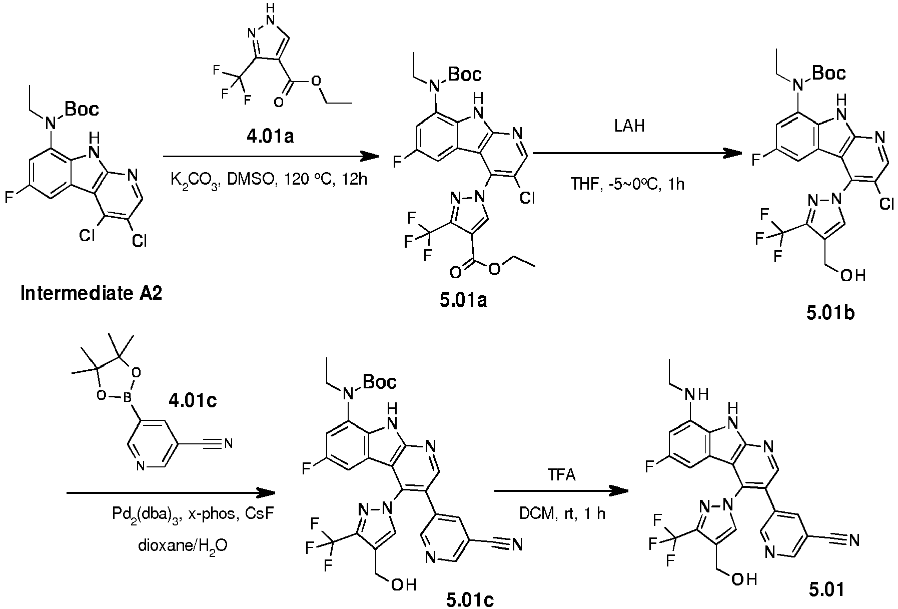

### Step (a) Preparation of ethyl 1-(8-((tert-butoxycarbonyl)(ethyl)amino)-3-chloro-6-fluoro-9H-pvrido[2,3-blindol-4-yl)-3-(trifluoromethyl)-1H-pyrazole-4-carboxylate (compound 5.01a)

To a solution of *tert-*butyl (3,4-dichloro-6-fluoro-9H-pyrido[2,3-b]indol-8-yl)(ethyl)carbamate (440 mg, 1.1 mmol, **Intermediate A2)** and K₂CO₃ (610 mg, 4.42 mmol) in *DMSO (6 mL) was added ethyl 3-(trifluoromethyl)-1H-pyrazole-4-carboxylate (920 mg, 4.42* mmol, compound 4.01a), and the reaction solution was stirred at 120 °C for 12 h. After cooled back to r.t., the mixture was poured into water and extracted by EtOAc (100 mL) two times. The combined organics were washed by brine, dried with anhy. Na₂SO₄, filtered, and concentrated *in vacuo* to give the crude product, which was purified by Prep-TLC and further purified by silica gel flash chromatography (TFA as additive) to give ethyl 1*-*(8-((*tert-*butoxycarbonyl)(ethyl)amino)-3-chloro-6-fluoro-9H-pyrido[2,3-b]indol-4-yl)-3-(trifluoromethyl)-1H-pyrazole-4-carboxylate (330 mg, 52.4% yield). MS (ESI): 570.1 ({³⁵Cl}M+H)⁺, 572.2 ({³⁷Cl}M+H)⁺.

### Step (b) Preparation of tert-butyl (3-chloro-6-fluoro-4-(4-(hydroxymethyl)-3-(trifluoromethyl)-1H-pyrazol-1-yl)-9H-pyrido[2,3-b]indol-8-yl)(ethyl)carbamate (compound 5.01b)

To a solution of ethyl 1-(8-((*tert*-butoxycarbonyl)(ethyl)amino)-3-chloro-6-fluoro-9H-pyrido[2,3-b]indol-4-yl)-3-(trifluoromethyl)-1H-pyrazole-4-carboxylate (180 mg, 0.316 mmol, compound 5.01a) in THF (0.316 mL) was added LAH (30 mg, 0.79 mmol) at 0 °C under N₂, and the resulting mixture was stirred at 0 °C for 1 h. The mixture was then poured into water and extracted by EtOAc (100 mL) two times. The combined organics were washed by brine, dried over anhy. Na₂SO₄, filtered, and concentrated *in vacuo* to give *tert-*butyl (3-chloro-6-fluoro-4-(4-(hydroxymethyl)-3-(trifluoromethyl)-1H-pyrazol-1-yl)-9H-pyrido[2,3-b]indol-8-yl)(ethyl)carbamate (140 mg, 84% yield) as a yellow solid. It was used in the next step directly without purification. MS (ESI): 528.1 ({³⁵Cl}M+H)⁺, 530.1 ({³⁷Cl}M+H)⁺.

### Step (c) Preparation of tert-butyl (3-(5-cyanopyridin-3-yl)-6-fluoro-4-(4-(hydroxymethyl)-3-(trifluoromethyl)-1H-pyrazol-1-yl)-9H-pyrido[2,3-b]indol-8-yl)(ethyl)carbamate (compound 5.01c)

A solution of *tert-*butyl (3-chloro-6-fluoro-4-(4-(hydroxymethyl)-3-(trifluoromethyl)-1H-pyrazol-1-yl)-9H-pyrido[2,3-b]indol-8-yl)(ethyl)carbamate (130 mg, 0.246 mmol, compound 5.01b), 5-(4,4,5,5-tetramethyl-1,3,2-dioxaborolan-2-yl)nicotinonitrile (113 mg, 0.493 mmol), and CsF (150 mg, 0.986 mmol) in dioxane/H₂O (4 mL, v/v=10:1) was degassed with N₂ for five times, and then Pd₂(dba)₃ (22.5 mg, 0.0246 mmol) and XPhos (23.5 mg, 0.0493 mmol) was added into the mixture at 0 °C under N₂. The reaction solution was degassed with N₂ for five times again, and the mixture was stirred at 110 °C for 12 h. After cooled back to r.t., the mixture was poured into water and extracted by EtOAc (100 mL) two times. The combined organics were washed by brine, dried over anhy. Na₂SO₄, filtered, and concentrated *in vacuo* to give the crude product, which was purified by Prep-TLC to give *tert-*butyl (3-(5-cyanopyridin-3-yl)-6-fluoro-4-(4-(hydroxymethyl)-3-(trifluoromethyl)-1H-pyrazol-1-yl)-9H-pyrido[2,3-b]indol-8-yl)(ethyl)carbamate (120 mg, yield: 81.8% yield) as a pale yellow solid. MS (ESI): 596.3 (M+H)⁺, 618.2 (M+Na)⁺.

### Step (d) Preparation of 5-[8-(ethylamino)-6-fluoro-4-[4-(hydroxymethyl)-3-(trifluoromethyl)pyrazol-1-yl]-9H-pyrido[2,3-b]indol-3-yl]pyridine-3-carbonitrile (Example 5.01)

To a solution of *tert-*butyl (3-(5-cyanopyridin-3-yl)-6-fluoro-4-(4-(hydroxymethyl)-3-(trifluoromethyl)-1H-pyrazol-1-yl)-9H-pyrido[2,3-b]indol-8-yl)(ethyl)carbamate (110 mg, 0.185 mmol, compound 5.01c) in DCM (3 mL) was added TFA (1.5 mL). The reaction mixture was stirred at 28 °C for 1 h, then concentrated *in vacuo* to give the crude product, which was purified by Prep-HPLC (0.5% TFA in water) to give 5-(8-(ethylamino)-6-fluoro-4-(4-(hydroxymethyl)-3-(trifluoromethyl)-1H-pyrazol-1-yl)-9H-pyrido[2,3-b]indol-3-yl)nicotinonitrile (77 mg, 84.2% yield) as a yellow solid. ¹H NMR (400 MHz, DMSO-*d6*) *δ*ppm: 12.16 (s, 1 H), 8.99 (d, *J*=1.25 Hz, 1 H), 8.74 (s, 1 H), 8.59 (d, *J*=1.76 Hz, 1 H), 8.08 ~ 8.24 (m, 2 H), 6.51 (d, *J*=12.30 Hz, 1 H), 5.92 (d, *J*=9.03 Hz, 1 H), 4.51 (s, 4 H), 3.26 (q, *J*=6.94 Hz, 2 H), 1.32 (t, *J*=7.03 Hz, 3 H). MS (ESI): 496.2 (M+H)⁺, 248.6 (M/2+H)⁺, 518.2 (M+Na)⁺.

### Example 6.01

### 5-[[5-[8-(ethylamino)-6-fluoro-4-[3-(trifluoromethyl)pyrazol-1-yl]-9H-pyrido[2,3-b]indol-3-yl]-3-pyridyl]methyl]-3H-1,3,4-oxadiazol-2-one

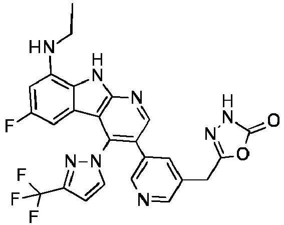

The titled compound was synthesized according to the following scheme:

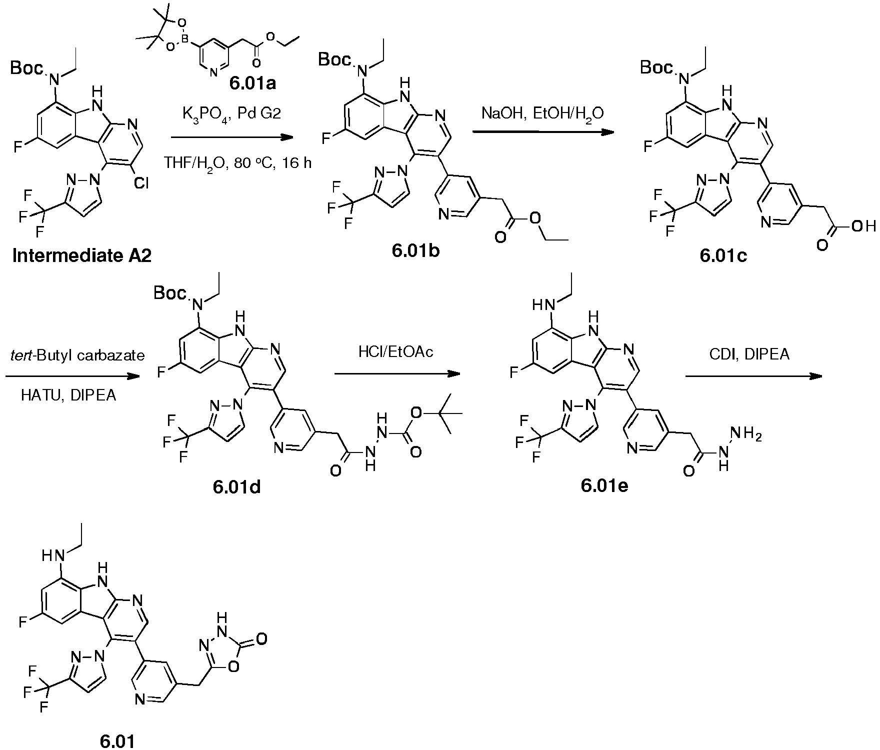

### Step (a) Preparation of ethyl 2-(5-(8-((tert-butoxycarbonyl)(ethyl)amino)-6-fluoro-4-(3-(trifluoromethyl)-1H-pyrazol-1-yl)-9H-pyrido[2,3-b]indol-3-yl)pyridin-3-yl)acetate (compound 6.01b)

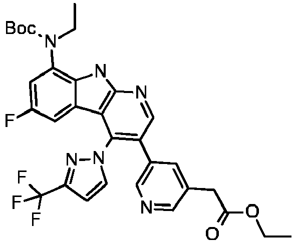

To a mixture of *tert-*butyl (3-chloro-6-fluoro-4-(3-(trifluoromethyl)-1H-pyrazol-1-yl)-9H-pyrido[2,3-b]indol-8-yl)(ethyl)carbamate (100 mg, 0.2 mmol), ethyl 2-(5-(4,4,5,5-tetramethyl-1,3,2-dioxaborolan-2-yl)pyridin-3-yl)acetate (83.56 mg, 0.4 mmol) in THF (2 mL) and water (0.2 mL) was added K₃PO₄ (127.91 mg, 0.6 mmol) and cataCXium^{®} A Pd G2 (13.43 mg, 0.02 mmol, Sigma-Aldrich, Catalog #: 761311). the mixture was stirred at 80 °C for 16 h under argon. After cooled back to r.t., the mixture was concentrated in vacuo to give the crude product, which was purified by Prep-TLC (petroleum ether/EtOAc = 2/1) to give ethyl 2-(5-(8-((*tert-*butoxycarbonyl)(ethyl)amino)-6-fluoro-4-(3-(trifluoromethyl)-1H-pyrazol-1-yl)-9H-pyrido[2,3-b]indol-3-yl)pyridin-3-yl)acetate (100 mg, 79.5% yield) as a brown solid. MS (ESI): 627.2 [(M+H)⁺].

### Step (b) Preparation of 2-(5-(8-((tert-butoxycarbonyl)(ethyl)amino)-6-fluoro-4-(3-(trifluoromethyl)-1H-pyrazol-l-yl)-9H-pyrido[2,3-b]indol-3-yl)pyridin-3-yl)acetic acid (compound 6.01c)

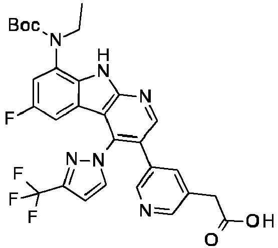

To a solution of ethyl 2-(5-(8-((*tert*-butoxycarbonyl)(ethyl)amino)-6-fluoro-4-(3-(trifluoromethyl)-1H-pyrazol-1-yl)-9H-pyrido[2,3-b]indol-3-yl)pyridin-3-^{y}l)acetate (100 mg, 0.160 mmol) in EtOH (5 mL) was added aq. NaOH solution (1 mL, 1 N, 1 mmol). After been stirred at 20 °C for 1 h, the mixture was adjusted to pH = 4-5 with aq. HCl solution (1N) and then extracted with EtOAc (20 mL) three times. The combined organics were dried over anhy. Na₂SO₄, filtered, and concentrated *in vacuo* to give 2-(5-(8-((*tert*-butoxycarbonyl)(ethyl)amino)-6-fluoro-4-(3-(trifluoromethyl)-1H-pyrazol-1-yl)-9H-pyrido[2,3-b]indol-3-yl)pyridin-3-yl)acetic acid (90 mg, 94.2% yield) as a yellow solid. MS (ESI): 599.0 [(M+H)⁺].

### Step (c) Preparation of tert-butyl 2-(2-(5-(8-((tert-butoxycarbonyl)(ethyl)amino)-6-fluoro-4-(3-(trifluoromethyl)-1H-pyrazol-1-yl)-9H-pyrido[2,3-b]indol-3-yl)t)pyridin-3-yl)acetyl)hydrazine-carboxylate (compound 6.01d)

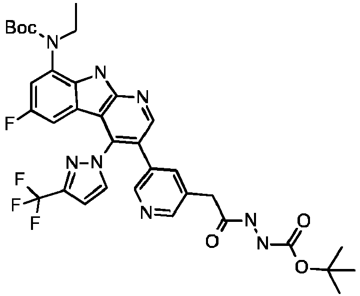

A solution of 2-(5-(8-((*tert*-butoxycarbonyl)(ethyl)amino)-6-fluoro-4-(3-(trifluoromethyl)-1H-pyrazol-1-yl)-9H-pyrido[2,3-b]indol-3-yl)pyridin-3-yl)acetic acid (90 mg, 0.15 mmol), HATU (68.61 mg, 0.180 mmol), and DIPEA (0.08 mL, 0.450 mmol) in DMF (3 mL) was stirred at 20 °C for 0.5 h, then *tert*-Butyl carbazate (23.85 mg, 0.180 mmol) was added and the resulting mixture was stirred at 20 °C for another 16 h. EtOAc (30 mL) was added and the mixture was washed with brine (20 mL), satd. aq. NaHCOs solution (20 mL). The organic layer was dried over anhy. Na₂SO₄, filtered, and concentrated *in vacuo* to give *tert-*butyl 2-(2-(5-(8-((*tert-*butoxycarbonyl)(ethyl)amino)-6-fluoro-4-(3-(trifluoromethyl)-1H-pyrazol-1-yl)-9H-pyrido[2,3-b]indol-3-yl)pyridin-3-yl)acetyl)hydrazine-carboxylate (100 mg, 93.3% yield) as a yellow solid. MS (ESI): 713.2 [(M+H)⁺].

### Step (d) Preparation of 2-(5-(8-(ethylamino)-6-fluoro-4-(3-(trifluoromethyl)-1H-pyrazol-1-yl)-9H-pyrido[2,3-b]indol-3-yl)pyridin-3-yl)acetohydrazide (compound 6.01e)

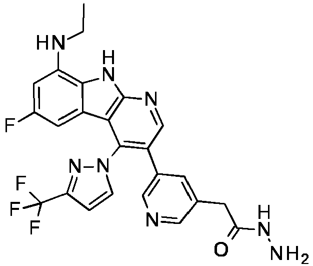

To a mixture of *tert-*butyl 2-(2-(5-(8-((*tert*-butoxycarbonyl)(ethyl)amino)-6-fluoro-4-(3-(trifluoromethyl)-1H-pyrazol-1-yl)-9H-pyrido[2,3-b]indol-3-yl)pyridin-3-yl)acetyl)hydrazine-carboxylate (100 mg, 0.14 mmol) in EtOAc (5 mL) was added HCl solution in EtOAc (1.0 mL, 4N, 4 mmol). The mixture was stirred at 20 °C for 1 h before it was concentrated *in vacuo* to give a crude product of 2-(5-(8-(ethylamino)-6-fluoro-4-(3-(trifluoromethyl)-1H-pyrazol-1-yl)-9H-pyrido[2,3-b]indol-3-yl)pyridin-3-yl)acetohydrazide (100 mg, 65.2% yield) as a yellow solid. MS (ESI): 513.1 [(M+H)⁺].

### Step (e) Preparation of 5-[[5-[8-(ethylamino)-6-fluoro-4-[3-(trifluoromethyl)pyrazol-1-yl]-9H-pvrido[2,3-b]indol-3-yl]-3-pyridyl]methyl]-3H-1,3,4-oxadiazol-2-one (Example 6.01)

To a solution of 2-(5-(8-(ethylamino)-6-fluoro-4-(3-(trifluoromethyl)-1H-pyrazol-1-yl)-9H-pyrido[2,3-b]indol-3-yl)pyridin-3-yl)acetohydrazide (100 mg, 0.2 mmol) in THF (5 mL) was added DIPEA (0.1 mL, 0.590 mmol), then the mixture was stirred at 20 °C for 0.5 h. Then GDI (63 mg, 0.39 mmol) was added and the mixture was stirred at 20 °C for another 16 h. EtOAc (20 mL) was added and the mixture was washed with brine (20 mL), satd. NH₄Cl solution (20 mL) two times. The organic layer was dried over anhy. Na₂SO₄, filtered, and concentrated *in vacuo* to give the crude product, which was purified by Prep-HPLC (FA) to give 5-((5-(8-(ethylamino)-6-fluoro-4-(3-(trifluoromethyl)-1H-pyrazol-1-yl)-9H-pyrido[2,3-b]indol-3-yl)pyridin-3-yl)methyl)-1,3,4-oxadiazol-2(3H)-one (19.2 mg, 18.3% yield) as a yellow solid. MS (ESI): 539.1 [(M+H)⁺]. ¹H NMR (400 MHz, DMSO-*d6*) *δ*ppm: 12.22 (brs, 1H) 8.71 (s, 1H) 8.49 (d, *J*=1.6 Hz, 1H) 8.44 (brs, 1H) 8.36 (d, *J*=1.6 Hz, 1H) 8.25 (d, *J*=1.2 Hz, 1H) 7.48 (s, 1H) 7.02 (d, *J*=1.2 Hz, 1H) 6.48 (dd, *J*=12.0, 2.0 Hz, 1H) 5.96 (brs, 1H) 5.71 (dd, *J*=12.0, 2.0 Hz, 1H) 3.93 (s, 2H) 3.18 - 3.28 (m, 2 H) 1.32 (t, *J*=7.2 Hz, 3 H).

### Example 7.01

### 2-(5-(8-(Ethylamino)-6-fluoro-4-(3-(trifluoromethyl)-1H-pyrazol-1-yl)-9H-pyrido[2,3-b]indol-3-yl)pyridin-3-yl)ethanol

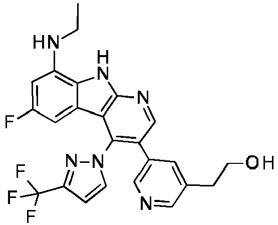

The titled compound was synthesized according to the following scheme:

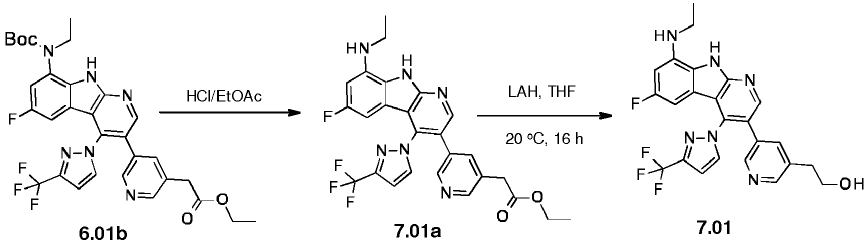

### Step (a) Preparation of ethyl 2-(5-(8-(ethylamino)-6-fluoro-4-(3-(trifluoromethyl)-1H-pyrazol-1-yl)-9H-pyrido[2,3-blindol-3-yl)pyridin-3-yl)acetate (compound 7.01a)

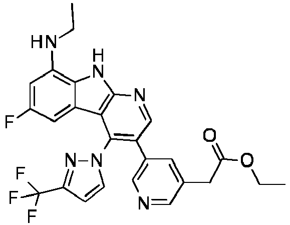

To a solution of ethyl 2-(5-(8-((*tert*-butoxycarbonyl)(ethyl)amino)-6-fluoro-4-(3-(trifluoromethyl)-1H-pyrazol-1-yl)-9H-pyrido[2,3-b]indol-3-yl)pyridin-3-yl)acetate (50 mg, 0.08 mmol) in EtOAc (2 mL) was added HCl solution in EtOAc (1.0 mL, 4N, 4 mmol), and the mixture was stirred at 20 °C for 2 h before concentrated *in vacuo* to give a crude product of ethyl 2-(5-(8-(ethylamino)-6-fluoro-4-(3-(trifluoromethyl)-1H-pyrazol-1-yl)-9H-pyrido[2,3-b]indol-3-yl)pyridin-3-yl)acetate (40 mg) as a yellow solid.

### Step (b) Preparation of 5-((5-(8-(ethylamino)-6-fluoro-4-(3-(trifluoromethyl)-1H-pyrazol-1-yl)-9H-pvrido[2,3-b]indol-3-yl)pyridin-3-yl)methyl)-1,3,4-oxadiazol-2(3H)-one (compound 7.01)

To a solution of ethyl 2-(5-(8-(ethylamino)-6-fluoro-4-(3-(trifluoromethyl)-1H-pyrazol-1-yl)-9H-pyrido[2,3-b]indol-3-yl)pyridin-3-yl)acetate (40 mg, 0.08 mmol) in THF (2 mL) was added LAH (2.88 mg, 0.08 mmol) at 20 °C, then the mixture was stirred at 20 °C for 16 h. Water (10 mL) was added to quench the reaction and the mixture was extracted with EtOAc (10 mL) three times. The combined organics were dried over anhy. Na₂SO₄, filtered, and concentrated *in vacuo* to give the crude material, which was purified by Prep-HPLC (FA) to give 5-((5-(8-(ethylamino)-6-fluoro-4-(3-(trifluoromethyl)-1H-pyrazol-1-yl)-9H-pyrido[2,3-b]indol-3-yl)pyridin-3-yl)methyl)-1,3,4-oxadiazol-2(3H)-one (8.1 mg, 21.2% yield) as a yellow solid. MS (ESI): 485.2 [(M+H)⁺]. ¹H NMR (400 MHz, DMSO) *δ*ppm: 12.17 (brs, 1H) 8.71 (s, 1H) 8.43 (brs, 1H) 8.38 (s, 1H) 8.29 (s, 1H) 8.21 (s, 1H) 7.35 (s, 1H) 7.07 (s, 1H) 6.48 (m, 1H) 5.96 (brs, 1H) 5.70 (m, 1H) 4.71 (brs, 1H) 3.53 (m, 1H) 3.26 (m, 1H) 2.62 - 2.72 (m, 2H) 1.32 (m, 3H).

### Example 8.01

### 3-(5-((1H-imidazol-2-yl)methyl)pyridin-3-yl)-N-ethyl-6-fluoro-4-(3-(trifluoromethyl)-1H-pyrazol-1-yl)-9H-pyrido[2,3-b]indol-8-amine

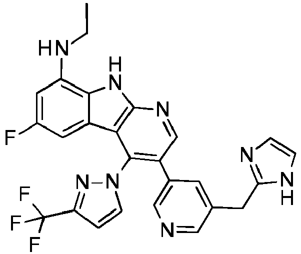

The titled compound was synthesized according to the following scheme:

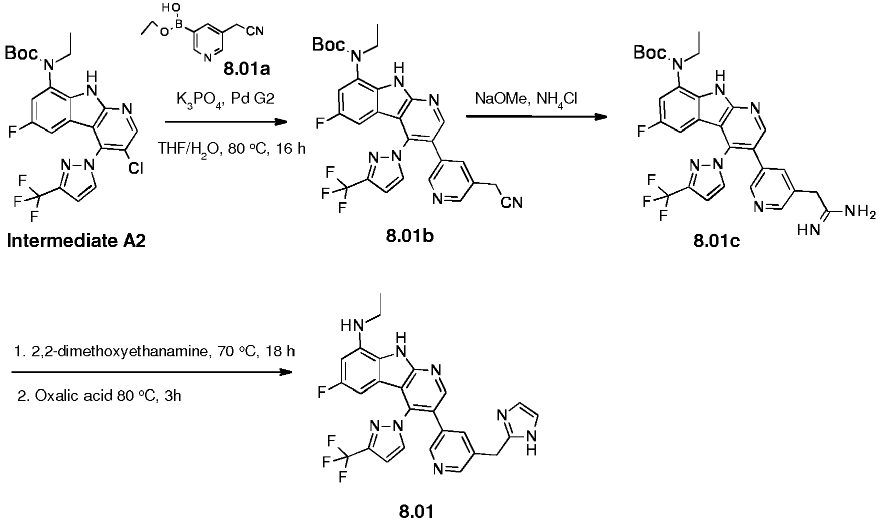

### Step (a) Preparation of tert-butyl (3-(5-(cyanomethyl)pyridin-3-yl)-6-fluoro-4-(3-(trifluoromethyl)-1H-pyrazol-1-yl)-9H-pyrido[2,3-b]indol-8-yl)(ethyl)carbamate (compound 8.01b)

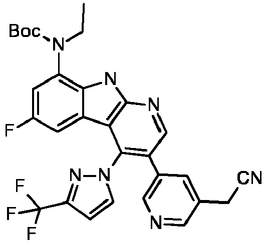

To a solution of *tert-*butyl (3-chloro-6-fluoro-4-(3-(trifluoromethyl)-1H-pyrazol-1-yl)-9H-pyrido[2,3-b]indol-8-yl)(ethyl)carbamate (250 mg, 0.5 mmol) and (5-(cyanomethyl)pyridin-3-yl)boronic acid (110 mg, 0.68 mmol) in THF/H₂O (10 mL/1 mL) was added cataCXium^{®} A Pd G2 (40 mg, 0.06 mmol, Sigma-Aldrich, Catalog #: 761311) and K₃PO₄ (280 mg, 2.63 mmol), then the solution was stirred at 80 °C for 18 h under Ar. After cooled back to r.t., the reaction mixture was concentrated *in vacuo* to give a crude product, which was purified by Prep-TLC (petroleum ether/EtOAc = ½) to give *tert-*butyl (3-(5-(cyanomethyl)pyridin-3-yl)-6-fluoro-4-(3-(trifluoromethyl)-1H-pyrazol-1-yl)-9H-pyrido[2,3-b]indol-8-yl)(ethyl)carbamate (260.0 mg, 89.3% yield) as a yellow solid. MS (ESI): 580.1 [(M+H)⁺].

### Step (b) Preparation of tert-butyl (3-(5-(2-amino-2-iminoethyl)pyridin-3-yl)-6-fluoro-4-(3-(trifluoromethyl)-1H-pyrazol-1-yl)-9H-pyrido[2,3-b]indol-8-yl)(ethyl)carbamate (compound 8.01c)

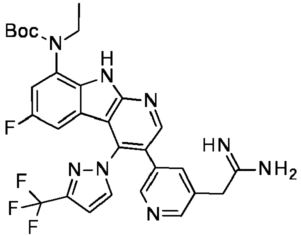

To a solution of *tert-*butyl (3-(5-(cyanomethyl)pyridin-3-yl)-6-fluoro-4-(3-(trifluoromethyl)-1H-pyrazol-1-yl)-9H-pyrido[2,3-b]indol-8-yl)(ethyl)carbamate (80 mg, 0.138 mmol) in MeOH (5 mL) was added NaOMe (10 mg, 0.185 mmol). After the reaction mixture was stirred at 20 °C for 120 h under N₂, NH₄Cl (30 mg, 0.56 mmol) was added and then the resulting solution was stirred at 20 °C for additional 20 h. Then the reaction mixture was concentrated *in vacuo* to give a crude product, which was re-dissolved in EtOAc (50 mL) and filtered. The filtrate was then concentrated *in vacuo* to give a crude product of *tert-*butyl (3-(5-(2-amino-2-iminoethyl)pyridin-3-yl)-6-fluoro-4-(3-(trifluoromethyl)-1H-pyrazol-1-yl)-9H-pyrido[2,3-b]indol-8-yl)(ethyl)carbamate (76.0 mg) as a white solid. It was used in the next step without further purification. MS (ESI): 597.2 [(M+H)⁺].

### Step (c) Preparation of 3-(5-((1H-imidazol-2-yl)methyl)pyridin-3-yl)-N-ethyl-6-fluoro-4-(3-(trifluoromethyl)-1H-pyrazol-1-yl)-9H-pyrido[2,3-b]indol-8-amine (compound 8.01)

To a solution of *tert-*butyl (3-(5-(2-amino-2-iminoethyl)pyridin-3-yl)-6-fluoro-4-(3-(trifluoromethyl)-1H-pyrazol-1-yl)-9H-pyrido[2,3-b]indol-8-yl)(ethyl)carbamate (76 mg, 0.051 mmol) in MeOH (5 mL) was added 2,2-dimethoxyethanamine (15 mg, 0.143 mmol). After been stirred at 70 °C for 18 h, the reaction mixture was concentrated *in vacuo* to give a crude solid, which was dissolved in aq. oxalic acid solution (1N, 5 mL) and the resulting mixture was stirred at 80 °C for another 3 h. The reaction mixture was cooled back to r.t., diluted with H₂O (30 mL), and basified with satd. aq. Na₂CO₃ solution to pH ~ 9. The aqueous solution was extracted with EtOAc (30 mL) three times, and the combined organics were dried over anhy. Na₂SO₄, filtered, and concentrated *in vacuo* to give a crude product. The crude product was then purified by Prep-HPLC to give 3-(5-((1H-imidazol-2-yl)methyl)pyridin-3-yl)-N-ethyl-6-fluoro-4-(3-(trifluoromethyl)-1H-pyrazol-1-yl)-9H-pyrido[2,3-b]indol-8-amine (13.2 mg, 49.8% yield) as a light-yellow solid. MS (ESI): 521.1 [(M+H)⁺]. ¹H NMR (400 MHz, DMSO-*d6*) *δ*ppm: 12.20 (brs, 1H), 8.65 (s, 1H), 8.41 (s, 1H), 8.30 (s, 1H), 8.26 (s, 0.23H), 8.19 (m, 2H), 7.50 (s, 1H), 7.02 (s, 1H), 6.89 (s, 2H), 6.48-6.45 (d, J= 11.6 Hz, 1H), 5.95 (s, 1H), 5.69-5.67 (m, 1H), 3.95 (s, 2H), 3.27-3.22 (q, J=7.2 Hz, 2H), 1.32-1.29 (t, J=7.2 Hz, 2H).

### BIOLOGICAL EXAMPLES

### Example 9

### 50% Growth Inhibitory Concentration (IC₅₀) Determination Assay:

The in vitro antimicrobial activity of the compounds against *E. coli* (BW25113, obtained from Coli Genetic Stock Center, #7636) and K. *pneumoniae* (ATCC10031) was determined according to the following procedure:
The assay used a 10-points Iso-Sensitest broth medium to measure quantitatively the in vitro activity of the compounds against *E. coli* BW25113 and K. *pneumoniae* ATCC 10031.

Stock compounds in DMSO were serially two-fold diluted (range from 50 to 0.097 µM final concentration) in 384 wells microtiter plates and inoculated with 49 µL the bacterial suspension in Iso-Sensitest broth medium to have a final cell concentration of ~ 5×10⁵ CFU/mL in a final volume/well of 50 µL/well. Microtiter plates were incubated at 35 ± 2 °C.

Bacterial cell growth was determined with the measurement of optical density at λ=600nm each 20 minutes over a time course of 16h.

Growth inhibition was calculated during the logarithmic growth of the bacterial cells with determination of the concentration inhibiting 50% (IC₅₀) of the growth.

GP-6 (Example 4.131 disclosed in WO 2012/125746 A1) was used as the reference compound in Table 5-10.

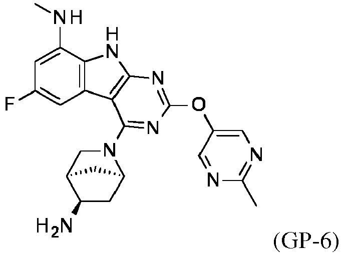

Compounds of the present invention were tested for their concentration inhibiting 50% (IC₅₀). The data of IC₅₀ over *E. coli* (BW25113) and K. *pneumoniae* (ATCC10031) are illustrated in Table 4. Particular compounds of the present invention were found to have IC₅₀ ≤ 1 µM.

**Table 4: IC₅₀ values of the compounds of this invention against E. coli and K. pneumoniae**

| **Example No.** | **IC50 (µM**) | |
|---|---|---|
| | *E. coli* K12 BW25113 | *K. pneumoniae* ATCC10031 |
| **GP-6** | 0.170 | 0.098* |
| **1.02** | 1.616 | 0.108 |
| **1.03** | 1.608 | 0.206 |
| **1.04** | 1.559 | 0.130 |
| **1.05** | 0.701 | 0.098* |
| **1.06** | 0.495 | 0.098* |
| **1.08** | 1.248 | 0.102 |
| **1.09** | 0.346 | 0.098* |
| **1.11** | 0.803 | 0.208 |
| **1.12** | 0.536 | 0.098* |
| **1.13** | 1.766 | 0.287 |
| **1.15** | 0.289 | 0.098* |
| **1.19** | 0.739 | 0.196 |
| **1.20** | 1.476 | 0.098* |
| **1.23** | 1.978 | 0.098* |
| **1.26** | 0.374 | 0.098* |
| **1.27** | 0.462 | 0.098* |
| **1.28** | 0.800 | 0.199 |
| **1.29** | 1.055 | 0.175 |
| **1.30** | 1.560 | 0.159 |
| **1.34** | 1.998 | 0.098* |
| **1.36** | 0.928 | 0.098* |
| **1.37** | 1.324 | 0.098* |
| **1.39** | 2.366 | 0.098* |
| **1.43** | 0.796 | 0.098* |
| **1.45** | 2.041 | 0.098* |
| **1.46** | 0.770 | 0.098* |
| **1.47** | 1.293 | 0.105 |
| **1.49** | 0.698 | 0.098 |
| **1.50** | 0.422 | 0.098 |
| **1.52** | 1.217 | 0.146 |
| **1.53** | 1.290 | 0.121 |
| **1.54** | 1.119 | 0.111 |
| **1.55** | 1.035 | 0.098* |
| **1.56** | 1.001 | 0.098* |
| **1.57** | 0.631 | 0.098* |
| **1.58** | 1.520 | 0.098* |
| **1.59** | 1.866 | 0.368 |
| **2.02** | 1.665 | 0.254 |
| **3.03** | 0.775 | 0.098* |
| **3.04** | 0.204 | 0.098* |
| **3.06** | 1.451 | 0.098* |
| **4.01** | 1.737 | 0.098* |
| **5.01** | 0.971 | 0.098* |
| **6.01** | 0.827 | 0.441 |

| | | |
|---|---|---|
| * Detection limit | | |

### Example 10

### Minimal Inhibitory Concentration Protocol (MIC) Assay:

The in vitro potency of compounds to inhibit *E. coli* (ATCC 25922) growth was assessed by MIC (Minimal Inhibitory Concentration) assay. Samples were prepared from 10 mM DMSO stock solutions. After serial 2-fold dilution in DMSO in a master plate (Greiner, Cat No: 651201), 180 µL sterile distilled water was added to 20 µL each aliquot of the samples. Then 10µL diluted compounds were transferred into a new assay plate (Costar, 3599).

The growth medium Caution-Adjusted Mueller Hinton Broth (CAMHB) was prepared by adding the 20 mg per liter CaCl₂ and 20 mg per liter MgCl₂ into the MHB medium (Jianglai Company, sterilized).

Vials of each of the test microorganisms were maintained frozen in the vapor phase of a liquid nitrogen freezer. Took out the bacteria *E.coli* ATCC 25922 (KWIKSTIK, 0335K) from liquid nitrogen freezer, thawed it in room temperature, and diluted the bacterial in the CAMHB medium to achieve a final inoculum of 5 × 105 CFU/mL, 90 µL CAMHB with bacteria was dispensed to the assay plate and pipetted 5 times.

Then the assay plates were incubated for 20 hours at 35°C in ambient air. Following incubation, the MIC (µg/mL), the lowest concentration of drug that inhibits visible growth of the microorganism was read and recorded throw the microscope.

**Table 5: MIC values of the compounds of this invention against E. coli**

| **Example No.** | **MIC** (**µg/mL**) |
|---|---|
| | *E. coli* ATCC 25922 |
| **GP-6** | 0.127 |
| **1.03** | 0.679 |
| **1.04** | 0.552 |
| **1.05** | 0.314 |
| **1.06** | 0.267 |
| **1.09** | 0.153 |
| **1.11** | 0.288 |
| **1.12** | 0.259 |
| **1.15** | 0.142 |
| **1.19** | 0.281 |
| **1.25** | 0.751 |
| **1.26** | 0.618 |
| **1.27** | 0.417 |
| **1.28** | 0.700 |
| **1.29** | 0.354 |
| **1.34** | 0.727 |
| **1.35** | 0.601 |
| **1.49** | 0.359 |
| **1.50** | 0.534 |
| **1.54** | 0.284 |
| **1.57** | 0.284 |
| **2.03** | 0.653 |
| **3.04** | 0.142 |
| **3.06** | 0.734 |
| **3.07** | 0.767 |
| **6.01** | 0.731 |

### Example 11

### Lyophilisation Solubility Assay (LYSA):

The solubility of compounds of present invention was assessed by LYSA assay. Samples were prepared in duplicate from 10 mM DMSO stock solutions (20µL) diluted in 30µL pure DMSO. After evaporation of DMSO with a centrifugal vacuum evaporator, the residue was dissolved in 0.05 M phosphate buffer (150µL, pH 6.5), stirred for one hour and shook for two hours. After one night, the solution was filtered using a microtiter filter plate. Then the filtrate and its 1/10 dilution were analyzed by HPLC-UV. In addition a four-point calibration curve was prepared from the 10 mM stock solutions and used for the solubility determination of the compounds. The results were in µg/mL. and summarized in Table 6. Particular compounds of the present invention were found to have LYSA > 10 µg/mL with much improved solubility compared to GP-6.

**Table 6: Solubility data of Examples**

| **Example No.** | **Lysa (µg/mL)** |
|---|---|
| **GP-6** | <1.0* |
| **1.10** | 7.6 |
| **1.24** | 8.3 |
| **1.26** | 32.5 |
| **1.39** | 42 |
| **1.45** | 16 |
| **1.46** | 23 |
| **1.49** | 14 |
| **1.53** | 21 |
| **2.01** | 67 |
| **2.02** | 148 |
| **2.04** | 133 |
| **2.05** | 24 |

| | |
|---|---|
| * Detection limit | |

### Example 12

### Cytotoxicity assay:

The cytotoxicity of compounds of present invention was assessed by HepG2 assay. HepG2 cells (ATCC HB-8065^{™}) were seeded into 96-well plates (1.2×104 cells per well) and treated with compounds for 3 days, cell viability was measured at day 3 post compound treatment using the CellTiter-Blue^{®} Cell Viability Assay (Promega, Cat. No. G8082). 20µL of Cell titer blue buffer were added to each well of the cells, incubated for 3 hours at 37°C, and the fluorescence value was measured by a plate reader (Molecular Device SpectraMax M2). The CC₅₀ value of each compound is plotted with GraphPad Prism using four parameter logistic equation. Results of cytotoxicity are given in Table 7.

**Table 7: CC₅₀ of Examples**

| **Example No.** | **CC₅₀ (µM)** |
|---|---|
| **GP-6** | 5.6 |
| **1.06** | >100* |
| **1.09** | 14.64 |
| **1.14** | 13.28 |
| **1.17** | >100* |
| **1.21** | 30.47 |
| **1.24** | 11.83 |
| **1.34** | 14.14 |
| **1.35** | 57.96 |
| **1.37** | 11.07 |
| **1.38** | 10.98 |
| **1.39** | 49.01 |
| **1.42** | >100* |
| **1.43** | 11.07 |
| **1.46** | 27.45 |
| **1.49** | 12.77 |
| **1.54** | 12.40 |
| **2.01** | 38.63 |
| **2.02** | 62.62 |
| **2.03** | 20.73 |
| **2.04** | 13.80 |
| **2.05** | 27.60 |
| **3.03** | 12.99 |
| **3.04** | 20.90 |
| **3.05** | >100* |
| **6.01** | 11.43 |

| | |
|---|---|
| * Detection limit | |

## Claims

1. A compound of formula (I), 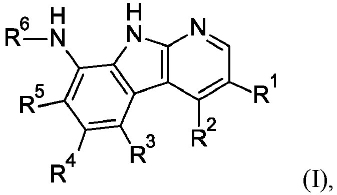 wherein
R¹ is naphthyridinyl;
pyrazolo[1,5-a]pyrimidinyl which is unsubstituted or substituted by cyano; pyridinyl which is substituted by 2-oxo-3H-1,3,4-oxadiazolyl; amino; aminoC₁₋₆alkyl; aminocarbonyl; aminocarbonylC₁₋₆alkyl; C₁₋₆alkoxyaminocarbonyl; C₁₋₆alkoxyC₁₋₆alkylaminocarbonyl; C₁₋₆alkoxyC₁₋₆alkylC₃₋₇cycloalkylaminocarbonyl; C₁₋₆alkyl; C₁₋₆alkyl-1,3,4-oxadiazolylC₁₋₆alkyl; C₁₋₆alkylaminocarbonyl; cyano; halogen; hydroxyC₁₋₆alkyl; imidazolylC₁₋₆alkyl; morpholinyl; or morpholinylC₁₋₆alkyl; or
pyrimidinyl which is unsubstituted or substituted by amino; aminoC₁₋₆alkoxy; aminoC₁₋₆alkyl; aminocarbonyl; azetidinyloxy; C₁₋₆alkoxy; C₁₋₆alkoxyaminocarbonylC₃₋₇cycloalkyl; C₁₋₆alkoxyC₁₋₆alkylamino; C₁₋₆alkyl; C₁₋₆alkylamino; C₁₋₆alkylaminocarbonylC₃₋₇cycloalkyl; carboxy(C₃₋₇cycloalkyl)C₁₋₆alkylamino; carboxyC₁₋₆alkoxy; carboxyC₁₋₆alkylamino; carboxyC₃₋₇cycloalkyl; hydroxyC₁₋₆alkyl; morpholinyl; piperazinyl; piperidinylamino; piperidinyloxy; pyrrolidinylamino; pyrrolidinyloxy; or tetrahydrofuranylamino;
R² is 4,5,6,7-tetrahydropyrazolo[3,4-c]pyridinyl substituted by cyano;
pyrazolyl which is substituted once or twice by substituents independently selected from haloC₁₋₆alkyl; aminoC₁₋₆alkyl; aminocarbonyl; C₁₋₆alkyl; cyano; and hydroxyC₁₋₆alkyl;
pyrrolo[3,4-c]pyrazolyl which is unsubstituted or substituted by cyano or haloC₁₋₆alkyl; or
thiazolyl;
R³ is H or halogen;
R⁴ is halogen;
R⁵ is H or halogen;
R⁶ is C₁₋₆alkyl;
or pharmaceutically acceptable salt thereof.

2. A compound according to claim 1, wherein
R¹ is naphthyridinyl;
pyrazolo[1,5-a]pyrimidinyl which is unsubstituted or substituted by cyano; pyridinyl which is substituted by 2-oxo-3H-1,3,4-oxadiazolyl; amino; aminocarbonyl; aminocarbonylmethyl; aminomethyl; cyano; fluoro; hydroxyethyl; hydroxymethyl; imidazolylmethyl; methoxyaminocarbonyl; methoxyethylaminocarbonyl; methoxymethylcyclopropylaminocarbonyl; methyl; methyl-1,3,4-oxadiazolylmethyl; methylaminocarbonyl; morpholinyl; or morpholinylmethyl; or
pyrimidinyl which is unsubstituted or substituted by amino; amino(dimethyl)ethoxy; aminocarbonyl; aminomethyl; azetidinyloxy; carboxy(cyclopropyl)methylamino; carboxy(dimethyl)ethoxy; carboxy(dimethyl)ethylamino; carboxycyclopropyl; ethoxy; ethyl; hydroxy(methyl)ethyl; hydroxyethyl; hydroxymethyl; methoxy; methoxyaminocarbonylcyclopropyl; methoxyethylamino; methyl; methylamino; methylaminocarbonylcyclopropyl; morpholinyl; piperazinyl; piperidinylamino; piperidinyloxy; pyrrolidinylamino; pyrrolidinyloxy; or tetrahydrofuranylamino;
R² is 4,5,6,7-tetrahydropyrazolo[3,4-c]pyridinyl substituted by cyano;
pyrazolyl which is substituted once or twice by substituents independently selected from aminocarbonyl; aminomethyl; cyano; difluoromethyl; ethyl; hydroxymethyl; and trifluoromethyl;
pyrrolo[3,4-c]pyrazolyl which is unsubstituted or substituted by cyano or trifluoromethyl; or
thiazolyl;
R³ is H or fluoro;
R⁴ is fluoro or chloro;
R⁵ is H or fluoro;
R⁶ is ethyl or methyl;
or pharmaceutically acceptable salt thereof.

3. A compound according to claim 1, or pharmaceutically acceptable salt thereof, wherein
R¹ is naphthyridinyl;
pyrazolo[1,5-a]pyrimidinyl;
pyridinyl which is substituted by 2-oxo-3H-1,3,4-oxadiazolyl; amino; aminoC₁₋₆alkyl; aminocarbonyl; C₁₋₆alkoxyaminocarbonyl; C₁₋₆alkoxyC₁₋₆alkylaminocarbonyl; C₁₋₆alkylaminocarbonyl; cyano; or hydroxyC₁₋₆alkyl; or
pyrimidinyl which is unsubstituted or substituted by amino; aminoC₁₋₆alkoxy; aminoC₁₋₆alkyl; aminocarbonyl; C₁₋₆alkoxy; C₁₋₆alkoxyC₁₋₆alkylamino; C₁₋₆alkyl; C₁₋₆alkylamino; hydroxyC₁₋₆alkyl; piperazinyl; piperidinylamino; piperidinyloxy; pyrrolidinylamino; pyrrolidinyloxy; or tetrahydrofuranyl amino.

4. A compound according to claim 3, or pharmaceutically acceptable salt thereof, wherein R² is pyrazolyl which is substituted once or twice by substituents independently selected from haloC₁₋₆alkyl, aminoC₁₋₆alkyl, aminocarbonyl, C₁₋₆alkyl, cyano, and hydroxyC₁₋₆alkyl.

5. A compound according to claim 3 or 4, or pharmaceutically acceptable salt thereof, wherein
R¹ is naphthyridinyl;
pyrazolo[1,5-a]pyrimidinyl;
pyridinyl which is substituted by 2-oxo-3H-1,3,4-oxadiazolyl; amino; aminoC₁₋₆alkyl; aminocarbonyl; C₁₋₆alkoxyaminocarbonyl; C₁₋₆alkoxyC₁₋₆alkylaminocarbonyl; C₁₋₆alkylaminocarbonyl; cyano; or hydroxyC₁₋₆alkyl; or
pyrimidinyl which is unsubstituted or substituted by amino; aminoC₁₋₆alkoxy; aminoC₁₋₆alkyl; aminocarbonyl; C₁₋₆alkoxy; C₁₋₆alkoxyC₁₋₆alkylamino; C₁₋₆alkyl; C₁₋₆alkylamino; hydroxyC₁₋₆alkyl; piperazinyl; piperidinylamino; piperidinyloxy; pyrrolidinylamino; pyrrolidinyloxy; or tetrahydrofuranylamino;
R² is pyrazolyl which is substituted once or twice by substituents independently selected from haloC₁₋₆alkyl, aminoC₁₋₆alkyl, aminocarbonyl, C₁₋₆alkyl, cyano, and hydroxyC₁₋₆alkyl;
R³ is H or halogen;
R⁴ is halogen;
R⁵ is H or halogen;
R⁶ is C₁₋₆alkyl;
or pharmaceutically acceptable salt thereof.

6. A compound according to claim 5, or pharmaceutically acceptable salt thereof, wherein
R¹ is naphthyridinyl; pyrazolo[1,5-a]pyrimidinyl; cyanopyridinyl; aminocarbonylpyridinyl; hydroxymethylpyridinyl; aminopyridinyl; methylaminocarbonylpyridinyl; aminomethylpyridinyl; methoxyaminocarbonylpyridinyl; 2-oxo-3H-1,3,4-oxadiazolylpyridinyl; hydroxyethylpyridinyl; methoxyethylaminocarbonylpyridinyl; amino(dimethyl)ethoxypyrimidinyl; aminocarbonylpyrimidinyl; aminomethylpyrimidinyl; aminopyrimidinyl; ethoxypyrimidinyl; hydroxy(methyl)ethylpyrimidinyl; hydroxyethylpyrimidinyl; hydroxymethylpyrimidinyl; methoxyethylaminopyrimidinyl; methoxypyrimidinyl; methylaminopyrimidinyl; methylpyrimidinyl; piperazinylpyrimidinyl; piperidinylaminopyrimidinyl; piperidinyloxypyrimidinyl; pyrrolidinylaminopyrimidinyl; pyrrolidinyloxypyrimidinyl; or tetrahydrofuranylaminopyrimidinyl;
R² is aminocarbonyl(trifluoromethyl)pyrazolyl; aminomethyl(trifluoromethyl)pyrazolyl; cyanopyrazolyl; difluoromethylpyrazolyl; ethylpyrazolyl; hydroxymethyl(trifluoromethyl)pyrazolyl; or trifluoromethylpyrazolyl;
R³ is H or fluoro;
R⁴ is fluoro or chloro;
R⁵ is H or fluoro;
R⁶ is methyl or ethyl;
or pharmaceutically acceptable salt thereof.

7. A compound according to any of the claims 1-6 selected from:
5-[8-(Ethylamino)-6-fluoro-4-[3-(trifluoromethyl)pyrazol-1-yl]-9H-pyrido[2,3-b]indol-3-yl]pyridine-3-carbonitrile;
6-Fluoro-3-(2-methoxypyrimidin-5-yl)-N-methyl-4-[3-(trifluoromethyl)pyrazol-1-yl]-9H-pyrido[2,3-b]indol-8-amine;
5-[8-(Ethylamino)-6-fluoro-4-[4-(trifluoromethyl)pyrazol-1-yl]-9H-pyrido[2,3-b]indol-3-yl]pyridine-3-carbonitrile;
N-Ethyl-6-fluoro-3-(2-methoxypyrimidin-5-yl)-4-[3-(trifluoromethyl)pyrazol-1-yl]-9H-pyrido[2,3 -b]indol-8-amine;
5-[4-(3-Cyanopyrazol-1-yl)-8-(ethylamino)-6-fluoro-9H-pyrido[2,3-b]indol-3-yl]pyridine-3 -carbonitrile;
3-(2-Aminopyrimidin-5-yl)-N-ethyl-6-fluoro-4-[3-(trifluoromethyl)pyrazol-1-yl]-9H-pyrido[2,3 -b]indol-8-amine;
N-ethyl-6-fluoro-3-(6-methyl-3-pyridyl)-4-[3-(trifluoromethyl)pyrazol-1-yl]-9H-pyrido[2,3 -b]indol-8-amine;
5-[6-Fluoro-8-(methylamino)-4-[4-(trifluoromethyl)pyrazol-1-yl]-9H-pyrido[2,3-b]indol-3-yl]pyridine-3-carbonitrile;
5-[4-(4-Cyanopyrazol-1-yl)-6-fluoro-8-(methylamino)-9H-pyrido[2,3-b]indol-3-yl]pyridine-3-carbonitrile;
N-Ethyl-6-fluoro-3-[2-(4-piperidylamino)pyrimidin-5-yl]-4-[3-(trifluoromethyl)pyrazol-1-yl]-9H-pyrido[2,3-b]indol-8-amine;
N-Ethyl-6-fluoro-3-(1,5-naphthyridin-3-yl)-4-[3-(trifluoromethyl)pyrazol-1-yl]-9H-pyrido[2,3 -b]indol-8-amine;
N-Ethyl-6-fluoro-3-pyrimidin-5-yl-4-[3-(trifluoromethyl)pyrazol-1-yl]-9H-pyrido[2,3-b]indol-8-amine;
N-Ethyl-6-fluoro-3-[2-(2-methoxyethylamino)pyrimidin-5-yl]-4-[3-(trifluoromethyl)pyrazol-1-yl]-9H-pyrido[2,3-b]indol-8-amine;
5-[4-[4-(Aminomethyl)-3-(trifluoromethyl)pyrazol-1-yl]-6-fluoro-8-(methylamino)-9H-pyrido[2,3-b]indol-3-yl]pyridine-3-carbonitrile;
5-[8-(Ethylamino)-6-fluoro-4-[3-(trifluoromethyl)pyrazol-1-yl]-9H-pyrido[2,3-b]indol-3 -yl]pyridine-3 -carboxamide;
N-Ethyl-6-fluoro-3-(2-morpholinopyrimidin-5-yl)-4-[3-(trifluoromethyl)pyrazol-1-yl]-9H-pyrido[2,3 -b]indol-8-amine;
N-Ethyl-6-fluoro-3-[2-(methylamino)pyrimidin-5-yl]-4-[3-(trifluoromethyl)pyrazol-1-yl]-9H-pyrido[2,3 -b]indol-8-amine;
N-Ethyl-6-fluoro-3-(5-fluoro-3-pyridyl)-4-[3-(trifluoromethyl)pyrazol-1-yl]-9H-pyrido[2,3 -b]indol-8-amine;
N-Ethyl-6-fluoro-3-pyrazolo[1,5-a]pyrimidin-6-yl-4-[3-(trifluoromethyl)pyrazol-1-yl]-9H-pyrido[2,3 -b]indol-8-amine;
N-Ethyl-6-fluoro-3-(2-pyrrolidin-3-yloxypyrimidin-5-yl)-4-[3-(trifluoromethyl)pyrazol-1-yl]-9H-pyrido[2,3-b]indol-8-amine;
4-(5,6-Dihydro-4H-pyrrolo[3,4-c]pyrazol-1-yl)-N-ethyl-6-fluoro-3-(2-methoxypyrimidin-5-yl)-9H-pyrido[2,3-b]indol-8-amine;
3-(2-Ethoxypyrimidin-5-yl)-N-ethyl-6-fluoro-4-[3-(trifluoromethyl)pyrazol-l-yl]-9H-pyrido[2,3 -b]indol-8-amine;
3-[2-(Azetidin-3-yloxy)pyrimidin-5-yl]-N-ethyl-6-fluoro-4-[3-(trifluoromethyl)pyrazol-1-yl]-9H-pyrido[2,3-b]indol-8-amine;
N-Ethyl-6-fluoro-3-[2-(pyrrolidin-3-ylamino)pyrimidin-5-yl]-4-[3-(trifluoromethyl)pyrazol-1-yl]-9H-pyrido[2,3-b]indol-8-amine;
N-Ethyl-6-fluoro-3-[2-(tetrahydrofuran-3-ylamino)pyrimidin-5-yl]-4-[3-(trifluoromethyl)pyrazol-1-yl]-9H-pyrido[2,3-b]indol-8-amine;
5-[4-[4-(Aminomethyl)-3-(trifluoromethyl)pyrazol-1-yl]-8-(ethylamino)-6-fluoro-9H-pyrido[2,3-b]indol-3-yl]pyridine-3-carbonitrile;
4-[4-(Aminomethyl)-3-(trifluoromethyl)pyrazol-1-yl]-N-ethyl-6-fluoro-3-(2-methoxypyrimidin-5-yl)-9H-pyrido[2,3-b]indol-8-amine;
5-[8-(Ethylamino)-5,6-difluoro-4-[3-(trifluoromethyl)pyrazol-1-yl]-9H-pyrido[2,3-b]indol-3-yl]pyridine-3-carbonitrile;
N-Ethyl-5,6-difluoro-3-(2-methoxypyrimidin-5-yl)-4-[3-(trifluoromethyl)pyrazol-1-yl]-9H-pyrido[2,3 -b]indol-8-amine;
3-[2-(2-Amino-2-methyl-propoxy)pyrimidin-5-yl]-N-ethyl-6-fluoro-4-[3-(trifluoromethyl)pyrazol-1-yl]-9H-pyrido[2,3-b]indol-8-amine;
2-[5-[8-(Ethylamino)-6-fluoro-4-[3-(trifluoromethyl)pyrazol-1-yl]-9H-pyrido[2,3-b]indol-3-yl]pyrimidin-2-yl]propan-2-ol;
5-[8-(Ethylamino)-4-(3-ethylpyrazol-1-yl)-6-fluoro-9H-pyrido[2,3-b]indol-3-yl]pyridine-3-carbonitrile;
6-Chloro-N-ethyl-3-(2-methoxypyrimidin-5-yl)-4-[3-(trifluoromethyl)pyrazol-l-yl]-9H-pyrido[2,3 -b]indol-8-amine;
5-[8-(Ethylamino)-6-fluoro-4-[3-(trifluoromethyl)-5,6-dihydro-4H-pyrrolo[3,4-c]pyrazol-1-yl]-9H-pyrido[2,3-b]indol-3-yl]pyridine-3-carbonitrile;
N-Ethyl-6-fluoro-3-(2-methoxypyrimidin-5-yl)-4-[3-(trifluoromethyl)-5,6-dihydro-4H-pyrrolo[3,4-c]pyrazol-1-yl]-9H-pyrido[2,3-b]indol-8-amine;
[5-[8-(Ethylamino)-6-fluoro-4-[3-(trifluoromethyl)pyrazol-1-yl]-9H-pyrido[2,3-b]indol-3-yl]-3-pyridyl]methanol;
3-(5-Amino-3-pyridyl)-N-ethyl-6-fluoro-4-[3-(trifluoromethyl)pyrazol-1-yl]-9H-pyrido[2,3 -b]indol-8-amine;
1-[6-Fluoro-3-(2-methoxypyrimidin-5-yl)-8-(methylamino)-9H-pyrido[2,3-b]indol-4-yl]-5,6-dihydro-4H-pyrrolo[3,4-c]pyrazole-3-carbonitrile;
1-[8-(Ethylamino)-6-fluoro-3-(2-methoxypyrimidin-5-yl)-9H-pyrido[2,3-b]indol-4-yl]-4,5,6,7-tetrahydropyrazolo[3,4-c]pyridine-3-carbonitrile;
3-(6-Amino-3-pyridyl)-N-ethyl-6-fluoro-4-[3-(trifluoromethyl)pyrazol-1-yl]-9H-pyrido[2,3 -b]indol-8-amine;
5-[4-[3-(Difluoromethyl)pyrazol-l-yl]-8-(ethylamino)-6-fluoro-9H-pyrido[2,3-b]indol-3-yl]pyridine-3-carbonitrile;
N-Ethyl-6-fluoro-3-[6-(morpholinomethyl)-3-pyridyl]-4-[3-(trifluoromethyl)pyrazol-1-yl]-9H-pyrido[2,3 -b]indol-8-amine;
5-[8-(Ethylamino)-6-fluoro-4-[3-(trifluoromethyl)pyrazol-1-yl]-9H-pyrido[2,3-b]indol-3-yl]-N-methyl-pyridine-3-carboxamide;
N-Ethyl-6-fluoro-3-(5-morpholino-3-pyridyl)-4-[3-(trifluoromethyl)pyrazol-1-yl]-9H-pyrido[2,3 -b]indol-8-amine;
3-[5-(Aminomethyl)-3-pyridyl]-N-ethyl-6-fluoro-4-[3-(trifluoromethyl)pyrazol-1-yl]-9H-pyrido[2,3 -b]indol-8-amine;
3-[2-(Aminomethyl)pyrimidin-5-yl]-N-ethyl-6-fluoro-4-[3-(trifluoromethyl)pyrazol-1-yl]-9H-pyrido[2,3 -b]indol-8-amine;
3-(2-Ethylpyrimidin-5-yl)-6-fluoro-N-methyl-4-[3-(trifluoromethyl)pyrazol-1-yl]-9H-pyrido[2,3-b]indol-8-amine;
[5-[8-(Ethylamino)-6-fluoro-4-[3-(trifluoromethyl)pyrazol-1-yl]-9H-pyrido[2,3-b]indol-3-yl]pyrimidin-2-yl]methanol;
5-[4-[4-(Aminomethyl)-3-(trifluoromethyl)pyrazol-1-yl]-6,7-difluoro-8-(methylamino)-9H-pyrido[2,3-b]indol-3-yl]pyridine-3-carbonitrile;
N-ethyl-6-fluoro-3-(2-methylpyrimidin-5-yl)-4-[3-trifluoromethyl)pyrazol-1-yl]-9H-pyrido[2,3 -b]indol-8-amine;
5-[8-(ethylamino)-6-fluoro-4-thiazol-5-yl-9H-pyrido[2,3-b]indol-3-yl]pyridine-3-carbonitrile;
N-ethyl-6-fluoro-3-(2-piperazin-1-ylpyrimidin-5-yl)-4-[3-(trifluoromethyl)pyrazol-1-yl]-9H-pyrido[2,3 -b]indol-8-amine;
N-ethyl-6-fluoro-3-[2-(4-piperidyloxy)pyrimidin-5-yl]-4-[3-(trifluoromethyl)pyrazol-1-yl]-9H-pyrido[2,3 -b]indol-8-amine;
1-[5-[8-(ethylamino)-6-fluoro-4-[3-(trifluoromethyl)pyrazol-1-yl]-9H-pyrido[2,3-b]indol-3 -yl]pyrimidin-2-yl] ethanol;
N-ethyl-6-fluoro-3-[2-[(3*R*)-pyrrolidin-3-yl]oxypyrimidin-5-yl]-4-[3-(trifluoromethyl)pyrazol-1-yl]-9H-pyrido[2,3-b]indol-8-amine;
N-ethyl-6-fluoro-3-[2-[(3*S*)-pyrrolidin-3-yl]oxypyrimidin-5-yl]-4-[3-(trifluoromethyl)pyrazol-1-yl]-9H-pyrido[2,3-b]indol-8-amine;
(1*S*)-1-[5-[8-(ethylamino)-6-fluoro-4-[3-(trifluoromethyl)pyrazol-1-yl]-9H-pyrido[2,3-b]indol-3 -yl]pyrimidin-2-yl] ethanol;
N-ethyl-6-fluoro-3-[5-[(5-methyl-1,3,4-oxadiazol-2-yl)methyl]-3-pyridyl]-4-[3-(trifluoromethyl)pyrazol-1-yl]-9H-pyrido[2,3-b]indol-8-amine;
6-[8-(ethylamino)-6-fluoro-4-[3-(trifluoromethyl)pyrazol-l-yl]-9H-pyrido[2,3-b]indol-3-yl]pyrazolo[1,5-a]pyrimidine-3-carbonitrile;
4-[4-(Aminomethyl)-3-(trifluoromethyl)pyrazol-1-yl]-N-ethyl-6-fluoro-3-pyrazolo[1,5-a]pyrimidin-6-yl-9H-pyrido[2,3-b]indol-8-amine;
1-[5-[8-(ethylamino)-6-fluoro-4-[3-(trifluoromethyl)pyrazol-1-yl]-9H-pyrido[2,3-b]indol-3-yl]pyrimidin-2-yl]cyclopropanecarboxylic acid;
1-(5-(8-(ethylamino)-6-fluoro-4-(3-(trifluoromethyl)-1H-pyrazol-1-yl)-9H-pyrido[2,3-b]indol-3-yl)pyrimidin-2-yl)-N-methylcyclopropanecarboxamide;
1-(5-(8-(ethylamino)-6-fluoro-4-(3-(trifluoromethyl)-1H-pyrazol-1-yl)-9H-pyrido[2,3-b]indol-3-yl)pyrimidin-2-yl)-N-methoxycyclopropanecarboxamide;
5-[8-(ethylamino)-6-fluoro-4-[3-(trifluoromethyl)pyrazol-1-yl]-9H-pyrido[2,3-b]indol-3-yl]-N-methoxy-pyridine-3-carboxamide;
5-[8-(ethylamino)-6-fluoro-4-[3-(trifluoromethyl)pyrazol-1-yl]-9H-pyrido[2,3-b]indol-3-yl]pyrimidine-2-carboxamide;
2-[5-[8-(ethylamino)-6-fluoro-4-[3-(trifluoromethyl)pyrazol-1-yl]-9H-pyrido[2,3-b]indol-3-yl]-3-pyridyl]acetamide;
5-[8-(ethylamino)-6-fluoro-4-[3-(trifluoromethyl)pyrazol-1-yl]-9H-pyrido[2,3-b]indol-3-yl]-N-(2-methoxyethyl)pyridine-3-carboxamide;
5-[8-(ethylamino)-6-fluoro-4-[3-(trifluoromethyl)pyrazol-1-yl]-9H-pyrido[2,3-b]indol-3-yl]-N-[1-(methoxymethyl)cyclopropyl]pyridine-3-carboxamide;
1-(3-(5-cyanopyridin-3-yl)-8-(ethylamino)-6-fluoro-9H-pyrido[2,3-b]indol-4-yl)-3-(trifluoromethyl)-1H-pyrazole-4-carboxamide;
5-[8-(ethylamino)-6-fluoro-4-[4-(hydroxymethyl)-3-(trifluoromethyl)pyrazol-1-yl]-9H-pyrido[2,3-b]indol-3-yl]pyridine-3-carbonitrile;
5-[[5-[8-(ethylamino)-6-fluoro-4-[3-(trifluoromethyl)pyrazol-1-yl]-9H-pyrido[2,3-b]indol-3-yl]-3-pyridyl]methyl]-3H-1,3,4-oxadiazol-2-one;
2-(5-(8-(ethylamino)-6-fluoro-4-(3-(trifluoromethyl)-1H-pyrazol-1-yl)-9H-pyrido[2,3-b]indol-3-yl)pyridin-3-yl)ethanol; and
3-(5-((1H-imidazol-2-yl)methyl)pyridin-3-yl)-N-ethyl-6-fluoro-4-(3-(trifluoromethyl)-1H-pyrazol-1-yl)-9H-pyrido[2,3-b]indol-8-amine;
or pharmaceutically acceptable salt thereof.

8. A process for the preparation of a compound according to any one of claims 1 to 7 comprising the reaction of compound of formula (Ii), 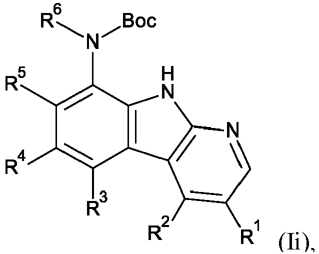 with an acid, wherein R¹ to R⁶ are defined as in any one of claims 1 to 6.

9. A compound according to any one of claims 1 to 7 for use as therapeutically active substance.

10. A pharmaceutical composition comprising a compound in accordance with any one of claims 1 to 7 and a therapeutically inert carrier.

11. A compound according to any one of claims 1 to 7 for use in the treatment or prophylaxis of bacterial infection.

## Patentansprüche

1. Verbindung der Formel (I) 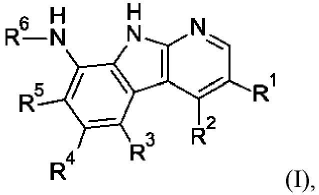 wobei
R¹ Naphthyridinyl;
Pyrazolo[1,5-a]pyrimidinyl, das unsubstituiert oder mit Cyano substituiert ist;
Pyridinyl, das mit 2-Oxo-3H-1,3,4-oxadiazolyl; Amino; Amino-C₁₋₆-Alkyl; Aminocarbonyl; Aminocarbonyl-C₁₋₆-alkyl; C₁₋₆-Alkoxyaminocarbonyl; C₁₋₆-Alkoxy-C₁₋₆-alkylaminocarbonyl; C₁₋₆-Alkoxy-C₁₋₆-alkyl-C₃₋₇-cycloalkylaminocarbonyl; C₁₋₆-Alkyl; C₁₋₆-Alkyl-1,3,4-oxadiazolyl-C₁₋₆-alkyl; C₁₋₆-Alkylaminocarbonyl, Cyano; Halogen; Hydroxy-C₁₋₆-alkyl, Imidazolyl-C₁₋₆-alkyl; Morpholinyl oder Morpholinyl-C₁₋₆-alkyl substituiert ist; oder
Pyrimidinyl, das unsubstituiert oder mit Amino; Amino-C₁₋₆-alkoxy, Amino-C₁₋₆-alkyl; Aminocarbonyl; Azetidinyloxy; C₁₋₆-Alkoxy; C₁₋₆-Alkoxyaminocarbonyl-C₃₋₇-cycloalkyl; C₁₋₆-Alkoxy-C₁₋₆-alkylamino; C₁₋₆-Alkyl, C₁₋₆-Alkylamino; C₁₋₆-Alkylaminocarbonyl-C₃₋₇-cycloalkyl; Carboxy-(C₃₋₇-cycloalkyl)-C₁₋₆-alkylamino; Carboxy-C₁₋₆-alkoxy, Carboxy-C₁₋₆-alkylamino; Carboxy-C₃₋₇-cycloalkyl; Hydroxy-C₁₋₆-alkyl, Morpholinyl; Piperazinyl; Piperidinylamino; Piperidinyloxy; Pyrrolidinylamino; Pyrrolidinyloxy oder Tetrahydrofuranylamino substituiert ist, ist;
R² mit Cyano substituiertes 4,5,6,7-Tetrahydropyrazolo[3,4-c]pyridinyl;
Pyrazolyl, das einmal oder zweimal mit Substituenten substituiert ist, die unabhängig voneinander aus Halogen-C₁₋₆-alkyl, Amino-C₁₋₆-alkyl; Aminocarbonyl; C₁₋₆-Alkyl; Cyano und Hydroxy-C₁₋₆-Alkyl ausgewählt sind;
Pyrrolo[3,4-c]pyrazolyl, das unsubstituiert oder mit Cyano oder Halogen-C₁₋₆-alkyl substituiert ist; oder
Thiazolyl ist;
R³ H oder Halogen ist;
R⁴ Halogen ist;
R⁵ H oder Halogen ist;
R⁶ C₁₋₆-Alkyl ist;
oder pharmazeutisch unbedenkliches Salz davon.

2. Verbindung nach Anspruch 1, wobei
R¹ Naphthyridinyl;
Pyrazolo[1,5-a]pyrimidinyl, das unsubstituiert oder mit Cyano substituiert ist;
Pyridinyl, das mit 2-Oxo-3H-1,3,4-oxadiazolyl; Amino; Aminocarbonyl; Aminocarbonylmethyl; Aminomethyl; Cyano; Fluor; Hydroxyethyl; Hydroxymethyl; Imidazolylmethyl; Methoxyaminocarbonyl; Methoxyethylaminocarbonyl; Methoxymethylcyclopropylaminocarbonyl, Methyl; Methyl-1,3,4-oxadiazolylmethyl; Methylaminocarbonyl; Morpholinyl oder Morpholinylmethyl substituiert ist; oder
Pyrimidinyl, das unsubstituiert oder mit Amino; Amino(dimethyl)ethoxy; Aminocarbonyl; Aminomethyl; Azetidinyloxy; Carboxy(cyclopropyl)methylamino; Carboxy(dimethyl)ethoxy; Carboxy(dimethyl)ethylamino; Carboxycyclopropyl; Ethoxy; Ethyl; Hydroxy(methyl)ethyl; Hydroxyethyl; Hydroxymethyl; Methoxy; Methoxyaminocarbonylcyclopropyl; Methoxyethylamino; Methyl; Methylamino; Methylaminocarbonylcyclopropyl; Morpholinyl; Piperazinyl; Piperidinylamino; Piperidinyloxy; Pyrrolidinylamino; Pyrrolidinyloxy oder Tetrahydrofuranylamino substituiert ist, ist;
R² mit Cyano substituiertes 4,5,6,7-Tetrahydropyrazolo[3,4-c]pyridinyl;
Pyrazolyl, das einmal oder zweimal mit Substituenten substituiert ist, die unabhängig voneinander aus Aminocarbonyl; Aminomethyl; Cyano; Difluormethyl; Ethyl; Hydroxymethyl und Trifluormethyl ausgewählt sind;
Pyrrolo[3,4-c]pyrazolyl, das unsubstituiert oder mit Cyano oder Trifluormethyl substituiert ist; oder
Thiazolyl ist;
R³ H oder Fluor ist;
R⁴ Fluor oder Chlor ist;
R⁵ H oder Fluor ist;
R⁶ Ethyl oder Methyl ist;
oder pharmazeutisch unbedenkliches Salz davon.

3. Verbindung nach Anspruch 1 oder pharmazeutisch unbedenkliches Salz davon, wobei
R¹ Naphthyridinyl;
Pyrazolo[1,5-a]pyrimidinyl;
Pyridinyl, das mit 2-Oxo-3H-1,3,4-oxadiazolyl; Amino; Amino-C₁₋₆-alkyl; Aminocarbonyl; C₁₋₆-Alkoxyaminocarbonyl; C₁₋₆-Alkoxy-C₁₋₆-alkylaminocarbonyl; C₁₋₆-Alkylaminocarbonyl; Cyano oder Hydroxy-C₁₋₆-alkyl substituiert ist; oder
Pyrimidinyl, das unsubstituiert oder mit Amino; Amino-C₁₋₆-alkoxy, Amino-C₁₋₆-alkyl; Aminocarbonyl; C₁₋₆-Alkoxy, C₁₋₆-Alkoxy-C₁₋₆-alkylamino; C₁₋₆-Alkyl; C₁₋₆-Alkylamino; Hydroxy-C₁₋₆-alkyl; Piperazinyl; Piperidinylamino; Piperidinyloxy; Pyrrolidinylamino; Pyrrolidinyloxy oder Tetrahydrofuranylamino substituiert ist, ist.

4. Verbindung nach Anspruch 3 oder pharmazeutisch unbedenkliches Salz davon, wobei R² Pyrazolyl ist, das einmal oder zweimal mit Substituenten substituiert ist, die unabhängig voneinander aus Halogen-C₁₋₆-alkyl, Amino-C₁₋₆-alkyl, Aminocarbonyl, C₁₋₆-Alkyl, Cyano und Hydroxy-C₁₋₆-alkyl ausgewählt sind.

5. Verbindung nach Anspruch 3 oder 4 oder pharmazeutisch unbedenkliches Salz davon, wobei
R¹ Naphthyridinyl;
Pyrazolo[1,5-a]pyrimidinyl;
Pyridinyl, das mit 2-Oxo-3H-1,3,4-oxadiazolyl; Amino; Amino-C₁₋₆-alkyl; Aminocarbonyl; C₁₋₆-Alkoxyaminocarbonyl; C₁₋₆-Alkoxy-C₁₋₆-alkylaminocarbonyl; C₁₋₆-Alkylaminocarbonyl; Cyano oder Hydroxy-C₁₋₆-alkyl substituiert ist; oder
Pyrimidinyl, das unsubstituiert oder mit Amino; Amino-C₁₋₆-alkoxy, Amino-C₁₋₆-alkyl; Aminocarbonyl; C₁₋₆-Alkoxy, C₁₋₆-Alkoxy-C₁₋₆-alkylamino; C₁₋₆-Alkyl; C₁₋₆-Alkylamino; Hydroxy-C₁₋₆-alkyl; Piperazinyl; Piperidinylamino; Piperidinyloxy; Pyrrolidinylamino; Pyrrolidinyloxy oder Tetrahydrofuranylamino substituiert ist, ist;
R² Pyrazolyl ist, das einmal oder zweimal mit Substituenten substituiert ist, die unabhängig voneinander aus Halogen-C₁₋₆-alkyl, Amino-C₁₋₆-alkyl, Aminocarbonyl, C₁₋₆-Alkyl, Cyano und Hydroxy-C₁₋₆-alkyl ausgewählt sind;
R³ H oder Halogen ist;
R⁴ Halogen ist;
R⁵ H oder Halogen ist;
R⁶ C₁₋₆-Alkyl ist;
oder pharmazeutisch unbedenkliches Salz davon.

6. Verbindung nach Anspruch 5 oder pharmazeutisch unbedenkliches Salz davon, wobei
R¹ Naphthyridinyl; Pyrazolo[1,5-a]pyrimidinyl; Cyanopyridinyl; Aminocarbonylpyridinyl; Hydroxymethylpyridinyl; Aminopyridinyl; Methylaminocarbonylpyridinyl; Aminomethylpyridinyl; Methoxyaminocarbonylpyridinyl; 2-Oxo-3H-1,3,4-oxadiazolylpyridinyl, Hydroxyethylpyridinyl; Methoxyethylaminocarbonylpyridinyl; Amino(dimethyl)ethoxypyrimidinyl; Aminocarbonylpyrimidinyl; Aminomethylpyrimidinyl; Aminopyrimidinyl; Ethoxypyrimidinyl; Hydroxy(methyl)ethylpyrimidinyl; Hydroxyethylpyrimidinyl; Hydroxymethylpyrimidinyl; Methoxyethylaminopyrimidinyl; Methoxypyrimidinyl; Methylaminopyrimidinyl; Methylpyrimidinyl; Piperazinylpyrimidinyl; Piperidinylaminopyrimidinyl; Piperidinyloxypyrimidinyl; Pyrrolidinylaminopyrimidinyl; Pyrrolidinyloxypyrimidinyl oder Tetrahydrofuranylaminopyrimidinyl ist;
R² Aminocarbonyl(trifluormethyl)pyrazolyl; Aminomethyl(trifluormethyl)pyrazolyl; Cyanopyrazolyl; Difluormethylpyrazolyl; Ethylpyrazolyl; Hydroxymethyl(trifluormethyl)pyrazolyl oder Trifluormethylpyrazolyl ist;
R³ H oder Fluor ist;
R⁴ Fluor oder Chlor ist;
R⁵ H oder Fluor ist;
R⁶ Methyl oder Ethyl ist;
oder pharmazeutisch unbedenkliches Salz davon.

7. Verbindung nach einem der Ansprüche 1-6, ausgewählt aus:
5-[8-(Ethylamino)-6-fluor-4-[3-(trifluormethyl)pyrazol-1-yl]-9H-pyrido[2,3-b]indol-3-yl]pyridin-3-carbonitril;
6-Fluor-3-(2-methoxypyrimidin-5-yl)-N-methyl-4-[3-(trifluormethyl)pyrazol-1-yl]-9H-pyrido[2,3-b]indol-8-amin;
5-[8-(Ethylamino)-6-fluor-4-[4-(trifluormethyl)pyrazol-1-yl]-9H-pyrido[2,3-b]indol-3-yl]pyridin-3-carbonitril;
N-Ethyl-6-fluor-3-(2-methoxypyrimidin-5-yl)-4-[3-(trifluormethyl)pyrazol-1-yl]-9H-pyrido[2,3-b]indol-8-amin;
5-[4-(3-Cyanopyrazol-1-yl)-8-(ethylamino)-6-fluor-9H-pyrido[2,3-b]indol-3-yl]pyridin-3-carbonitril;
3-(2-Aminopyrimidin-5-yl)-N-ethyl-6-fluor-4-[3-(trifluormethyl)pyrazol-1-yl]-9H-pyrido[2,3-b]indol-8-amin;
N-Ethyl-6-fluor-3-(6-methyl-3-pyridyl)-4-[3-(trifluormethyl)pyrazol-1-yl]-9H-pyrido[2,3-b]indol-8-amin;
5-[6-Fluor-8-(methylamino)-4-[4-(trifluormethyl)pyrazol-1-yl]-9H-pyrido[2,3-b]indol-3-yl]pyridin-3-carbonitril;
5-[4-(4-Cyanopyrazol-1-yl)-6-fluor-8-(methylamino)-9H-pyrido[2,3-b]indol-3-yl]pyridin-3-carbonitril;
N-Ethyl-6-fluor-3-[2-(4-piperidylamino)pyrimidin-5-yl]-4-[3-(trifluormethyl)pyrazol-1-yl]-9H-pyrido[2,3-b]indol-8-amin;
N-Ethyl-6-fluor-3-(1,5-naphthyridin-3-yl)-4-[3-(trifluormethyl)pyrazol-1-yl]-9H-pyrido[2,3-b]indol-8-amin;
N-Ethyl-6-fluor-3-pyrimidin-5-yl-4-[3-(trifluormethyl)pyrazol-1-yl]-9H-pyrido[2,3-b]indol-8-amin;
N-Ethyl-6-fluor-3-[2-(2-methoxyethylamino)pyrimidin-5-yl]-4-[3-(trifluormethyl)pyrazol-1-yl]-9H-pyrido[2,3-b]indol-8-amin,
5-[4-[4-(Aminomethyl)-3-(trifluormethyl)pyrazol-1-yl]-6-fluor-8-(methylamino)-9H-pyrido[2,3-b]indol-3-yl]pyridin-3-carbonitril;
5-[8-(Ethylamino)-6-fluor-4-[3-(trifluormethyl)pyrazol-1-yl]-9H-pyrido[2,3-b]indol-3-yl]pyridin-3-carboxamid;
N-Ethyl-6-fluor-3-(2-morpholinopyrimidin-5-yl)-4-[3-(trifluormethyl)pyrazol-1-yl]-9H-pyrido[2,3-b]indol-8-amin;
N-Ethyl-6-fluor-3-[2-(methylamino)pyrimidin-5-yl]-4-[3-(trifluormethyl)pyrazol-1-yl]-9H-pyrido[2,3-b]indol-8-amin;
N-Ethyl-6-fluor-3-(5-fluor-3-pyridyl)-4-[3-(trifluormethyl)pyrazol-1-yl]-9H-pyrido[2,3-b]indol-8-amin;
N-Ethyl-6-fluor-3-pyrazolo[1,5-a]pyrimidin-6-yl-4-[3-(trifluormethyl)pyrazol-1-yl]-9H-pyrido[2,3-b]indol-8-amin;
N-Ethyl-6-fluor-3-(2-pyrrolidin-3-yloxypyrimidin-5-yl)-4-[3-(trifluormethyl)pyrazol-1-yl]-9H-pyrido[2,3-b]indol-8-amin;
4-(5,6-Dihydro-4H-pyrrolo[3,4-c]pyrazol-1-yl)-N-ethyl-6-fluor-3-(2-methoxypyrimidin-5-yl)-9H-pyrido[2,3-b]indol-8-amin;
3-(2-Ethoxypyrimidin-5-yl)-N-ethyl-6-fluor-4-[3-(trifluormethyl)pyrazol-1-yl]-9H-pyrido[2,3-b]indol-8-amin;
3-[2-(Azetidin-3-yloxy)pyrimidin-5-yl]-N-ethyl-6-fluor-4-[3-(trifluormethyl)pyrazol-1-yl]-9H-pyrido[2,3-b]indol-8-amin;
N-Ethyl-6-fluor-3-[2-(pyrrolidin-3-ylamino)pyrimidin-5-yl]-4-[3-(trifluormethyl)pyrazol-1-yl]-9H-pyrido[2,3-b]indol-8-amin;
N-Ethyl-6-fluor-3-[2-(tetrahydrofuran-3-ylamino)pyrimidin-5-yl]-4-[3-(trifluormethyl)pyrazol-1-yl]-9H-pyrido[2,3 -b]indol-8-amin;
5-[4-[4-(Aminomethyl)-3-(trifluormethyl)pyrazol-1-yl]-8-(ethylamino)-6-fluor-9H-pyrido[2,3-b]indol-3-yl]pyridin-3-carbonitril;
4-[4-(Aminomethyl)-3-(trifluormethyl)pyrazol-1-yl]-N-ethyl-6-fluor-3-(2-methoxypyrimidin-5-yl)-9H-pyrido[2,3-b]indol-8-amin;
5-[8-(Ethylamino)-5,6-difluor-4-[3-(trifluormethyl)pyrazol-1-yl]-9H-pyrido[2,3-b]indol-3-yl]pyridin-3-carbonitril;
N-Ethyl-5,6-difluor-3-(2-methoxypyrimidin-5-yl)-4-[3-(trifluormethyl)pyrazol-1-yl]-9H-pyrido[2,3-b]indol-8-amin;
3-[2-(2-Amino-2-methylpropoxy)pyrimidin-5-yl]-N-ethyl-6-fluor-4-[3-(trifluormethyl)pyrazol-1-yl]-9H-pyrido[2,3-b]indol-8-amin;
2-[5-[8-(Ethylamino)-6-fluor-4-[3-(trifluormethyl)pyrazol-1-yl]-9H-pyrido[2,3-b]indol-3-yl]pyrimidin-2-yl]propan-2-ol;
5-[8-(Ethylamino)-4-(3-ethylpyrazol-1-yl)-6-fluor-9H-pyrido[2,3-b]indol-3-yl]pyridin-3-carbonitril;
6-Chlor-N-ethyl-3-(2-methoxypyrimidin-5-yl)-4-[3-(trifluormethyl)pyrazol-1-yl]-9H-pyrido[2,3-b]indol-8-amin;
5-[8-(Ethylamino)-6-fluor-4-[3-(trifluormethyl)-5,6-dihydro-4H-pyrrolo[3,4-c]pyrazol-1-yl]-9H-pyrido[2,3-b]indol-3-yl]pyridin-3-carbonitril;
N-Ethyl-6-fluor-3-(2-methoxypyrimidin-5-yl)-4-[3-(trifluormethyl)-5,6-dihydro-4H-pyrrolo[3,4-c]pyrazol-1-yl]-9H-pyrido[2,3-b]indol-8-amin;
[5-[8-(Ethylamino)-6-fluor-4-[3-(trifluormethyl)pyrazol-1-yl]-9H-pyrido[2,3-b]indol-3-yl]-3-pyridyl]methanol;
3-(5-Amino-3-pyridyl)-N-ethyl-6-fluor-4-[3-(trifluormethyl)pyrazol-1-yl]-9H-pyrido[2,3-b]indol-8-amin;
1-[6-Fluor-3-(2-methoxypyrimidin-5-yl)-8-(methylamino)-9H-pyrido[2,3-b]indol-4-yl]-5,6-dihydro-4H-pyrrolo[3,4-c]pyrazol-3-carbonitril;
1-[8-(Ethylamino)-6-fluor-3-(2-methoxypyrimidin-5-yl)-9H-pyrido[2,3-b]indol-4-yl]-4,5,6,7-tetrahydropyrazolo[3,4-c]pyridin-3-carbonitril;
3-(6-Amino-3-pyridyl)-N-ethyl-6-fluor-4-[3-(trifluormethyl)pyrazol-1-yl]-9H-pyrido[2,3-b]indol-8-amin;
5-[4-[3-(Difluormethyl)pyrazol-1-yl]-8-(ethylamino)-6-fluor-9H-pyrido[2,3-b]indol-3-yl]pyridin-3-carbonitril;
N-Ethyl-6-fluor-3-[6-(morpholinomethyl)-3-pyridyl]-4-[3-(trifluormethyl)pyrazol-1-yl]-9H-pyrido[2,3-b]indol-8-amin;
5-[8-(Ethylamino)-6-fluor-4-[3-(trifluormethyl)pyrazol-1-yl]-9H-pyrido[2,3-b]indol-3-yl]-N-methylpyridin-3-carboxamid;
N-Ethyl-6-fluor-3-(5-morpholino-3-pyridyl)-4-[3-(trifluormethyl)pyrazol-1-yl]-9H-pyrido[2,3-b]indol-8-amin;
3-[5-(Aminomethyl)-3-pyridyl]-N-ethyl-6-fluor-4-[3-(trifluormethyl)pyrazol-1-yl]-9H-pyrido[2,3-b]indol-8-amin;
3-[2-(Aminomethyl)pyrimidin-5-yl]-N-ethyl-6-fluor-4-[3-(trifluormethyl)pyrazol-1-yl]-9H-pyrido[2,3-b]indol-8-amin;
3-(2-Ethylpyrimidin-5-yl)-6-fluor-N-methyl-4-[3-(trifluormethyl)pyrazol-1-yl]-9H-pyrido[2,3-b]indol-8-amin;
[5-[8-(Ethylamino)-6-fluor-4-[3-(trifluormethyl)pyrazol-1-yl]-9H-pyrido[2,3-b]indol-3-yl]pyrimidin-2-yl]methanol;
5-[4-[4-(Aminomethyl)-3-(trifluormethyl)pyrazol-1-yl]-6,7-difluor-8-(methylamino)-9H-pyrido[2,3-b]indol-3-yl]pyridin-3-carbonitril;
N-Ethyl-6-fluor-3-(2-methylpyrimidin-5-yl)-4-[3-trifluormethyl)pyrazol-1-yl]-9H-pyrido[2,3-b]indol-8-amin;
5-[8-(Ethylamino)-6-fluor-4-thiazol-5-yl-9H-pyrido[2,3-b]indol-3-yl]pyridin-3-carbonitril;
N-Ethyl-6-fluor-3-(2-piperazin-1-ylpyrimidin-5-yl)-4-[3-(trifluormethyl)pyrazol-1-yl]-9H-pyrido[2,3-b]indol-8-amin;
N-Ethyl-6-fluor-3-[2-(4-piperidyloxy)pyrimidin-5-yl]-4-[3-(trifluormethyl)pyrazol-1-yl]-9H-pyrido[2,3-b]indol-8-amin;
1-[5-[8-(Ethylamino)-6-fluor-4-[3-(trifluormethyl)pyrazol-1-yl]-9H-pyrido[2,3-b]indol-3-yl]pyrimidin-2-yl] ethanol;
N-Ethyl-6-fluor-3-[2-[(3R)-pyrrolidin-3-yl]oxypyrimidin-5-yl]-4-[3-(trifluormethyl)pyrazol-1-yl]-9H-pyrido[2,3 -b]indol-8-amin;
N-Ethyl-6-fluor-3-[2-[(3*S*)-pyrrolidin-3-yl]oxypyrimidin-5-yl]-4-[3-(trifluormethyl)pyrazol-1-yl]-9H-pyrido[2,3-b]indol-8-amin;
(1*S*)-1-[5-[8-(Ethylamino)-6-fluor-4-[3-(trifluormethyl)pyrazol-1-yl]-9H-pyrido[2,3-b]indol-3-yl]pyrimidin-2-yl]ethanol;
N-Ethyl-6-fluor-3-[5-[(5-methyl-1,3,4-oxadiazol-2-yl)methyl]-3-pyridyl]-4-[3-(trifluormethyl)pyrazol-1-yl]-9H-pyrido[2,3-b]indol-8-amin;
6-[8-(Ethylamino)-6-fluor-4-[3-(trifluormethyl)pyrazol-1-yl]-9H-pyrido[2,3-b]indol-3-yl]pyrazolo[1,5-a]pyrimidin-3-carbonitril;
4-[4-(Aminomethyl)-3-(trifluormethyl)pyrazol-1-yl]-N-ethyl-6-fluor-3-pyrazolo[1,5-a]pyrimidin-6-yl-9H-pyrido[2,3-b]indol-8-amin;
1-[5-[8-(Ethylamino)-6-fluor-4-[3-(trifluormethyl)pyrazol-1-yl]-9H-pyrido[2,3-b]indol-3-yl]pyrimidin-2-yl]cyclopropancarbonsäure;
1-(5-(8-(Ethylamino)-6-fluor-4-(3-(trifluormethyl)-1H-pyrazol-1-yl)-9H-pyrido[2,3-b]indol-3-yl)pyrimidin-2-yl)-N-methylcyclopropancarboxamid;
1-(5-(8-(Ethylamino)-6-fluor-4-(3-(trifluormethyl)-1H-pyrazol-1-yl)-9H-pyrido[2,3-b]indol-3-yl)pyrimidin-2-yl)-N-methoxycyclopropancarboxamid;
5-[8-(Ethylamino)-6-fluor-4-[3-(trifluormethyl)pyrazol-1-yl]-9H-pyrido[2,3-b]indol-3-yl]-N-methoxypyridin-3-carboxamid;
5-[8-(Ethylamino)-6-fluor-4-[3-(trifluormethyl)pyrazol-1-yl]-9H-pyrido[2,3-b]indol-3-yl]pyrimidin-2-carboxamid;
2-[5-[8-(Ethylamino)-6-fluor-4-[3-(trifluormethyl)pyrazol-1-yl]-9H-pyrido[2,3-b]indol-3-yl]-3-pyridyl]acetamid;
5-[8-(Ethylamino)-6-fluor-4-[3-(trifluormethyl)pyrazol-1-yl]-9H-pyrido[2,3-b]indol-3-yl]-N-(2-methoxyethyl)pyridin-3-carboxamid;
5-[8-(Ethylamino)-6-fluor-4-[3-(trifluormethyl)pyrazol-1-yl]-9H-pyrido[2,3-b]indol-3-yl]-N-[1-(methoxymethyl)cyclopropyl]pyridin-3-carboxamid;
1-(3-(5-Cyanopyridin-3-yl)-8-(ethylamino)-6-fluor-9H-pyrido[2,3-b]indol-4-yl)-3-(trifluormethyl)-1H-pyrazol-4-carboxamid;
5-[8-(Ethylamino)-6-fluor-4-[4-(hydroxymethyl)-3-(trifluormethyl)pyrazol-1-yl]-9H-pyrido[2,3-b]indol-3-yl]pyridin-3-carbonitril;
5-[[5-[8-(Ethylamino)-6-fluor-4-[3-(trifluormethyl)pyrazol-1-yl]-9H-pyrido[2,3-b]indol-3-yl]-3-pyridyl]methyl]-3H-1,3,4-oxadiazol-2-on;
2-(5-(8-(Ethylamino)-6-fluor-4-(3-(trifluormethyl)-1H-pyrazol-1-yl)-9H-pyrido[2,3-b]indol-3-yl)pyridin-3-yl)ethanol und
3-(5-((1H-Imidazol-2-yl)methyl)pyridin-3-yl)-N-ethyl-6-fluor-4-(3-(trifluormethyl)-1H-pyrazol-1-yl)-9H-pyrido[2,3-b]indol-8-amin;
oder pharmazeutisch unbedenkliches Salz davon.

8. Verfahren zur Herstellung einer Verbindung nach einem der Ansprüche 1 bis 7, umfassend die Reaktion einer Verbindung der Formel (Ii) 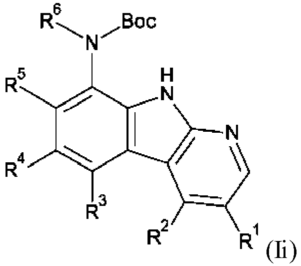 mit einer Säure, wobei R¹ bis R⁶ wie in einem der Ansprüche 1 bis 6 definiert sind.

9. Verbindung nach einem der Ansprüche 1 bis 7 zur Verwendung als therapeutisch wirksame Substanz.

10. Pharmazeutische Zusammensetzung, umfassend eine Verbindung nach einem der Ansprüche 1 bis 7 und einen therapeutisch inerten Träger.

11. Verbindung nach einem der Ansprüche 1 bis 7 zur Verwendung bei der Behandlung oder Prophylaxe einer bakteriellen Infektion.

## Revendications

1. Composé de formule (I), 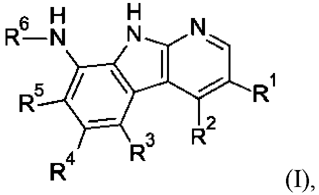 dans lequel
R¹ représente un naphthyridinyle ;
un pyrazolo[1,5-a]pyrimidinyle qui est non substitué ou substitué par un cyano ;
un pyridinyle qui est substitué par un 2-oxo-3H-1,3,4-oxadiazolyle ; un amino ; un aminoalkyle en C₁₋₆ ; un aminocarbonyle ; un aminocarbonylalkyle en C₁₋₆ ; un alcoxy en C₁₋₆-aminocarbonyle ; un alcoxy en C₁₋₆-alkyle en C₁₋₆-aminocarbonyle ; un alcoxy en C₁₋₆-alkyle en C₁₋₆-cycloalkyle en C₃₋₇-aminocarbonyle ; un alkyle en C₁₋₆ ; un alkyle en C₁₋₆-1,3,4-oxadiazolylalkyle en C₁₋₆ ; un alkyle en C₁₋₆-aminocarbonyle ; un cyano ; un halogène ; un hydroxyalkyle en C₁₋₆ ; un imidazolylalkyle en C₁₋₆ ; un morpholinyle ; ou un morpholinylalkyle en C₁₋₆ ; ou un pyrimidinyle qui est non substitué ou substitué par un amino ; un aminoalcoxy en C₁₋₆ ;
un aminoalkyle en C₁₋₆ ; un aminocarbonyle ; un azétidinyloxy ; un alcoxy en C₁₋₆ ; un alcoxy en C₁₋₆-aminocarbonylcycloalkyle en C₃₋₇ ; un alcoxy en C₁₋₆-alkyle en C₁₋₆-amino ; un alkyle en C₁₋₆ ; un alkyle en C₁₋₆-amino ; un alkyle en C₁₋₆-aminocarbonylcycloalkyle en C₃₋₇ ; un carboxy(cycloalkyle en C₃₋₇)alkyle en C₁₋₆-amino ; un carboxyalcoxy en C₁₋₆ ; un carboxyalkyle en C₁₋₆-amino ; un carboxycycloalkyle en C₃₋₇ ; un hydroxyalkyle en C₁₋₆ ; un morpholinyle ; un pipérazinyle ; un pipéridinylamino ; un pipéridinyloxy ; un pyrrolidinylamino ; un pyrrolidinyloxy ; ou un tétrahydrofuranylamino ;
R² représente un 4,5,6,7-tétrahydropyrazolo[3,4-c]pyridinyle substitué par un cyano ;
un pyrazolyle qui est substitué une ou deux fois par des substituants indépendamment choisis parmi un halogénoalkyle en C₁₋₆ ; un aminoalkyle en C₁₋₆ ; un aminocarbonyle ; un alkyle en C₁₋₆ ; un cyano ; et un hydroxyalkyle en C₁₋₆ ;
un pyrrolo[3,4-c]pyrazolyle qui est non substitué ou substitué par un cyano ou un halogénoalkyle en C₁₋₆ ; ou
un thiazolyle ;
R³ représente H ou un halogène ;
R⁴ représente un halogène ;
R⁵ représente H ou un halogène ;
R⁶ représente un alkyle en C₁₋₆ ;
ou un sel pharmaceutiquement acceptable de celui-ci.

2. Composé selon la revendication 1, dans lequel
R¹ représente un naphthyridinyle ;
un pyrazolo[1,5-a]pyrimidinyle qui est non substitué ou substitué par un cyano ;
un pyridinyle qui est substitué par un 2-oxo-3H-1,3,4-oxadiazolyle ; un amino ; un aminocarbonyle ; un aminocarbonylméthyle ; un aminométhyle ; un cyano ; un fluor ; un hydroxyéthyle ; un hydroxyméthyle ; un imidazolylméthyle ; un méthoxyaminocarbonyle ; un méthoxyéthylaminocarbonyle ; un méthoxyméthylcyclopropylaminocarbonyle ; un méthyle ; un méthyl-1,3,4-oxadiazolylméthyle ; un méthylaminocarbonyle ; un morpholinyle ; ou un morpholinylméthyle ; ou
un pyrimidinyle qui est non substitué ou substitué par un amino ; un amino(diméthyl)éthoxy ; un aminocarbonyle ; un aminométhyle ; un azétidinyloxy ; un carboxy(cyclopropyl)méthylamino ; un carboxy(diméthyl)éthoxy ; un carboxy(diméthyl)éthylamino ; un carboxycyclopropyle ; un éthoxy ; un éthyle ; un hydroxy(méthyl)éthyle ; un hydroxyéthyle ; un hydroxyméthyle ; un méthoxy ; un méthoxyaminocarbonylcyclopropyle ; un méthoxyéthylamino ; un méthyle ; un méthylamino ; un méthylaminocarbonylcyclopropyle ; un morpholinyle ; un pipérazinyle ; un pipéridinylamino ; un pipéridinyloxy ; un pyrrolidinylamino ; un pyrrolidinyloxy ; ou un tétrahydrofuranylamino ;
R² représente un 4,5,6,7-tétrahydropyrazolo[3,4-c]pyridinyle substitué par un cyano ;
un pyrazolyle qui est substitué une ou deux fois par des substituants indépendamment choisis parmi un aminocarbonyle ; un aminométhyle ; un cyano ; un difluorométhyle ; un éthyle ; un hydroxyméthyle ; et un trifluorométhyle ;
un pyrrolo[3,4-c]pyrazolyle qui est non substitué ou substitué par un cyano ou un trifluorométhyle ; ou
un thiazolyle ;
R³ représente H ou un fluor ;
R⁴ représente un fluor ou un chlore ;
R⁵ représente H ou un fluor ;
R⁶ représente un éthyle ou un méthyle ;
ou un sel pharmaceutiquement acceptable de celui-ci.

3. Composé selon la revendication 1, ou un sel pharmaceutiquement acceptable de celui-ci, dans lequel
R¹ représente un naphthyridinyle ;
un pyrazolo[1,5-a]pyrimidinyle ;
un pyridinyle qui est substitué par un 2-oxo-3H-1,3,4-oxadiazolyle ; un amino ; un aminoalkyle en C₁₋₆; un aminocarbonyle ; un alcoxy en C₁₋₆-aminocarbonyle ; un alcoxy en C₁₋₆-alkyle en C₁₋₆-aminocarbonyle ; un alkyle en C₁₋₆-aminocarbonyle ; un cyano ; ou un hydroxyalkyle en C₁₋₆ ; ou
un pyrimidinyle qui est non substitué ou substitué par un amino ; un aminoalcoxy en C₁₋₆ ; un aminoalkyle en C₁₋₆ ; un aminocarbonyle ; un alcoxy en C₁₋₆ ; un alcoxy en C₁₋₆-alkyle en C₁₋₆-amino ; un alkyle en C₁₋₆ ; un alkyle en C₁₋₆-amino ; un hydroxyalkyle en C₁₋₆ ; un pipérazinyle ; un pipéridinylamino ; un pipéridinyloxy ; un pyrrolidinylamino ; un pyrrolidinyloxy ; ou un tétrahydrofuranylamino.

4. Composé selon la revendication 3, ou un sel pharmaceutiquement acceptable de celui-ci, dans lequel R² représente un pyrazolyle qui est substitué une ou deux fois par des substituants indépendamment choisis parmi un halogénoalkyle en C₁₋₆, un aminoalkyle en C₁₋₆, un aminocarbonyle, un alkyle en C₁₋₆, un cyano et un hydroxyalkyle en C₁₋₆.

5. Composé selon la revendication 3 ou 4, ou un sel pharmaceutiquement acceptable de celui-ci, dans lequel
R¹ représente un naphthyridinyle ;
un pyrazolo[1,5-a]pyrimidinyle ;
un pyridinyle qui est substitué par un 2-oxo-3H-1,3,4-oxadiazolyle ; un amino ; un aminoalkyle en C₁₋₆ ; un aminocarbonyle ; un alcoxy en C₁₋₆-aminocarbonyle ; un alcoxy en C₁₋₆-alkyle en C₁₋₆-aminocarbonyle ; un alkyle en C₁₋₆-aminocarbonyle ; un cyano ; ou un hydroxyalkyle en C₁₋₆ ; ou
un pyrimidinyle qui est non substitué ou substitué par un amino ; un aminoalcoxy en C₁₋₆ ; un aminoalkyle en C₁₋₆ ; un aminocarbonyle ; un alcoxy en C₁₋₆ ; un alcoxy en C₁₋₆-alkyle en C₁₋₆-amino ; un alkyle en C₁₋₆ ; un alkyle en C₁₋₆-amino ; un hydroxyalkyle en C₁₋₆ ; un pipérazinyle ; un pipéridinylamino ; un pipéridinyloxy ; un pyrrolidinylamino ; un pyrrolidinyloxy ; ou un tétrahydrofuranylamino ;
R² représente un pyrazolyle qui est substitué une ou deux fois par des substituants indépendamment choisis parmi un halogénoalkyle en C₁₋₆, un aminoalkyle en C₁₋₆, un aminocarbonyle, un alkyle en C₁₋₆, un cyano et un hydroxyalkyle en C₁₋₆ ;
R³ représente H ou un halogène ;
R⁴ représente un halogène ;
R⁵ représente H ou un halogène ;
R⁶ représente un alkyle en C₁₋₆ ;
ou un sel pharmaceutiquement acceptable de celui-ci.

6. Composé selon la revendication 5, ou un sel pharmaceutiquement acceptable de celui-ci, dans lequel
R¹ représente un naphthyridinyle ; un pyrazolo[1,5-a]pyrimidinyle ; un cyanopyridinyle ; un aminocarbonylpyridinyle ; un hydroxyméthylpyridinyle ; un aminopyridinyle ; un méthylaminocarbonylpyridinyle ; un aminométhylpyridinyle ; un méthoxyaminocarbonylpyridinyle ; un 2-oxo-3H-1,3,4-oxadiazolylpyridinyle ; un hydroxyéthylpyridinyle ; un méthoxyéthylaminocarbonylpyridinyle ; un amino(diméthyl)éthoxypyrimidinyle ; un aminocarbonylpyrimidinyle ; un aminométhylpyrimidinyle ; un aminopyrimidinyle ; un éthoxypyrimidinyle ; un hydroxy(méthyl)éthylpyrimidinyle ; un hydroxyéthylpyrimidinyle ; un hydroxyméthylpyrimidinyle ; un méthoxyéthylaminopyrimidinyle ; un méthoxypyrimidinyle ; un méthylaminopyrimidinyle ; un méthylpyrimidinyle ; un pipérazinylpyrimidinyle ; un pipéridinylaminopyrimidinyle ; un pipéridinyloxypyrimidinyle ; un pyrrolidinylaminopyrimidinyle ; un pyrrolidinyloxypyrimidinyle ; ou un tétrahydrofuranylaminopyrimidinyle ;
R² représente un aminocarbonyl(trifluorométhyl)pyrazolyle ; un aminométhyl(trifluorométhyl)pyrazolyle ; un cyanopyrazolyle ; un difluorométhylpyrazolyle ; un éthylpyrazolyle ; un hydroxyméthyl(trifluorométhyl)pyrazolyle ; ou un trifluorométhylpyrazolyle ;
R³ représente H ou un fluor ;
R⁴ représente un fluor ou un chlore ;
R⁵ représente H ou un fluor ;
R⁶ représente un méthyle ou un éthyle ;
ou un sel pharmaceutiquement acceptable de celui-ci.

7. Composé selon l'une quelconque des revendications 1 à 6 choisi parmi :
le 5-[8-(éthylamino)-6-fluoro-4-[3-(trifluorométhyl)pyrazol-1-yl]-9H-pyrido[2,3-b]indol-3-yl]pyridine-3-carbonitrile ;
la 6-fluoro-3-(2-méthoxypyrimidin-5-yl)-N-méthyl-4-[3-(trifluorométhyl)pyrazol-1-yl]-9H-pyrido[2,3-b]indol-8-amine ;
le 5-[8-(éthylamino)-6-fluoro-4-[4-(trifluorométhyl)pyrazol-1-yl]-9H-pyrido[2,3-b]indol-3-yl]pyridine-3-carbonitrile ;
la N-éthyl-6-fluoro-3-(2-méthoxypyrimidin-5-yl)-4-[3-(trifluorométhyl)pyrazol-1-yl]-9H-pyrido[2,3-b]indol-8-amine ;
le 5-[4-(3-cyanopyrazol-1-yl)-8-(éthylamino)-6-fluoro-9H-pyrido[2,3-b]indol-3-yl]pyridine-3-carbonitrile ;
la 3-(2-aminopyrimidin-5-yl)-N-éthyl-6-fluoro-4-[3-(trifluorométhyl)pyrazol-1-yl]-9H-pyrido[2,3-b]indol-8-amine ;
la N-éthyl-6-fluoro-3-(6-méthyl-3-pyridyl)-4-[3-(trifluorométhyl)pyrazol-1-yl]-9H-pyrido[2,3-b]indol-8-amine ;
le 5-[6-fluoro-8-(méthylamino)-4-[4-(trifluorométhyl)pyrazol-1-yl]-9H-pyrido[2,3-b]indol-3-yl]pyridine-3-carbonitrile ;
le 5-[4-(4-cyanopyrazol-1-yl)-6-fluoro-8-(méthylamino)-9H-pyrido[2,3-b]indol-3-yl]pyridine-3-carbonitrile ;
la N-éthyl-6-fluoro-3-[2-(4-pipéridylamino)pyrimidin-5-yl]-4-[3-(trifluorométhyl)pyrazol-1-yl]-9H-pyrido[2,3-b]indol-8-amine ;
la N-éthyl-6-fluoro-3-(1,5-naphthyridin-3-yl)-4-[3-(trifluorométhyl)pyrazol-1-yl]-9H-pyrido[2,3-b]indol-8-amine ;
laN-éthyl-6-fluoro-3-pyrimidin-5-yl-4-[3-(trifluorométhyl)pyrazol-1-yl]-9H-pyrido[2,3-b]indol-8-amine ;
la N-éthyl-6-fluoro-3-[2-(2-méthoxyéthylamino)pyrimidin-5-yl]-4-[3-(trifluorométhyl)pyrazol-1-yl]-9H-pyrido[2,3-b]indol-8-amine ;
le 5-[4-[4-(aminométhyl)-3-(trifluorométhyl)pyrazol-1-yl]-6-fluoro-8-(méthylamino)-9H-pyrido[2,3-b]indol-3-yl]pyridine-3-carbonitrile ;
le 5-[8-(éthylamino)-6-fluoro-4-[3-(trifluorométhyl)pyrazol-1-yl]-9H-pyrido[2,3-b]indol-3-yl]pyridine-3-carboxamide ;
la N-éthyl-6-fluoro-3-(2-morpholinopyrimidin-5-yl)-4-[3-(trifluorométhyl)pyrazol-1-yl]-9H-pyrido[2,3-b]indol-8-amine ;
la N-éthyl-6-fluoro-3-[2-(méthylamino)pyrimidin-5-yl]-4-[3-(trifluorométhyl)pyrazol-1-yl]-9H-pyrido[2,3-b]indol-8-amine ;
la N-éthyl-6-fluoro-3-(5-fluoro-3-pyridyl)-4-[3-(trifluorométhyl)pyrazol-1-yl]-9H-pyrido[2,3-b]indol-8-amine ;
la N-éthyl-6-fluoro-3-pyrazolo[1,5-a]pyrimidin-6-yl-4-[3-(trifluorométhyl)pyrazol-1-yl]-9H-pyrido[2,3-b]indol-8-amine ;
la N-éthyl-6-fluoro-3-(2-pyrrolidin-3-yloxypyrimidin-5-yl)-4-[3-(trifluorométhyl)pyrazol-1-yl]-9H-pyrido[2,3-b]indol-8-amine ;
la 4-(5,6-dihydro-4H-pyrrolo[3,4-c]pyrazol-1-yl)-N-éthyl-6-fluoro-3-(2-méthoxypyrimidin-5-yl)-9H-pyrido[2,3-b]indol-8-amine ;
la 3-(2-éthoxypyrimidin-5-yl)-N-éthyl-6-fluoro-4-[3-(trifluorométhyl)pyrazol-1-yl]-9H-pyrido[2,3-b]indol-8-amine ;
la 3-[2-(azétidin-3-yloxy)pyrimidin-5-yl]-N-éthyl-6-fluoro-4-[3-(trifluorométhyl)pyrazol-1-yl]-9H-pyrido[2,3-b]indol-8-amine ;
la N-éthyl-6-fluoro-3-[2-(pyrrolidin-3-ylamino)pyrimidin-5-yl]-4-[3-(trifluorométhyl)pyrazol-1-yl]-9H-pyrido[2,3-b]indol-8-amine ;
la N-éthyl-6-fluoro-3-[2-(tétrahydrofuran-3-ylamino)pyrimidin-5-yl]-4-[3-(trifluorométhyl)pyrazol-1-yl]-9H-pyrido[2,3-b]indol-8-amine ;
le 5-[4-[4-(aminométhyl)-3-(trifluorométhyl)pyrazol-1-yl]-8-(éthylamino)-6-fluoro-9H-pyrido[2,3-b]indol-3-yl]pyridine-3-carbonitrile ;
la 4-[4-(aminométhyl)-3-(trifluorométhyl)pyrazol-1-yl]-N-éthyl-6-fluoro-3-(2-méthoxypyrimidin-5-yl)-9H-pyrido[2,3-b]indol-8-amine ;
le 5-[8-(éthylamino)-5,6-difluoro-4-[3-(trifluorométhyl)pyrazol-1-yl]-9H-pyrido[2,3-b]indol-3-yl]pyridine-3-carbonitrile ;
la N-éthyl-5,6-difluoro-3-(2-méthoxypyrimidin-5-yl)-4-[3-(trifluorométhyl)pyrazol-1-yl]-9H-pyrido[2,3-b]indol-8-amine ;
la 3-[2-(2-amino-2-méthyl-propoxy)pyrimidin-5-yl]-N-éthyl-6-fluoro-4-[3-(trifluorométhyl)pyrazol-1-yl]-9H-pyrido[2,3-b]indol-8-amine ;
le 2-[5-[8-(éthylamino)-6-fluoro-4-[3-(trifluorométhyl)pyrazol-1-yl]-9H-pyrido[2,3-b]indol-3-yl]pyrimidin-2-yl]propan-2-ol ;
le 5-[8-(éthylamino)-4-(3-éthylpyrazol-1-yl)-6-fluoro-9H-pyrido[2,3-b]indol-3-yl]pyridine-3-carbonitrile ;
la 6-chloro-N-éthyl-3-(2-méthoxypyrimidin-5-yl)-4-[3-(trifluorométhyl)pyrazol-1-yl]-9H-pyrido[2,3-b]indol-8-amine ;
le 5-[8-(éthylamino)-6-fluoro-4-[3-(trifluorométhyl)-5,6-dihydro-4H-pyrrolo[3,4-c]pyrazol-1-yl]-9H-pyrido[2,3-b]indol-3-yl]pyridine-3-carbonitrile ;
la N-éthyl-6-fluoro-3-(2-méthoxypyrimidin-5-yl)-4-[3-(trifluorométhyl)-5,6-dihydro-4H-pyrrolo[3,4-c]pyrazol-1-yl]-9H-pyrido[2,3-b]indol-8-amine ;
le [5-[8-(éthylamino)-6-fluoro-4-[3-(trifluorométhyl)pyrazol-1-yl]-9H-pyrido[2,3-b]indol -3 -yl] -3 -pyridyl]méthanol ;
la 3-(5-amino-3-pyridyl)-N-éthyl-6-fluoro-4-[3-(trifluorométhyl)pyrazol-1-yl]-9H-pyrido[2,3-b]indol-8-amine ;
le 1-[6-fluoro-3-(2-méthoxypyrimidin-5-yl)-8-(méthylamino)-9H-pyrido[2,3-b]indol-4-yl]-5,6-dihydro-4H-pyrrolo[3,4-c]pyrazole-3-carbonitrile ;
le 1-[8-(éthylamino)-6-fluoro-3-(2-méthoxypyrimidin-5-yl)-9H-pyrido[2,3-b]indol-4-yl]-4,5,6,7-tétrahydropyrazolo[3,4-c]pyridine-3-carbonitrile ;
la 3-(6-amino-3-pyridyl)-N-éthyl-6-fluoro-4-[3-(trifluorométhyl)pyrazol-1-yl]-9H-pyrido[2,3-b]indol-8-amine ;
le 5-[4-[3-(difluorométhyl)pyrazol-1-yl]-8-(éthylamino)-6-fluoro-9H-pyrido[2,3-b]indol-3-yl]pyridine-3-carbonitrile ;
la N-éthyl-6-fluoro-3-[6-(morpholinométhyl)-3-pyridyl]-4-[3-(trifluorométhyl)pyrazol-1-yl]-9H-pyrido[2,3-b]indol-8-amine ;
le 5-[8-(éthylamino)-6-fluoro-4-[3-(trifluorométhyl)pyrazol-1-yl]-9H-pyrido[2,3-b]indol-3-yl]-N-méthyl-pyridine-3-carboxamide ;
la N-éthyl-6-fluoro-3-(5-morpholino-3-pyridyl)-4-[3-(trifluorométhyl)pyrazol-1-yl]-9H-pyrido[2,3-b]indol-8-amine ;
la 3-[5-(aminométhyl)-3-pyridyl]-N-éthyl-6-fluoro-4-[3-(trifluorométhyl)pyrazol-1-yl]-9H-pyrido[2,3-b]indol-8-amine ;
la 3-[2-(aminométhyl)pyrimidin-5-yl]-N-éthyl-6-fluoro-4-[3-(trifluorométhyl)pyrazol-1-yl]-9H-pyrido[2,3-b]indol-8-amine ;
la 3-(2-éthylpyrimidin-5-yl)-6-fluoro-N-méthyl-4-[3-(trifluorométhyl)pyrazol-1-yl]-9H-pyrido[2,3-b]indol-8-amine ;
le [5-[8-(éthylamino)-6-fluoro-4-[3-(trifluorométhyl)pyrazol-1-yl]-9H-pyrido[2,3-b]indol-3-yl]pyrimidin-2-yl]méthanol ;
le 5-[4-[4-(aminométhyl)-3-(trifluorométhyl)pyrazol-1-yl]-6,7-difluoro-8-(méthylamino)-9H-pyrido[2,3-b]indol-3-yl]pyridine-3-carbonitrile ;
la N-éthyl-6-fluoro-3-(2-méthylpyrimidin-5-yl)-4-[3-trifluorométhyl)pyrazol-1-yl]-9H-pyrido[2,3-b]indol-8-amine ;
le 5-[8-(éthylamino)-6-fluoro-4-thiazol-5-yl-9H-pyrido[2,3-b]indol-3-yl]pyridine-3-carbonitrile ;
la N-éthyl-6-fluoro-3-(2-pipérazin-1-ylpyrimidin-5-yl)-4-[3-(trifluorométhyl)pyrazol-1-yl]-9H-pyrido[2,3-b]indol-8-amine ;
la N-éthyl-6-fluoro-3-[2-(4-pipéridyloxy)pyrimidin-5-yl]-4-[3-(trifluorométhyl)pyrazol-1-yl]-9H-pyrido[2,3-b]indol-8-amine ;
le 1-[5-[8-(éthylamino)-6-fluoro-4-[3-(trifluorométhyl)pyrazol-1-yl]-9H-pyrido[2,3-b]indol-3-yl]pyrimidin-2-yl]éthanol ;
la N-éthyl-6-fluoro-3-[2-[(3R)-pyrrolidin-3-yl]oxypyrimidin-5-yl]-4-[3-(trifluorométhyl)pyrazol-1-yl]-9H-pyrido[2,3-b]indol-8-amine ;
la N-éthyl-6-fluoro-3-[2-[(3*S*)-pyrrolidin-3-yl]oxypyrimidin-5-yl]-4-[3-(trifluorométhyl)pyrazol-1-yl]-9H-pyrido[2,3-b]indol-8-amine ;
le (1*S*)-1-[5-[8-(éthylamino)-6-fluoro-4-[3-(trifluorométhyl)pyrazol-1-yl]-9H-pyrido[2,3-b]indol-3-yl]pyrimidin-2-yl]éthanol ;
la N-éthyl-6-fluoro-3-[5-[(5-méthyl-1,3,4-oxadiazol-2-yl)méthyl]-3-pyridyl]-4-[3-(trifluorométhyl)pyrazol-1-yl]-9H-pyrido[2,3-b]indol-8-amine ;
le 6-[8-(éthylamino)-6-fluoro-4-[3-(trifluorométhyl)pyrazol-1-yl]-9H-pyrido[2,3-b]indol-3-yl]pyrazolo[1,5-a]pyrimidine-3-carbonitrile ;
la 4-[4-(aminométhyl)-3-(trifluorométhyl)pyrazol-1-yl]-N-éthyl-6-fluoro-3-pyrazolo[1,5-a]pyrimidin-6-yl-9H-pyrido[2,3-b]indol-8-amine ;
l'acide 1-[5-[8-(éthylamino)-6-fluoro-4-[3-(trifluorométhyl)pyrazol-1-yl]-9H-pyrido[2,3-b]indol-3-yl]pyrimidin-2-yl]cyclopropanecarboxylique ;
le 1-(5-(8-(éthylamino)-6-fluoro-4-(3-(trifluorométhyl)-1H-pyrazol-1-yl)-9H-pyrido[2,3-b]indol-3-yl)pyrimidin-2-yl)-N-méthylcyclopropanecarboxamide ;
le 1-(5-(8-(éthylamino)-6-fluoro-4-(3-(trifluorométhyl)-1H-pyrazol-1-yl)-9H-pyrido[2,3-b]indol-3-yl)pyrimidin-2-yl)-N-méthoxycyclopropanecarboxamide ;
le 5-[8-(éthylamino)-6-fluoro-4-[3-(trifluorométhyl)pyrazol-1-yl]-9H-pyrido[2,3-b]indol-3-yl]-N-méthoxy-pyridine-3-carboxamide ;
le 5-[8-(éthylamino)-6-fluoro-4-[3-(trifluorométhyl)pyrazol-1-yl]-9H-pyrido[2,3-b]indol-3-yl]pyrimidine-2-carboxamide ;
le 2-[5-[8-(éthylamino)-6-fluoro-4-[3-(trifluorométhyl)pyrazol-1-yl]-9H-pyrido[2,3-b]indol-3-yl]-3-pyridyl]acétamide ;
le 5-[8-(éthylamino)-6-fluoro-4-[3-(trifluorométhyl)pyrazol-1-yl]-9H-pyrido[2,3-b]indol-3-yl]-N-(2-méthoxyéthyl)pyridine-3-carboxamide ;
le 5-[8-(éthylamino)-6-fluoro-4-[3-(trifluorométhyl)pyrazol-1-yl]-9H-pyrido[2,3-b]indol-3-yl]-N-[1-(méthoxyméthyl)cyclopropyl]pyridine-3-carboxamide ;
le 1-(3-(5-cyanopyridin-3-yl)-8-(éthylamino)-6-fluoro-9H-pyrido[2,3-b]indol-4-yl)-3-(trifluorométhyl)-1H-pyrazole-4-carboxamide ;
le 5-[8-(éthylamino)-6-fluoro-4-[4-(hydroxyméthyl)-3-(trifluorométhyl)pyrazol-1-yl]-9H-pyrido[2,3-b]indol-3-yl]pyridine-3-carbonitrile ;
la 5-[[5-[8-(éthylamino)-6-fluoro-4-[3-(trifluorométhyl)pyrazol-1-yl]-9H-pyrido[2,3-b]indol-3-yl]-3-pyridyl]méthyl]-3H-1,3,4-oxadiazol-2-one ;
le 2-(5-(8-(éthylamino)-6-fluoro-4-(3-(trifluorométhyl)-1H-pyrazol-1-yl)-9H-pyrido[2,3-b]indol-3-yl)pyridin-3-yl)éthanol ; et
la 3-(5-((1H-imidazol-2-yl)méthyl)pyridin-3-yl)-N-éthyl-6-fluoro-4-(3-(trifluorométhyl)-1H-pyrazol-1-yl)-9H-pyrido[2,3-b]indol-8-amine ;
ou un sel pharmaceutiquement acceptable de ceux-ci.

8. Procédé de préparation d'un composé selon l'une quelconque des revendications 1 à 7 comprenant la réaction d'un composé de formule (Ii), 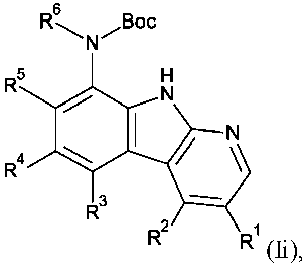 avec un acide, dans lequel R¹ à R⁶ sont tels que définis dans l'une quelconque des revendications 1 à 6.

9. Composé selon l'une quelconque des revendications 1 à 7 pour une utilisation comme substance thérapeutiquement active.

10. Composition pharmaceutique comprenant un composé selon l'une quelconque des revendications 1 à 7 et un véhicule thérapeutiquement inerte.

11. Composé selon l'une quelconque des revendications 1 à 7 pour une utilisation dans le traitement ou la prophylaxie d'une infection bactérienne.
